# EUROPEAN PATENT APPLICATION

(11) **EP 2 500 035 A1**
(43) Date of publication of application: **19.09.2012**
(21) Application number: 11002145.8
(22) Date of filing: 15.03.2011
(51) Int. Cl.: A61K 39/395, C07K 16/00, A61K 47/48, A61P 35/00

(54) **Pharmaceutical formulation containing immunglobulin**

(71) Applicant: Icon Genetics GmbH, 80333 München (DE)
(72) Inventor: Olbrich, Carsten, Dr., 10961 Berlin (DE); Krause, Michael, Dr., 14195 Berlin (DE); Trill, Thomas, 16552 Schildow (DE)
(74) Representative: Blodig, Wolfgang

(57) **Abstract**

A set of at least two different protein conjugate preparations, each protein conjugate preparation comprising histidine as a buffering agent and a protein conjugate comprising one or more immunoglobulin moieties conjugated to a carrier protein; wherein the immunoglobulin moieties of each element of said set of protein conjugate preparation have identical complementarity determining regions (CDRs); and wherein different protein conjugate preparations differ in that the immunoglobulin moieties of the protein conjugates have different CDRs.

SEQUENCE LISTING

<110>  Icon Genetics GmbH

<120>  Pharmaceutical Formulation containing Immunoglobulin

<130>  EAD-15480

<160>  26

<170>  PatentIn version 3.5

<210>  1
<211>  3408
<212>  PRT
<213>  Megathura crenulata

<400>  1

Met Leu Ser Val Arg Leu Leu Ile Val Val Leu Ala Leu Ala Asn Ala
1               5                   10                  15

Glu Asn Leu Val Arg Lys Ser Val Glu His Leu Thr Gln Glu Glu Thr
            20                  25                  30

Leu Asp Leu Gln Ala Ala Leu Arg Glu Leu Gln Met Asp Ser Ser Ser
        35                  40                  45

Ile Gly Phe Gln Lys Ile Ala Ala Ala His Gly Ala Pro Ala Ser Cys
    50                  55                  60

Val His Lys Asp Thr Ser Ile Ala Cys Cys Ile His Gly Met Pro Thr
65                  70                  75                  80

Phe Pro His Trp His Arg Ala Tyr Val Val His Met Glu Arg Ala Leu
                85                  90                  95

Gln Thr Lys Arg Arg Thr Ser Gly Leu Pro Tyr Trp Asp Trp Thr Glu
            100                 105                 110

Pro Ile Thr Gln Leu Pro Ser Leu Ala Ala Asp Pro Val Tyr Ile Asp
        115                 120                 125

Ser Gln Gly Gly Lys Ala His Thr Asn Tyr Trp Tyr Arg Gly Asn Ile
    130                 135                 140

Gly Phe Leu Asp Lys Lys Thr Asn Arg Ala Ala Asp Asp Arg Leu Phe
145                 150                 155                 160

Glu Lys Val Lys Pro Gly Gln His Thr His Leu Met Glu Ser Val Leu
                165                 170                 175

Asp Pro Leu Glu Gln Asp Glu Phe Cys Lys Phe Glu Ile Gln Phe Glu
            180                 185                 190

Leu Pro His Asn Ala Ile His Tyr Leu Val Gly Gly Lys His Asp Tyr
        195                 200                 205

Ser Met Ala Asn Leu Glu Tyr Thr Ala Tyr Asp Pro Ile Phe Phe Leu
    210                 215                 220

His His Ser Asn Val Asp Arg Ile Phe Ala Ile Trp Gln Arg Leu Gln
225                 230                 235                 240

Glu Leu Arg Asn Lys Asp Pro Lys Ala Met Asp Cys Ala Gln Glu Leu
                245                 250                 255

Leu His Gln Lys Met Glu Pro Phe Ser Trp Glu Asp Asn Asp Ile Pro
            260                 265                 270

Leu Thr Asn Asp Tyr Asp Thr Leu Asn Leu Asn Gly Met Thr Pro Glu
        275                 280                 285

Glu Leu Lys Thr Tyr Leu Asp Glu Arg Ser Ser Arg Ala Arg Ala Phe
    290                 295                 300

Ala Ser Phe Arg Leu Lys Gly Phe Gly Gly Ser Ala Asn Val Phe Val
305                 310                 315                 320

Tyr Val Cys Ile Pro Asp Asp Asn Asp Arg Asn Asp Asp His Cys Glu
                325                 330                 335

Lys Ala Gly Asp Phe Phe Val Leu Gly Gly Pro Ser Glu Met Lys Trp
            340                 345                 350

Gln Phe Tyr Arg Pro Tyr Leu Phe Asp Leu Ser Asp Thr Val His Lys
        355                 360                 365

Met Gly Met Lys Leu Asp Gly His Tyr Thr Val Lys Ala Glu Leu Phe
    370                 375                 380

Ser Val Asn Gly Thr Ala Leu Pro Asp Asp Leu Leu Pro His Pro Val
385                 390                 395                 400

Val Val His His Pro Glu Lys Gly Phe Thr Asp Pro Pro Val Lys His
                405                 410                 415

His Gln Ser Ala Asn Leu Leu Val Arg Lys Asn Ile Asn Asp Leu Thr
            420                 425                 430

Arg Glu Glu Val Leu Asn Leu Arg Glu Ala Phe His Lys Phe Gln Glu
        435                 440                 445

Asp Arg Ser Val Asp Gly Tyr Gln Ala Thr Ala Glu Tyr His Gly Leu
    450                 455                 460

Pro Ala Arg Cys Pro Arg Pro Asp Ala Lys Asp Arg Tyr Ala Cys Cys
465                 470                 475                 480

Val His Gly Met Pro Ile Phe Pro His Trp His Arg Leu Phe Val Thr
                485                 490                 495

Gln Val Glu Asp Ala Leu Val Gly Arg Gly Ala Thr Ile Gly Ile Pro
            500                 505                 510

Tyr Trp Gly Leu Pro Tyr Trp Asp Trp Thr Glu Pro Met Thr His Ile
        515                 520                 525

Pro Gly Leu Ala Gly Asn Lys Thr Tyr Val Asp Ser His Gly Ala Ser
    530                 535                 540

His Thr Asn Pro Phe His Ser Ser Val Ile Ala Phe Glu Glu Asn Ala
545                 550                 555                 560

Pro His Thr Lys Arg Gln Ile Asp Gln Arg Leu Phe Lys Pro Ala Thr
                565                 570                 575

Phe Gly His His Thr Asp Leu Phe Asn Gln Ile Leu Tyr Ala Phe Glu
            580                 585                 590

Gln Glu Asp Tyr Cys Asp Phe Glu Val Gln Phe Glu Ile Thr His Asn
        595                 600                 605

Thr Ile His Ala Trp Thr Gly Gly Ser Glu His Phe Ser Met Ser Ser
    610                 615                 620

Leu His Tyr Thr Ala Phe Asp Pro Leu Phe Tyr Phe His His Ser Asn
625                 630                 635                 640

Val Asp Arg Leu Trp Ala Val Trp Gln Ala Leu Gln Met Arg Arg His
                645                 650                 655

Lys Pro Tyr Arg Ala His Cys Ala Ile Ser Leu Glu His Met His Leu
            660                 665                 670

Lys Pro Phe Ala Phe Ser Ser Pro Leu Asn Asn Asn Glu Lys Thr His
        675                 680                 685

Ala Asn Ala Met Pro Asn Lys Ile Tyr Asp Tyr Glu Asn Val Leu His
    690                 695                 700

Tyr Thr Tyr Glu Asp Leu Thr Phe Gly Gly Ile Ser Leu Glu Asn Ile
705                 710                 715                 720

Glu Lys Met Ile His Glu Asn Gln Gln Glu Asp Arg Ile Tyr Ala Gly
                725                 730                 735

Phe Leu Leu Ala Gly Ile Arg Thr Ser Ala Asn Val Asp Ile Phe Ile
        740                 745                 750

Lys Thr Thr Asp Ser Val Gln His Lys Ala Gly Thr Phe Ala Val Leu
    755                 760                 765

Gly Gly Ser Lys Glu Met Lys Trp Gly Phe Asp Arg Val Phe Lys Phe
770                 775                 780

Asp Ile Thr His Val Leu Lys Asp Leu Asp Leu Thr Ala Asp Gly Asp
785                 790                 795                 800

Phe Glu Val Thr Val Asp Ile Thr Glu Val Asp Gly Thr Lys Leu Ala
                805                 810                 815

Ser Ser Leu Ile Pro His Ala Ser Val Ile Arg Glu His Ala Arg Gly
            820                 825                 830

Lys Leu Asn Arg Val Lys Phe Asp Lys Val Pro Arg Ser Arg Leu Ile
        835                 840                 845

Arg Lys Asn Val Asp Arg Leu Ser Pro Glu Glu Met Asn Glu Leu Arg
    850                 855                 860

Lys Ala Leu Ala Leu Leu Lys Glu Asp Lys Ser Ala Gly Gly Phe Gln
865                 870                 875                 880

Gln Leu Gly Ala Phe His Gly Glu Pro Lys Trp Cys Pro Ser Pro Glu
                885                 890                 895

Ala Ser Lys Lys Phe Ala Cys Cys Val His Gly Met Ser Val Phe Pro
            900                 905                 910

His Trp His Arg Leu Leu Thr Val Gln Ser Glu Asn Ala Leu Arg Arg
        915                 920                 925

His Gly Tyr Asp Gly Ala Leu Pro Tyr Trp Asp Trp Thr Ser Pro Leu
    930                 935                 940

Asn His Leu Pro Glu Leu Ala Asp His Glu Lys Tyr Val Asp Pro Glu
945                 950                 955                 960

Asp Gly Val Glu Lys His Asn Pro Trp Phe Asp Gly His Ile Asp Thr
                965                 970                 975

Val Asp Lys Thr Thr Thr Arg Ser Val Gln Asn Lys Leu Phe Glu Gln
            980                 985                 990

Pro Glu Phe Gly His Tyr Thr Ser  Ile Ala Lys Gln Val  Leu Leu Ala

995                 1000                1005

Leu Glu Gln Asp Asn Phe Cys Asp Phe Glu Ile Gln Tyr Glu Ile
    1010                1015                1020

Ala His Asn Tyr Ile His Ala Leu Val Gly Gly Ala Gln Pro Tyr
    1025                1030                1035

Gly Met Ala Ser Leu Arg Tyr Thr Ala Phe Asp Pro Leu Phe Tyr
    1040                1045                1050

Leu His His Ser Asn Thr Asp Arg Ile Trp Ala Ile Trp Gln Ala
    1055                1060                1065

Leu Gln Lys Tyr Arg Gly Lys Pro Tyr Asn Val Ala Asn Cys Ala
    1070                1075                1080

Val Thr Ser Met Arg Glu Pro Leu Gln Pro Phe Gly Leu Ser Ala
    1085                1090                1095

Asn Ile Asn Thr Asp His Val Thr Lys Glu His Ser Val Pro Phe
    1100                1105                1110

Asn Val Phe Asp Tyr Lys Thr Asn Phe Asn Tyr Glu Tyr Asp Thr
    1115                1120                1125

Leu Glu Phe Asn Gly Leu Ser Ile Ser Gln Leu Asn Lys Lys Leu
    1130                1135                1140

Glu Ala Ile Lys Ser Gln Asp Arg Phe Phe Ala Gly Phe Leu Leu
    1145                1150                1155

Ser Gly Phe Lys Lys Ser Ser Leu Val Lys Phe Asn Ile Cys Thr
    1160                1165                1170

Asp Ser Ser Asn Cys His Pro Ala Gly Glu Phe Tyr Leu Leu Gly
    1175                1180                1185

Asp Glu Asn Glu Met Pro Trp Ala Tyr Asp Arg Val Phe Lys Tyr
    1190                1195                1200

Asp Ile Thr Glu Lys Leu His Asp Leu Lys Leu His Ala Glu Asp
    1205                1210                1215

His Phe Tyr Ile Asp Tyr Glu Val Phe Asp Leu Lys Pro Ala Ser
    1220                1225                1230

Leu Gly Lys Asp Leu Phe Lys Gln Pro Ser Val Ile His Glu Pro
    1235                1240                1245

Arg Ile Gly His His Glu Gly Glu Val Tyr Gln Ala Glu Val Thr
    1250                1255                1260

Ser Ala Asn Arg Ile Arg Lys Asn Ile Glu Asn Leu Ser Leu Gly
    1265                1270                1275

Glu Leu Glu Ser Leu Arg Ala Ala Phe Leu Glu Ile Glu Asn Asp
    1280                1285                1290

Gly Thr Tyr Glu Ser Ile Ala Lys Phe His Gly Ser Pro Gly Leu
    1295                1300                1305

Cys Gln Leu Asn Gly Asn Pro Ile Ser Cys Cys Val His Gly Met
    1310                1315                1320

Pro Thr Phe Pro His Trp His Arg Leu Tyr Val Val Val Val Glu
    1325                1330                1335

Asn Ala Leu Leu Lys Lys Gly Ser Ser Val Ala Val Pro Tyr Trp
    1340                1345                1350

Asp Trp Thr Lys Arg Ile Glu His Leu Pro His Leu Ile Ser Asp
    1355                1360                1365

Ala Thr Tyr Tyr Asn Ser Arg Gln His His Tyr Glu Thr Asn Pro
    1370                1375                1380

Phe His His Gly Lys Ile Thr His Glu Asn Glu Ile Thr Thr Arg
    1385                1390                1395

Asp Pro Lys Asp Ser Leu Phe His Ser Asp Tyr Phe Tyr Glu Gln
    1400                1405                1410

Val Leu Tyr Ala Leu Glu Gln Asp Asn Phe Cys Asp Phe Glu Ile
    1415                1420                1425

Gln Leu Glu Ile Leu His Asn Ala Leu His Ser Leu Leu Gly Gly
    1430                1435                1440

Lys Gly Lys Tyr Ser Met Ser Asn Leu Asp Tyr Ala Ala Phe Asp
    1445                1450                1455

Pro Val Phe Phe Leu His His Ala Thr Thr Asp Arg Ile Trp Ala
    1460                1465                1470

Ile Trp Gln Asp Leu Gln Arg Phe Arg Lys Arg Pro Tyr Arg Glu
    1475                1480                1485

Ala Asn Cys Ala Ile Gln Leu Met His Thr Pro Leu Gln Pro Phe
    1490                1495                1500

Asp Lys Ser Asp Asn Asn Asp Glu Ala Thr Lys Thr His Ala Thr
    1505                1510                1515

Pro His Asp Gly Phe Glu Tyr Gln Asn Ser Phe Gly Tyr Ala Tyr
    1520                1525                1530

Asp Asn Leu Glu Leu Asn His Tyr Ser Ile Pro Gln Leu Asp His
    1535                1540                1545

Met Leu Gln Glu Arg Lys Arg His Asp Arg Val Phe Ala Gly Phe
    1550                1555                1560

Leu Leu His Asn Ile Gly Thr Ser Ala Asp Gly His Val Phe Val
    1565                1570                1575

Cys Leu Pro Thr Gly Glu His Thr Lys Asp Cys Ser His Glu Ala
    1580                1585                1590

Gly Met Phe Ser Ile Leu Gly Gly Gln Thr Glu Met Ser Phe Val
    1595                1600                1605

Phe Asp Arg Leu Tyr Lys Leu Asp Ile Thr Lys Ala Leu Lys Lys
    1610                1615                1620

Asn Gly Val His Leu Gln Gly Asp Phe Asp Leu Glu Ile Glu Ile
    1625                1630                1635

Thr Ala Val Asn Gly Ser His Leu Asp Ser His Val Ile His Ser
    1640                1645                1650

Pro Thr Ile Leu Phe Glu Ala Gly Thr Asp Ser Ala His Thr Asp
    1655                1660                1665

Asp Gly His Thr Glu Pro Val Met Ile Arg Lys Asp Ile Thr Gln
    1670                1675                1680

Leu Asp Lys Arg Gln Gln Leu Ser Leu Val Lys Ala Leu Glu Ser
    1685                1690                1695

Met Lys Ala Asp His Ser Ser Asp Gly Phe Gln Ala Ile Ala Ser
    1700                1705                1710

Phe His Ala Leu Pro Pro Leu Cys Pro Ser Pro Ala Ala Ser Lys
    1715                1720                1725

Arg Phe Ala Cys Cys Val His Gly Met Ala Thr Phe Pro Gln Trp
    1730                1735                1740

His Arg Leu Tyr Thr Val Gln Phe Gln Asp Ser Leu Arg Lys His
    1745                1750                1755

Gly Ala Val Val Gly Leu Pro Tyr Trp Asp Trp Thr Leu Pro Arg
    1760                1765                1770

Ser Glu Leu Pro Glu Leu Leu Thr Val Ser Thr Ile His Asp Pro
    1775                1780                1785

Glu Thr Gly Arg Asp Ile Pro Asn Pro Phe Ile Gly Ser Lys Ile
    1790                1795                1800

Glu Phe Glu Gly Glu Asn Val His Thr Lys Arg Asp Ile Asn Arg
    1805                1810                1815

Asp Arg Leu Phe Gln Gly Ser Thr Lys Thr His His Asn Trp Phe
    1820                1825                1830

Ile Glu Gln Ala Leu Leu Ala Leu Glu Gln Thr Asn Tyr Cys Asp
    1835                1840                1845

Phe Glu Val Gln Phe Glu Ile Met His Asn Gly Val His Thr Trp
    1850                1855                1860

Val Gly Gly Lys Glu Pro Tyr Gly Ile Gly His Leu His Tyr Ala
    1865                1870                1875

Ser Tyr Asp Pro Leu Phe Tyr Ile His His Ser Gln Thr Asp Arg
    1880                1885                1890

Ile Trp Ala Ile Trp Gln Ser Leu Gln Arg Phe Arg Gly Leu Ser
    1895                1900                1905

Gly Ser Glu Ala Asn Cys Ala Val Asn Leu Met Lys Thr Pro Leu
    1910                1915                1920

Lys Pro Phe Ser Phe Gly Ala Pro Tyr Asn Leu Asn Asp His Thr
    1925                1930                1935

His Asp Phe Ser Lys Pro Glu Asp Thr Phe Asp Tyr Gln Lys Phe
    1940                1945                1950

Gly Tyr Ile Tyr Asp Thr Leu Glu Phe Ala Gly Trp Ser Ile Arg
    1955                1960                1965

Gly Ile Asp His Ile Val Arg Asn Arg Gln Glu His Ser Arg Val
    1970                1975                1980

Phe Ala Gly Phe Leu Leu Glu Gly Phe Gly Thr Ser Ala Thr Val
    1985                1990                1995

Asp Phe Gln Val Cys Arg Thr Ala Gly Asp Cys Glu Asp Ala Gly

2000                    2005                    2010

Tyr Phe Thr Val Leu Gly Gly Glu Lys Glu Met Pro Trp Ala Phe
    2015                    2020                    2025

Asp Arg Leu Tyr Lys Tyr Asp Ile Thr Glu Thr Leu Asp Lys Met
    2030                    2035                    2040

Asn Leu Arg His Asp Glu Ile Phe Gln Ile Glu Val Thr Ile Thr
    2045                    2050                    2055

Ser Tyr Asp Gly Thr Val Leu Asp Ser Gly Leu Ile Pro Thr Pro
    2060                    2065                    2070

Ser Ile Ile Tyr Asp Pro Ala His His Asp Ile Ser Ser His His
    2075                    2080                    2085

Leu Ser Leu Asn Lys Val Arg His Asp Leu Ser Thr Leu Ser Glu
    2090                    2095                    2100

Arg Asp Ile Gly Ser Leu Lys Tyr Ala Leu Ser Ser Leu Gln Ala
    2105                    2110                    2115

Asp Thr Ser Ala Asp Gly Phe Ala Ala Ile Ala Ser Phe His Gly
    2120                    2125                    2130

Leu Pro Ala Lys Cys Asn Asp Ser His Asn Asn Glu Val Ala Cys
    2135                    2140                    2145

Cys Ile His Gly Met Pro Thr Phe Pro His Trp His Arg Leu Tyr
    2150                    2155                    2160

Thr Leu Gln Phe Glu Gln Ala Leu Arg Arg His Gly Ser Ser Val
    2165                    2170                    2175

Ala Val Pro Tyr Trp Asp Trp Thr Lys Pro Ile His Asn Ile Pro
    2180                    2185                    2190

His Leu Phe Thr Asp Lys Glu Tyr Tyr Asp Val Trp Arg Asn Lys
    2195                    2200                    2205

Val Met Pro Asn Pro Phe Ala Arg Gly Tyr Val Pro Ser His Asp
    2210                    2215                    2220

Thr Tyr Thr Val Arg Asp Val Gln Glu Gly Leu Phe His Leu Thr
    2225                    2230                    2235

Ser Thr Gly Glu His Ser Ala Leu Leu Asn Gln Ala Leu Leu Ala
    2240                    2245                    2250

Leu Glu Gln His Asp Tyr Cys Asp Phe Ala Val Gln Phe Glu Val
    2255                2260                2265

Met His Asn Thr Ile His Tyr Leu Val Gly Gly Pro Gln Val Tyr
    2270                2275                2280

Ser Leu Ser Ser Leu His Tyr Ala Ser Tyr Asp Pro Ile Phe Phe
    2285                2290                2295

Ile His His Ser Phe Val Asp Lys Val Trp Ala Val Trp Gln Ala
    2300                2305                2310

Leu Gln Glu Lys Arg Gly Leu Pro Ser Asp Arg Ala Asp Cys Ala
    2315                2320                2325

Val Ser Leu Met Thr Gln Asn Met Arg Pro Phe His Tyr Glu Ile
    2330                2335                2340

Asn His Asn Gln Phe Thr Lys Lys His Ala Val Pro Asn Asp Val
    2345                2350                2355

Phe Lys Tyr Glu Leu Leu Gly Tyr Arg Tyr Asp Asn Leu Glu Ile
    2360                2365                2370

Gly Gly Met Asn Leu His Glu Ile Glu Lys Glu Ile Lys Asp Lys
    2375                2380                2385

Gln His His Val Arg Val Phe Ala Gly Phe Leu Leu His Gly Ile
    2390                2395                2400

Arg Thr Ser Ala Asp Val Gln Phe Gln Ile Cys Lys Thr Ser Glu
    2405                2410                2415

Asp Cys His His Gly Gly Gln Ile Phe Val Leu Gly Gly Thr Lys
    2420                2425                2430

Glu Met Ala Trp Ala Tyr Asn Arg Leu Phe Lys Tyr Asp Ile Thr
    2435                2440                2445

His Ala Leu His Asp Ala His Ile Thr Pro Glu Asp Val Phe His
    2450                2455                2460

Pro Ser Glu Pro Phe Phe Ile Lys Val Ser Val Thr Ala Val Asn
    2465                2470                2475

Gly Thr Val Leu Pro Ala Ser Ile Leu His Ala Pro Thr Ile Ile
    2480                2485                2490

Tyr Glu Pro Gly Leu Asp His His Glu Asp His His Ser Ser Ser
    2495                2500                2505

Met Ala Gly His Gly Val Arg Lys Glu Ile Asn Thr Leu Thr Thr
    2510                2515                2520

Ala Glu Val Asp Asn Leu Lys Asp Ala Met Arg Ala Val Met Ala
    2525                2530                2535

Asp His Gly Pro Asn Gly Tyr Gln Ala Ile Ala Ala Phe His Gly
    2540                2545                2550

Asn Pro Pro Met Cys Pro Met Pro Asp Gly Lys Asn Tyr Ser Cys
    2555                2560                2565

Cys Thr His Gly Met Ala Thr Phe Pro His Trp His Arg Leu Tyr
    2570                2575                2580

Thr Lys Gln Met Glu Asp Ala Leu Thr Ala His Gly Ala Arg Val
    2585                2590                2595

Gly Leu Pro Tyr Trp Asp Gly Thr Thr Ala Phe Thr Ala Leu Pro
    2600                2605                2610

Thr Phe Val Thr Asp Glu Glu Asp Asn Pro Phe His His Gly His
    2615                2620                2625

Ile Asp Tyr Leu Gly Val Asp Thr Thr Arg Ser Pro Arg Asp Lys
    2630                2635                2640

Leu Phe Asn Asp Pro Glu Arg Gly Ser Glu Ser Phe Phe Tyr Arg
    2645                2650                2655

Gln Val Leu Leu Ala Leu Glu Gln Thr Asp Phe Cys Gln Phe Glu
    2660                2665                2670

Val Gln Phe Glu Ile Thr His Asn Ala Ile His Ser Trp Thr Gly
    2675                2680                2685

Gly Leu Thr Pro Tyr Gly Met Ser Thr Leu Glu Tyr Thr Thr Tyr
    2690                2695                2700

Asp Pro Leu Phe Trp Leu His His Ala Asn Thr Asp Arg Ile Trp
    2705                2710                2715

Ala Ile Trp Gln Ala Leu Gln Glu Tyr Arg Gly Leu Pro Tyr Asp
    2720                2725                2730

His Ala Asn Cys Glu Ile Gln Ala Met Lys Arg Pro Leu Arg Pro
    2735                2740                2745

Phe Ser Asp Pro Ile Asn His Asn Ala Phe Thr His Ser Asn Ala
    2750                2755                2760

Lys Pro Thr Asp Val Phe Glu Tyr Ser Arg Phe Asn Phe Gln Tyr
    2765                2770                2775

Asp Asn Leu Arg Phe His Gly Met Thr Ile Lys Lys Leu Glu His
    2780                2785                2790

Glu Leu Glu Lys Gln Lys Glu Glu Asp Arg Thr Phe Ala Ala Phe
    2795                2800                2805

Leu Leu His Gly Ile Lys Lys Ser Ala Asp Val Ser Phe Asp Val
    2810                2815                2820

Cys Asn His Asp Gly Glu Cys His Phe Ala Gly Thr Phe Ala Ile
    2825                2830                2835

Leu Gly Gly Glu His Glu Met Pro Trp Ser Phe Asp Arg Leu Phe
    2840                2845                2850

Arg Tyr Asp Ile Thr Gln Val Leu Lys Gln Met His Leu Glu Tyr
    2855                2860                2865

Asp Ser Asp Phe Thr Phe His Met Arg Ile Ile Asp Thr Ser Gly
    2870                2875                2880

Lys Gln Leu Pro Ser Asp Leu Ile Lys Met Pro Thr Val Glu His
    2885                2890                2895

Ser Pro Gly Gly Lys His His Glu Lys His His Glu Asp His His
    2900                2905                2910

Glu Asp Ile Leu Val Arg Lys Asn Ile His Ser Leu Ser His His
    2915                2920                2925

Glu Ala Glu Glu Leu Arg Asp Ala Leu Tyr Lys Leu Gln Asn Asp
    2930                2935                2940

Glu Ser His Gly Gly Tyr Glu His Ile Ala Gly Phe His Gly Tyr
    2945                2950                2955

Pro Asn Leu Cys Pro Glu Lys Gly Asp Glu Lys Tyr Pro Cys Cys
    2960                2965                2970

Val His Gly Met Ser Ile Phe Pro His Trp His Arg Leu His Thr
    2975                2980                2985

Ile Gln Phe Glu Arg Ala Leu Lys Lys His Gly Ser His Leu Gly
    2990                2995                3000

Ile Pro Tyr Trp Asp Trp Thr Gln Thr Ile Ser Ser Leu Pro Thr

3005                3010                3015

Phe Phe Ala Asp Ser Gly Asn Asn Asn Pro Phe Phe Lys Tyr His
    3020                3025                3030

Ile Arg Ser Ile Asn Gln Asp Thr Val Arg Asp Val Asn Glu Ala
    3035                3040                3045

Ile Phe Gln Gln Thr Lys Phe Gly Glu Phe Ser Ser Ile Phe Tyr
    3050                3055                3060

Leu Ala Leu Gln Ala Leu Glu Glu Asp Asn Tyr Cys Asp Phe Glu
    3065                3070                3075

Val Gln Tyr Glu Ile Leu His Asn Glu Val His Ala Leu Ile Gly
    3080                3085                3090

Gly Ala Glu Lys Tyr Ser Met Ser Thr Leu Glu Tyr Ser Ala Phe
    3095                3100                3105

Asp Pro Tyr Phe Met Ile His His Ala Ser Leu Asp Lys Ile Trp
    3110                3115                3120

Ile Ile Trp Gln Glu Leu Gln Lys Arg Arg Val Lys Pro Ala His
    3125                3130                3135

Ala Gly Ser Cys Ala Gly Asp Ile Met His Val Pro Leu His Pro
    3140                3145                3150

Phe Asn Tyr Glu Ser Val Asn Asn Asp Asp Phe Thr Arg Glu Asn
    3155                3160                3165

Ser Leu Pro Asn Ala Val Val Asp Ser His Arg Phe Asn Tyr Lys
    3170                3175                3180

Tyr Asp Asn Leu Asn Leu His Gly His Asn Ile Glu Glu Leu Glu
    3185                3190                3195

Glu Val Leu Arg Ser Leu Arg Leu Lys Ser Arg Val Phe Ala Gly
    3200                3205                3210

Phe Val Leu Ser Gly Ile Arg Thr Thr Ala Val Val Lys Val Tyr
    3215                3220                3225

Ile Lys Ser Gly Thr Asp Ser Asp Asp Glu Tyr Ala Gly Ser Phe
    3230                3235                3240

Val Ile Leu Gly Gly Ala Lys Glu Met Pro Trp Ala Tyr Glu Arg
    3245                3250                3255

Leu Tyr Arg Phe Asp Ile Thr Glu Thr Val His Asn Leu Asn Leu
    3260                3265                3270

Thr Asp Asp His Val Lys Phe Arg Phe Asp Leu Lys Lys Tyr Asp
    3275                3280                3285

His Thr Glu Leu Asp Ala Ser Val Leu Pro Ala Pro Ile Ile Val
    3290                3295                3300

Arg Arg Pro Asn Asn Ala Val Phe Asp Ile Ile Glu Ile Pro Ile
    3305                3310                3315

Gly Lys Asp Val Asn Leu Pro Pro Lys Val Val Val Lys Arg Gly
    3320                3325                3330

Thr Lys Ile Met Phe Met Ser Val Asp Glu Ala Val Thr Thr Pro
    3335                3340                3345

Met Leu Asn Leu Gly Ser Tyr Thr Ala Met Phe Lys Cys Lys Val
    3350                3355                3360

Pro Pro Phe Ser Phe His Ala Phe Glu Leu Gly Lys Met Tyr Ser
    3365                3370                3375

Val Glu Ser Gly Asp Tyr Phe Met Thr Ala Ser Thr Thr Glu Leu
    3380                3385                3390

Cys Asn Asp Asn Asn Leu Arg Ile His Val His Val Asp Asp Glu
    3395                3400                3405

<210> 2
<211> 3421
<212> PRT
<213> Megathura crenulata

<400> 2

Met Trp Thr Ile Leu Ala Leu Leu Thr Ala Thr Leu Leu Phe Glu Gly
1               5                   10                  15

Ala Phe Ser Val Asp Thr Val Val Arg Lys Asn Val Asp Ser Leu Ser
            20                  25                  30

Ser Asp Glu Val Leu Ala Leu Glu Lys Ala Leu Asp Asp Leu Gln Gln
        35                  40                  45

Asp Asp Ser Asn Gln Gly Tyr Gln Ala Ile Ala Gly Tyr His Gly Val
    50                  55                  60

Pro Thr Met Cys Val Asp Lys His Glu Lys Asn Val Ala Cys Cys Leu
65                  70                  75                  80

His Gly Met Pro Ser Phe Pro Leu Trp His Arg Leu Tyr Val Val Gln
                85                  90                  95

Leu Glu Arg Ala Leu Ile Arg Lys Lys Ala Thr Ile Ser Ile Pro Tyr
            100                 105                 110

Trp Asp Trp Thr Ser Glu Leu Thr His Leu Pro Glu Leu Val Ser His
        115                 120                 125

Pro Leu Phe Val Gly Thr Glu Gly Gly Lys Ala His Asp Asn Ser Trp
    130                 135                 140

Tyr Arg Ala Asp Ile Thr Phe Leu Asn Lys Lys Thr Ser Arg Ala Val
145                 150                 155                 160

Asp Asp Arg Leu Phe Glu Lys Val Gln Pro Gly His His Thr Arg Leu
                165                 170                 175

Met Glu Gly Ile Leu Asp Ala Leu Glu Gln Asp Glu Phe Cys Lys Phe
            180                 185                 190

Glu Ile Gln Phe Glu Leu Ala His Asn Ala Ile His Tyr Leu Val Gly
        195                 200                 205

Gly Arg His Thr Tyr Ser Met Ser His Leu Glu Tyr Thr Ser Tyr Asp
    210                 215                 220

Pro Leu Phe Phe Leu His His Ser Asn Thr Asp Arg Ile Phe Ala Ile
225                 230                 235                 240

Trp Gln Arg Leu Gln Gln Leu Arg Gly Lys Asp Pro Asn Ser Ala Asp
                245                 250                 255

Cys Ala His Asn Leu Ile His Thr Pro Met Glu Pro Phe Asp Arg Asp
            260                 265                 270

Thr Asn Pro Leu Asp Leu Thr Arg Glu His Ala Lys Pro Ala Asp Ser
        275                 280                 285

Phe Asp Tyr Gly Arg Leu Gly Tyr Gln Tyr Asp Asp Leu Ser Leu Asn
    290                 295                 300

Gly Met Ser Pro Glu Glu Leu Asn Val Tyr Leu Gly Glu Arg Ala Ala
305                 310                 315                 320

Lys Glu Arg Thr Phe Ala Ser Phe Ile Leu Ser Gly Phe Gly Gly Ser
                325                 330                 335

Ala Asn Val Val Val Tyr Val Cys Arg Pro Ala His Asp Glu Ile Ser
            340                 345                 350

Asp Asp Gln Cys Ile Lys Ala Gly Asp Phe Phe Leu Leu Gly Gly Pro
        355                 360                 365

Thr Glu Met Lys Trp Gly Phe Tyr Arg Ala Tyr His Phe Asp Val Thr
    370                 375                 380

Asp Ser Val Ala Ser Ile Asp Asp Asp Gly His Gly His Tyr Tyr Val
385                 390                 395                 400

Lys Ser Glu Leu Phe Ser Val Asn Gly Ser Ala Leu Ser Asn Asp Ile
                405                 410                 415

Leu Arg Gln Pro Thr Leu Val His Arg Pro Ala Lys Gly His Phe Asp
            420                 425                 430

Lys Pro Pro Val Pro Val Ala Gln Ala Asn Leu Ala Val Arg Lys Asn
        435                 440                 445

Ile Asn Asp Leu Thr Ala Glu Glu Thr Tyr Ser Leu Arg Lys Ala Met
    450                 455                 460

Glu Arg Phe Gln Asn Asp Lys Ser Val Asp Gly Tyr Gln Ala Thr Val
465                 470                 475                 480

Glu Phe His Ala Leu Pro Ala Arg Cys Pro Arg Pro Asp Ala Lys Asp
                485                 490                 495

Arg Phe Ala Cys Cys Val His Gly Met Ala Thr Phe Pro His Trp His
            500                 505                 510

Arg Leu Phe Val Thr Gln Val Glu Asp Ala Leu Leu Arg Arg Gly Ser
        515                 520                 525

Thr Ile Gly Leu Pro Asn Trp Asp Trp Thr Met Pro Met Asp His Leu
    530                 535                 540

Pro Glu Leu Ala Thr Ser Glu Thr Tyr Leu Asp Pro Val Thr Gly Glu
545                 550                 555                 560

Thr Lys Asn Asn Pro Phe His His Ala Gln Val Ala Phe Glu Asn Gly
                565                 570                 575

Val Thr Ser Arg Asn Pro Asp Ala Lys Leu Phe Met Lys Pro Thr Tyr
            580                 585                 590

Gly Asp His Thr Tyr Leu Phe Asp Ser Met Ile Tyr Ala Phe Glu Gln
        595                 600                 605

Glu Asp Phe Cys Asp Phe Glu Val Gln Tyr Glu Leu Thr His Asn Ala
    610                 615                 620

Ile His Ala Trp Val Gly Gly Ser Glu Lys Tyr Ser Met Ser Ser Leu
625                 630                 635                 640

His Tyr Thr Ala Phe Asp Pro Ile Phe Tyr Leu His His Ser Asn Val
                645                 650                 655

Asp Arg Leu Trp Ala Ile Trp Gln Ala Leu Gln Ile Arg Arg Gly Lys
            660                 665                 670

Ser Tyr Lys Ala His Cys Ala Ser Ser Gln Glu Arg Glu Pro Leu Lys
        675                 680                 685

Pro Phe Ala Phe Ser Ser Pro Leu Asn Asn Asn Glu Lys Thr Tyr His
    690                 695                 700

Asn Ser Val Pro Thr Asn Val Tyr Asp Tyr Val Gly Val Leu His Tyr
705                 710                 715                 720

Arg Tyr Asp Asp Leu Gln Phe Gly Gly Met Thr Met Ser Glu Leu Glu
                725                 730                 735

Glu Tyr Ile His Lys Gln Thr Gln His Asp Arg Thr Phe Ala Gly Phe
            740                 745                 750

Phe Leu Ser Tyr Ile Gly Thr Ser Ala Ser Val Asp Ile Phe Ile Asn
        755                 760                 765

Arg Glu Gly His Asp Lys Tyr Lys Val Gly Ser Phe Val Val Leu Gly
    770                 775                 780

Gly Ser Lys Glu Met Lys Trp Gly Phe Asp Arg Met Tyr Lys Tyr Glu
785                 790                 795                 800

Ile Thr Glu Ala Leu Lys Thr Leu Asn Val Ala Val Asp Asp Gly Phe
                805                 810                 815

Ser Ile Thr Val Glu Ile Thr Asp Val Asp Gly Ser Pro Pro Ser Ala
            820                 825                 830

Asp Leu Ile Pro Pro Pro Ala Ile Ile Phe Glu Arg Ala Asp Ala Lys
        835                 840                 845

Asp Phe Gly His Ser Arg Lys Ile Arg Lys Ala Val Asp Ser Leu Thr
    850                 855                 860

Val Glu Glu Gln Thr Ser Leu Arg Arg Ala Met Ala Asp Leu Gln Asp
865                 870                 875                 880

Asp Lys Thr Ser Gly Gly Phe Gln Gln Ile Ala Ala Phe His Gly Glu

                885                 890                 895

Pro Lys Trp Cys Pro Ser Pro Glu Ala Glu Lys Lys Phe Ala Cys Cys
            900                 905                 910

Val His Gly Met Ala Val Phe Pro His Trp His Arg Leu Leu Thr Val
        915                 920                 925

Gln Gly Glu Asn Ala Leu Arg Lys His Gly Phe Thr Gly Gly Leu Pro
    930                 935                 940

Tyr Trp Asp Trp Thr Arg Ser Met Ser Ala Leu Pro His Phe Val Ala
945                 950                 955                 960

Asp Pro Thr Tyr Asn Asp Ala Ile Ser Ser Gln Glu Glu Asp Asn Pro
                965                 970                 975

Trp His His Gly His Ile Asp Ser Val Gly His Asp Thr Thr Arg Asp
            980                 985                 990

Val Arg Asp Asp Leu Tyr Gln Ser Pro Gly Phe Gly His Tyr Thr Asp
        995                 1000                1005

Ile Ala Lys Gln Val Leu Leu Ala Phe Glu Gln Asp Asp Phe Cys
    1010                1015                1020

Asp Phe Glu Val Gln Phe Glu Ile Ala His Asn Phe Ile His Ala
    1025                1030                1035

Leu Val Gly Gly Asn Glu Pro Tyr Ser Met Ser Ser Leu Arg Tyr
    1040                1045                1050

Thr Thr Tyr Asp Pro Ile Phe Phe Leu His Arg Ser Asn Thr Asp
    1055                1060                1065

Arg Leu Trp Ala Ile Trp Gln Ala Leu Gln Lys Tyr Arg Gly Lys
    1070                1075                1080

Pro Tyr Asn Thr Ala Asn Cys Ala Ile Ala Ser Met Arg Lys Pro
    1085                1090                1095

Leu Gln Pro Phe Gly Leu Asp Ser Val Ile Asn Pro Asp Asp Glu
    1100                1105                1110

Thr Arg Glu His Ser Val Pro Phe Arg Val Phe Asp Tyr Lys Asn
    1115                1120                1125

Asn Phe Asp Tyr Glu Tyr Glu Ser Leu Ala Phe Asn Gly Leu Ser
    1130                1135                1140

Ile Ala Gln Leu Asp Arg Glu Leu Gln Arg Arg Lys Ser His Asp
    1145                1150                1155

Arg Val Phe Ala Gly Phe Leu Leu His Glu Ile Gly Gln Ser Ala
    1160                1165                1170

Leu Val Lys Phe Tyr Val Cys Lys His Asn Val Ser Asp Cys Asp
    1175                1180                1185

His Tyr Ala Gly Glu Phe Tyr Ile Leu Gly Asp Glu Ala Glu Met
    1190                1195                1200

Pro Trp Arg Tyr Asp Arg Val Tyr Lys Tyr Glu Ile Thr Gln Gln
    1205                1210                1215

Leu His Asp Leu Asp Leu His Val Gly Asp Asn Phe Phe Leu Lys
    1220                1225                1230

Tyr Glu Ala Phe Asp Leu Asn Gly Gly Ser Leu Gly Gly Ser Ile
    1235                1240                1245

Phe Ser Gln Pro Ser Val Ile Phe Glu Pro Ala Ala Gly Ser His
    1250                1255                1260

Gln Ala Asp Glu Tyr Arg Glu Ala Val Thr Ser Ala Ser His Ile
    1265                1270                1275

Arg Lys Asn Ile Arg Asp Leu Ser Glu Gly Glu Ile Glu Ser Ile
    1280                1285                1290

Arg Ser Ala Phe Leu Gln Ile Gln Lys Glu Gly Ile Tyr Glu Asn
    1295                1300                1305

Ile Ala Lys Phe His Gly Lys Pro Gly Leu Cys Glu His Asp Gly
    1310                1315                1320

His Pro Val Ala Cys Cys Val His Gly Met Pro Thr Phe Pro His
    1325                1330                1335

Trp His Arg Leu Tyr Val Leu Gln Val Glu Asn Ala Leu Leu Glu
    1340                1345                1350

Arg Gly Ser Ala Val Ala Val Pro Tyr Trp Asp Trp Thr Glu Lys
    1355                1360                1365

Ala Asp Ser Leu Pro Ser Leu Ile Asn Asp Ala Thr Tyr Phe Asn
    1370                1375                1380

Ser Arg Ser Gln Thr Phe Asp Pro Asn Pro Phe Phe Arg Gly His
    1385                1390                1395

Ile Ala Phe Glu Asn Ala Val Thr Ser Arg Asp Pro Gln Pro Glu
    1400                1405                1410

Leu Trp Asp Asn Lys Asp Phe Tyr Glu Asn Val Met Leu Ala Leu
    1415                1420                1425

Glu Gln Asp Asn Phe Cys Asp Phe Glu Ile Gln Leu Glu Leu Ile
    1430                1435                1440

His Asn Ala Leu His Ser Arg Leu Gly Gly Arg Ala Lys Tyr Ser
    1445                1450                1455

Leu Ser Ser Leu Asp Tyr Thr Ala Phe Asp Pro Val Phe Phe Leu
    1460                1465                1470

His His Ala Asn Val Asp Arg Ile Trp Ala Ile Trp Gln Asp Leu
    1475                1480                1485

Gln Arg Tyr Arg Lys Lys Pro Tyr Asn Glu Ala Asp Cys Ala Val
    1490                1495                1500

Asn Glu Met Arg Lys Pro Leu Gln Pro Phe Asn Asn Pro Glu Leu
    1505                1510                1515

Asn Ser Asp Ser Met Thr Leu Lys His Asn Leu Pro Gln Asp Ser
    1520                1525                1530

Phe Asp Tyr Gln Asn Arg Phe Arg Tyr Gln Tyr Asp Asn Leu Gln
    1535                1540                1545

Phe Asn His Phe Ser Ile Gln Lys Leu Asp Gln Thr Ile Gln Ala
    1550                1555                1560

Arg Lys Gln His Asp Arg Val Phe Ala Gly Phe Ile Leu His Asn
    1565                1570                1575

Ile Gly Thr Ser Ala Val Val Asp Ile Tyr Ile Cys Val Glu Gln
    1580                1585                1590

Gly Gly Glu Gln Asn Cys Lys Thr Lys Ala Gly Ser Phe Thr Ile
    1595                1600                1605

Leu Gly Gly Glu Thr Glu Met Pro Phe His Phe Asp Arg Leu Tyr
    1610                1615                1620

Lys Phe Asp Ile Thr Ser Ala Leu His Lys Leu Gly Val Pro Leu
    1625                1630                1635

Asp Gly His Gly Phe Asp Ile Lys Val Asp Val Arg Ala Val Asn
    1640                1645                1650

Gly Ser His Leu Asp Gln His Ile Leu Asn Glu Pro Ser Leu Leu
    1655                1660                1665

Phe Val Pro Gly Glu Arg Lys Asn Ile Tyr Tyr Asp Gly Leu Ser
    1670                1675                1680

Gln His Asn Leu Val Arg Lys Glu Val Ser Ser Leu Thr Thr Leu
    1685                1690                1695

Glu Lys His Phe Leu Arg Lys Ala Leu Lys Asn Met Gln Ala Asp
    1700                1705                1710

Asp Ser Pro Asp Gly Tyr Gln Ala Ile Ala Ser Phe His Ala Leu
    1715                1720                1725

Pro Pro Leu Cys Pro Ser Pro Ser Ala Ala His Arg His Ala Cys
    1730                1735                1740

Cys Leu His Gly Met Ala Thr Phe Pro Gln Trp His Arg Leu Tyr
    1745                1750                1755

Thr Val Gln Phe Glu Asp Ser Leu Lys Arg His Gly Ser Ile Val
    1760                1765                1770

Gly Leu Pro Tyr Trp Asp Trp Leu Lys Pro Gln Ser Ala Leu Pro
    1775                1780                1785

Asp Leu Val Thr Gln Glu Thr Tyr Glu His Leu Phe Ser His Lys
    1790                1795                1800

Thr Phe Pro Asn Pro Phe Leu Lys Ala Asn Ile Glu Phe Glu Gly
    1805                1810                1815

Glu Gly Val Thr Thr Glu Arg Asp Val Asp Ala Glu His Leu Phe
    1820                1825                1830

Ala Lys Gly Asn Leu Val Tyr Asn Asn Trp Phe Cys Asn Gln Ala
    1835                1840                1845

Leu Tyr Ala Leu Glu Gln Glu Asn Tyr Cys Asp Phe Glu Ile Gln
    1850                1855                1860

Phe Glu Ile Leu His Asn Gly Ile His Ser Trp Val Gly Gly Ser
    1865                1870                1875

Lys Thr His Ser Ile Gly His Leu His Tyr Ala Ser Tyr Asp Pro
    1880                1885                1890

Leu Phe Tyr Ile His His Ser Gln Thr Asp Arg Ile Trp Ala Ile

1895                1900                1905

Trp Gln  Ala Leu Gln Glu His  Arg Gly Leu Ser Gly  Lys Glu Ala
    1910                1915                1920

His Cys  Ala Leu Glu Gln Met  Lys Asp Pro Leu Lys  Pro Phe Ser
    1925                1930                1935

Phe Gly  Ser Pro Tyr Asn Leu  Asn Lys Arg Thr Gln  Glu Phe Ser
    1940                1945                1950

Lys Pro  Glu Asp Thr Phe Asp  Tyr His Arg Phe Gly  Tyr Glu Tyr
    1955                1960                1965

Asp Ser  Leu Glu Phe Val Gly  Met Ser Val Ser Ser  Leu His Asn
    1970                1975                1980

Tyr Ile  Lys Gln Gln Gln Glu  Ala Asp Arg Val Phe  Ala Gly Phe
    1985                1990                1995

Leu Leu  Lys Gly Phe Gly Gln  Ser Ala Ser Val Ser  Phe Asp Ile
    2000                2005                2010

Cys Arg  Pro Asp Gln Ser Cys  Gln Glu Ala Gly Tyr  Phe Ser Val
    2015                2020                2025

Leu Gly  Gly Ser Ser Glu Met  Pro Trp Gln Phe Asp  Arg Leu Tyr
    2030                2035                2040

Lys Tyr  Asp Ile Thr Lys Thr  Leu Lys Asp Met Lys  Leu Arg Tyr
    2045                2050                2055

Asp Asp  Thr Phe Thr Ile Lys  Val His Ile Lys Asp  Ile Ala Gly
    2060                2065                2070

Ala Glu  Leu Asp Ser Asp Leu  Ile Pro Thr Pro Ser  Val Leu Leu
    2075                2080                2085

Glu Glu  Gly Lys His Gly Ile  Asn Val Arg His Val  Gly Arg Asn
    2090                2095                2100

Arg Ile  Arg Met Glu Leu Ser  Glu Leu Thr Glu Arg  Asp Leu Ala
    2105                2110                2115

Ser Leu  Lys Ser Ala Met Arg  Ser Leu Gln Ala Asp  Asp Gly Val
    2120                2125                2130

Asn Gly  Tyr Gln Ala Ile Ala  Ser Phe His Gly Leu  Pro Ala Ser
    2135                2140                2145

Cys His Asp Asp Glu Gly His Glu Ile Ala Cys Cys Ile His Gly
    2150                2155                2160

Met Pro Val Phe Pro His Trp His Arg Leu Tyr Thr Leu Gln Met
    2165                2170                2175

Asp Met Ala Leu Leu Ser His Gly Ser Ala Val Ala Ile Pro Tyr
    2180                2185                2190

Trp Asp Trp Thr Lys Pro Ile Ser Lys Leu Pro Asp Leu Phe Thr
    2195                2200                2205

Ser Pro Glu Tyr Tyr Asp Pro Trp Arg Asp Ala Val Val Asn Asn
    2210                2215                2220

Pro Phe Ala Lys Gly Tyr Ile Lys Ser Glu Asp Ala Tyr Thr Val
    2225                2230                2235

Arg Asp Pro Gln Asp Ile Leu Tyr His Leu Gln Asp Glu Thr Gly
    2240                2245                2250

Thr Ser Val Leu Leu Asp Gln Thr Leu Leu Ala Leu Glu Gln Thr
    2255                2260                2265

Asp Phe Cys Asp Phe Glu Val Gln Phe Glu Val Val His Asn Ala
    2270                2275                2280

Ile His Tyr Leu Val Gly Gly Arg Gln Val Tyr Ala Leu Ser Ser
    2285                2290                2295

Gln His Tyr Ala Ser Tyr Asp Pro Ala Phe Phe Ile His His Ser
    2300                2305                2310

Phe Val Asp Lys Ile Trp Ala Val Trp Gln Ala Leu Gln Lys Lys
    2315                2320                2325

Arg Lys Arg Pro Tyr His Lys Ala Asp Cys Ala Leu Asn Met Met
    2330                2335                2340

Thr Lys Pro Met Arg Pro Phe Ala His Asp Phe Asn His Asn Gly
    2345                2350                2355

Phe Thr Lys Met His Ala Val Pro Asn Thr Leu Phe Asp Phe Gln
    2360                2365                2370

Asp Leu Phe Tyr Thr Tyr Asp Asn Leu Glu Ile Ala Gly Met Asn
    2375                2380                2385

Val Asn Gln Leu Glu Ala Glu Ile Asn Arg Arg Lys Ser Gln Thr
    2390                2395                2400

Arg Val Phe Ala Gly Phe Leu Leu His Gly Ile Gly Arg Ser Ala
    2405                2410                2415

Asp Val Arg Phe Trp Ile Cys Lys Thr Ala Asp Asp Cys His Ala
    2420                2425                2430

Ser Gly Met Ile Phe Ile Leu Gly Gly Ser Lys Glu Met His Trp
    2435                2440                2445

Ala Tyr Asp Arg Asn Phe Lys Tyr Asp Ile Thr Gln Ala Leu Lys
    2450                2455                2460

Ala Gln Ser Ile His Pro Glu Asp Val Phe Asp Thr Asp Ala Pro
    2465                2470                2475

Phe Phe Ile Lys Val Glu Val His Gly Val Asn Lys Thr Ala Leu
    2480                2485                2490

Pro Ser Ser Ala Ile Pro Ala Pro Thr Ile Ile Tyr Ser Ala Gly
    2495                2500                2505

Glu Gly His Thr Asp Asp His Gly Ser Asp His Ile Ala Gly Ser
    2510                2515                2520

Gly Val Arg Lys Asp Val Thr Ser Leu Thr Ala Ser Glu Ile Glu
    2525                2530                2535

Asn Leu Arg His Ala Leu Gln Ser Val Met Asp Asp Asp Gly Pro
    2540                2545                2550

Asn Gly Phe Gln Ala Ile Ala Ala Tyr His Gly Ser Pro Pro Met
    2555                2560                2565

Cys His Met His Asp Gly Arg Asp Val Ala Cys Cys Thr His Gly
    2570                2575                2580

Met Ala Ser Phe Pro His Trp His Arg Leu Phe Val Lys Gln Met
    2585                2590                2595

Glu Asp Ala Leu Ala Ala His Gly Ala His Ile Gly Ile Pro Tyr
    2600                2605                2610

Trp Asp Trp Thr Ser Ala Phe Ser His Leu Pro Ala Leu Val Thr
    2615                2620                2625

Asp His Glu His Asn Pro Phe His His Gly His Ile Ala His Arg
    2630                2635                2640

Asn Val Asp Thr Ser Arg Ser Pro Arg Asp Met Leu Phe Asn Asp
    2645                2650                2655

Pro Glu His Gly Ser Glu Ser Phe Phe Tyr Arg Gln Val Leu Leu
    2660                2665                2670

Ala Leu Glu Gln Thr Asp Phe Cys Gln Phe Glu Val Gln Phe Glu
    2675                2680                2685

Ile Thr His Asn Ala Ile His Ser Trp Thr Gly Gly His Thr Pro
    2690                2695                2700

Tyr Gly Met Ser Ser Leu Glu Tyr Thr Ala Tyr Asp Pro Leu Phe
    2705                2710                2715

Tyr Leu His His Ser Asn Thr Asp Arg Ile Trp Ala Ile Trp Gln
    2720                2725                2730

Ala Leu Gln Lys Tyr Arg Gly Phe Gln Tyr Asn Ala Ala His Cys
    2735                2740                2745

Asp Ile Gln Val Leu Lys Gln Pro Leu Lys Pro Phe Ser Glu Ser
    2750                2755                2760

Arg Asn Pro Asn Pro Val Thr Arg Ala Asn Ser Arg Ala Val Asp
    2765                2770                2775

Ser Phe Asp Tyr Glu Arg Leu Asn Tyr Gln Tyr Asp Thr Leu Thr
    2780                2785                2790

Phe His Gly His Ser Ile Ser Glu Leu Asp Ala Met Leu Gln Glu
    2795                2800                2805

Arg Lys Lys Glu Glu Arg Thr Phe Ala Ala Phe Leu Leu His Gly
    2810                2815                2820

Phe Gly Ala Ser Ala Asp Val Ser Phe Asp Val Cys Thr Pro Asp
    2825                2830                2835

Gly His Cys Ala Phe Ala Gly Thr Phe Ala Val Leu Gly Gly Glu
    2840                2845                2850

Leu Glu Met Pro Trp Ser Phe Glu Arg Leu Phe Arg Tyr Asp Ile
    2855                2860                2865

Thr Lys Val Leu Lys Gln Met Asn Leu His Tyr Asp Ser Glu Phe
    2870                2875                2880

His Phe Glu Leu Lys Ile Val Gly Thr Asp Gly Thr Glu Leu Pro
    2885                2890                2895

Ser Asp Arg Ile Lys Ser Pro Thr Ile Glu His His Gly Gly Asp

2900                2905                2910

His His Gly Gly Asp Thr Ser Gly His Asp His Ser Glu Arg His
    2915                2920                2925

Asp Gly Phe Phe Arg Lys Glu Val Gly Ser Leu Ser Leu Asp Glu
    2930                2935                2940

Ala Asn Asp Leu Lys Asn Ala Leu Tyr Lys Leu Gln Asn Asp Gln
    2945                2950                2955

Gly Pro Asn Gly Tyr Glu Ser Ile Ala Gly Tyr His Gly Tyr Pro
    2960                2965                2970

Phe Leu Cys Pro Glu His Gly Glu Asp Gln Tyr Ala Cys Cys Val
    2975                2980                2985

His Gly Met Pro Val Phe Pro His Trp His Arg Leu His Thr Ile
    2990                2995                3000

Gln Phe Glu Arg Ala Leu Lys Glu His Gly Ser His Leu Gly Ile
    3005                3010                3015

Pro Tyr Trp Asp Trp Thr Lys Ser Met Ile Ala Leu Pro Ala Phe
    3020                3025                3030

Phe Ala Asp Ser Ser Asn Ser Asn Pro Phe Tyr Lys Tyr His Ile
    3035                3040                3045

Met Lys Ala Gly His Asp Thr Ala Arg Ser Pro Ser Asp Leu Leu
    3050                3055                3060

Phe Asn Gln Pro Gln Leu His Gly Tyr Asp Tyr Leu Tyr Tyr Leu
    3065                3070                3075

Ala Leu Ser Thr Leu Glu Glu Asp Asn Tyr Cys Asp Phe Glu Val
    3080                3085                3090

His Tyr Glu Ile Leu His Asn Ala Val His Leu Trp Leu Gly Gly
    3095                3100                3105

Thr Glu Thr Tyr Ser Met Ser Ser Leu Ala Phe Ser Ala Tyr Asp
    3110                3115                3120

Pro Val Phe Met Ile Leu His Ser Gly Leu Asp Arg Leu Trp Ile
    3125                3130                3135

Ile Trp Gln Glu Leu Gln Lys Leu Arg Lys Lys Pro Tyr Asn Ala
    3140                3145                3150

Ala Lys Cys Ala Gly His Met Met Asp Glu Pro Leu His Pro Phe
    3155                3160                3165

Asn Tyr Glu Ser Ala Asn His Asp Ser Phe Thr Arg Ala Asn Ala
    3170                3175                3180

Lys Pro Ser Thr Val Phe Asp Ser His Lys Phe Asn Tyr His Tyr
    3185                3190                3195

Asp Asn Pro Asp Val Arg Gly Asn Ser Ile Gln Glu Ile Ser Ala
    3200                3205                3210

Ile Ile His Asp Leu Arg Asn Gln Pro Arg Val Phe Ala Gly Phe
    3215                3220                3225

Val Leu Ser Gly Ile Tyr Thr Ser Ala Asn Val Lys Ile Tyr Leu
    3230                3235                3240

Val Arg Glu Gly His Asp Asp Glu Asn Val Gly Ser Phe Val Val
    3245                3250                3255

Leu Gly Gly Pro Lys Glu Met Pro Trp Ala Tyr Glu Arg Ile Phe
    3260                3265                3270

Lys Tyr Asp Ile Thr Glu Val Ala Asn Arg Leu Asn Met His His
    3275                3280                3285

Asp Asp Thr Phe Asn Phe Arg Leu Glu Val Gln Ser Tyr Thr Gly
    3290                3295                3300

Glu Met Val Thr His His Leu Pro Glu Pro Leu Ile Ile Tyr Arg
    3305                3310                3315

Pro Ala Lys Gln Glu Tyr Asp Val Leu Val Ile Pro Leu Gly Ser
    3320                3325                3330

Gly His Lys Leu Pro Pro Lys Val Ile Val Lys Arg Gly Thr Arg
    3335                3340                3345

Ile Met Phe His Pro Val Asp Asp Thr Val Asn Arg Pro Val Val
    3350                3355                3360

Asp Leu Gly Ser His Thr Ala Leu Tyr Asn Cys Val Val Pro Pro
    3365                3370                3375

Phe Thr Tyr Asn Gly Tyr Glu Leu Asp His Ala Tyr Ser Leu Arg
    3380                3385                3390

Asp Gly His Tyr Tyr Ile Ala Gly Pro Thr Lys Asp Leu Cys Thr
    3395                3400                3405

Ser Gly Asn Val Arg Ile His Ile His Ile Glu Asp Glu
    3410                3415                3420

<210> 3
<211> 243
<212> PRT
<213> artificial

<220>
<223> T038 light chain

<400> 3

Met Gly Lys Gln Met Ala Ala Leu Cys Gly Phe Leu Leu Val Ala Leu
1               5                   10                  15

Leu Trp Leu Thr Pro Asp Val Ala Ser Gly Gln Ser Ala Leu Thr Gln
            20                  25                  30

Pro Ala Ser Val Ser Gly Ser Leu Gly Gln Ser Ile Thr Ile Thr Cys
        35                  40                  45

Ser Gly Ile Thr Thr Asp Ile Asn Ile Tyr Asn Leu Val Ser Trp Tyr
    50                  55                  60

Gln Gln His Pro Asp Lys Ala Pro Lys Leu Ile Ile Tyr Glu Val Asn
65                  70                  75                  80

Lys Arg Pro Ser Gly Val Ser Asp Arg Phe Ser Gly Ser Lys Ser Gly
                85                  90                  95

Asn Thr Ala Ser Leu Ile Ile Ser Gly Leu Gln Ala Glu Asp Glu Ala
            100                 105                 110

Asp Tyr His Cys Cys Ala Tyr Gly Pro Arg Ser Ser Ser Ala Val Phe
        115                 120                 125

Gly Gly Gly Thr His Leu Thr Val Leu Gly Gln Pro Lys Ala Asn Pro
    130                 135                 140

Thr Val Thr Leu Phe Pro Pro Ser Ser Glu Glu Leu Gln Ala Asn Lys
145                 150                 155                 160

Ala Thr Leu Val Cys Leu Ile Ser Asp Phe Tyr Pro Gly Ala Val Thr
                165                 170                 175

Val Ala Trp Lys Ala Asp Gly Ser Pro Val Lys Ala Gly Val Glu Thr
            180                 185                 190

Thr Lys Pro Ser Lys Gln Ser Asn Asn Lys Tyr Ala Ala Ser Ser Tyr
        195                 200                 205

Leu Ser Leu Thr Pro Glu Gln Trp Lys Ser His Arg Ser Tyr Ser Cys
    210                 215                 220

Gln Val Thr His Glu Gly Ser Thr Val Glu Lys Thr Val Ala Pro Thr
225                 230                 235                 240

Glu Cys Ser

<210>  4
<211>  729
<212>  DNA
<213>  artificial

<220>
<223>  T038 light chain

<400>  4
atggggaagc aaatggccgc cctgtgtggc tttctcctcg tggcgttgct ctggctcacg      60

cccgacgtcg cgtcaggtca gtctgccctg actcaacctg cctccgtgtc tgggtctctt     120

ggacagtcga tcaccatcac ctgcagtgga atcaccactg acataaacat ttataacctt     180

gtctcctggt atcaacaaca cccagacaaa gccccaaaac tcataattta tgaagtcaat     240

aagcggccct ctggggtttc tgatcgcttc tctggctcca agtctggcaa cacggcctcc     300

ttaataatct ctgggctcca ggctgaggac gaggctgatt atcactgctg tgcatatgga     360

cctaggagca gttctgctgt tttcggagga ggcacccatc tgaccgtcct cggccagcct     420

aaggccaatc ctacagttac tttgtttcca ccctcttctg aggaactaca agctaacaaa     480

gcaacactcg tgtgccttat ttctgacttc tatcctggtg cagttactgt tgcttggaaa     540

gcagatggat caccagtaaa ggctggtgtt gaaactacaa agccctccaa gcaaagcaac     600

aataagtatg ctgcctcttc atacctgtcc ttaacacctg agcaatggaa gagtcataga     660

agctacagtt gtcaggtaac tcacgaagga agtactgttg aaaagactgt cgctccaaca     720

gagtgttca                                                             729

<210>  5
<211>  483
<212>  PRT
<213>  artificial

<220>
<223>  T038 heavy chain

<400>  5

<210>  6
<211>  1449
<212>  DNA
<213>  artificial

<220>
<223>  T038 heavy chain

<400>  6
atggggaagc aaatggccgc cctgtgtggc tttctcctcg tggcgttgct ctggctcacg      60

cccgacgtcg cgtcaggtca ggtgctgttg caggagtcgg gcccaggact ggtgaagcct     120

tcacagaccc tgtcgctcat ctgcactgtc tctggtggct ccatccacag tgatgattac     180

cagtggagtt ggatccggca gcccccaggg cagggcctgg agtggattgg gaacatctct     240

tacagtggca gtacctacta caacccgtcc ctcaagagtc gagttaccat gtcgctagac     300

acgtccgaga atcatttctc cttgaaagtg acctctgtga ctgccgcaga cacggccatc 360

tattactgtg ccagagtcgt tacacattgc agtcgtggtg actgtttctt ttcccgggtc 420

gacgtctggg gcccagggac aacggtcacc gtctcctcag ccagcactaa aggaccatca 480

gtatttcccc ttgcaccttc atctaagagt acttctggtg gaactgcagc tttgggttgc 540

ttagtgaagg attatttccc tgagcctgtt actgtttctt ggaattctgg tgcccttact 600

tctggtgttc atactttccc tgcagtctta caaagcagtg gtttgtattc cctgagcagt 660

gttgtaactg ttccatcttc aagtctaggt acccagacct acatctgtaa tgtcaaccac 720

aagcccagca atactaaggt cgataagaag gttgagccca agtcatgtga taagacacat 780

acttgtccac cttgtccagc tccagaattg ttgggcggac catctgtttt tttgttccca 840

cccaagccta aagataccct catgatatcc agaacccctg aagttacatg cgtagttgta 900

gacgttagtc atgaggatcc tgaggtcaag tttaattggt atgtggatgg agtggaagtg 960

cataatgcaa agacaaagcc aagagaggag cagtacaact caacatacag agtggtatca 1020

gtgctgactg ttcttcacca agattggctg aatggaaaag agtataagtg caaggtgtct 1080

aataaggctt taccagctcc catcgaaaag accattagca aggctaaagg acaaccaaga 1140

gagcctcaag tatatacact tcctccatcc cgtgatgaat tgactaagaa ccaggtttct 1200

ctcacatgtc tagtgaaggg gttttaccca tctgacattg ctgttgaatg ggaaagcaat 1260

ggccagcctg aaaataacta taagaccaca ccacctgttc tagacagtga tggatccttt 1320

ttcctgtact ccaagctcac agtagacaaa tctagatggc aacaaggaaa cgtgttttca 1380

tgcagtgtta tgcatgaagc ccttcacaac cactatacac agaagtcact gagtctctca 1440

cctggtaag 1449

<210> 7
<211> 239
<212> PRT
<213> artificial

<220>
<223> T069 light chain

<400> 7

Met Ala Asn Lys His Leu Ser Leu Ser Leu Phe Leu Val Leu Leu Gly
1               5                   10                  15

Leu Ser Ala Ser Leu Ala Ser Gly Val Ile Val Met Thr Gln Ser Pro
            20                  25                  30

Ala Thr Leu Ser Val Ser Pro Gly Glu Ser Ala Thr Leu Ser Cys Arg
        35                  40                  45

Ala Ser Gln Thr Ile Tyr Asn Lys Leu Ala Trp Tyr Gln Met Lys Pro
    50                  55                  60

Gly Gln Thr Leu Arg Leu Leu Ile Tyr Gly Ala Ser Thr Arg Ala Ala
65                  70                  75                  80

Gly Ala Pro Ala Lys Phe Ser Gly Ser Gly Ser Gly Thr Glu Phe Thr
                85                  90                  95

Leu Ser Ile Ser Ser Leu Gln Ser Glu Asp Phe Ala Thr Tyr Tyr Cys
            100                 105                 110

Gln Gln Tyr Ser Asn Trp Pro Leu Thr Thr Phe Gly Gln Gly Thr Lys
        115                 120                 125

Val Glu Ile Lys Arg Thr Val Ala Ala Pro Ser Val Phe Ile Phe Pro
    130                 135                 140

Pro Ser Asp Glu Gln Leu Lys Ser Gly Thr Ala Ser Val Val Cys Leu
145                 150                 155                 160

Leu Asn Asn Phe Tyr Pro Arg Glu Ala Lys Val Gln Trp Lys Val Asp
                165                 170                 175

Asn Ala Leu Gln Ser Gly Asn Ser Gln Glu Ser Val Thr Glu Gln Asp
            180                 185                 190

Ser Lys Asp Ser Thr Tyr Ser Leu Ser Ser Thr Leu Thr Leu Ser Lys
        195                 200                 205

Ala Asp Tyr Glu Lys His Lys Val Tyr Ala Cys Glu Val Thr His Gln
    210                 215                 220

Gly Leu Ser Ser Pro Val Thr Lys Ser Phe Asn Arg Gly Glu Cys
225                 230                 235

<210>  8
<211>  645
<212>  DNA
<213>  artificial

<220>
<223>  T069 light chain

<400>  8
gtgatagtga tgacgcagtc tccagccacc ctgtctgtgt ctccagggga aagtgccacc      60

ctctcctgca gggccagtca gactatttac aacaagttag cctggtatca gatgaaacct     120

ggccagactc tcagactcct catttatggt gcatcaacca gggccgctgg tgccccagcc     180

aagttcagtg gcagtgggtc tgggacagag ttcactctca gcatcagcag cctgcagtct     240

gaagattttg caacttatta ctgtcagcag tacagtaatt ggcctctgac gacgttcggc     300

caagggacca aggtggaaat caaacgaaca gttgctgctc ctagtgtttt tatttttccc     360

ccatccgatg aacaattgaa atctggaact gcatccgtag tatgcttgtt gaacaatttc     420

taccctagag aagctaaggt tcaatggaaa gtcgataatg cactacagtc tggtaattca 480

caagagtctg ttactgaaca agactctaag gactctactt acagtctttc ttcaactctt 540

accctatcaa aggcagatta cgaaaagcat aaggtctatg cttgtgaagt tacacatcaa 600

ggattgagtt caccagttac aaagagtttt aaccgtggtg agtgt 645

<210> 9
<211> 476
<212> PRT
<213> artificial

<220>
<223> T069 heavy chain

<400> 9

Met Ala Asn Lys His Leu Ser Leu Ser Leu Phe Leu Val Leu Leu Gly
1               5                   10                  15

Leu Ser Ala Ser Leu Ala Ser Gly Glu Val Gln Leu Leu Glu Ser Gly
            20                  25                  30

Gly Gly Leu Val Leu Pro Gly Gly Phe Leu Arg Leu Ser Cys Val Gly
        35                  40                  45

Ser Gly Phe Thr Phe Arg Asn Tyr Thr Met Asn Trp Val Arg Gln Ala
    50                  55                  60

Pro Gly Lys Gly Leu Glu Trp Val Ser Gly Ile Ser Gly Gly Gly Asp
65                  70                  75                  80

Arg Thr Tyr Tyr Ala Asp Ser Val Gln Gly Arg Phe Ser Ile Ser Arg
                85                  90                  95

Asp Asn Ser Arg Asn Thr Leu Phe Leu His Met Tyr Ser Leu Arg Ala
            100                 105                 110

Glu Asp Thr Ala Leu Tyr Tyr Cys Ala Lys Asn Thr Thr Ala Ala Gly
        115                 120                 125

Asp Cys Cys Phe Phe Asp Ser Trp Gly Gln Gly Ala Leu Val Thr Val
    130                 135                 140

Ser Ser Ala Ser Thr Lys Gly Pro Ser Val Phe Pro Leu Ala Pro Ser
145                 150                 155                 160

Ser Lys Ser Thr Ser Gly Gly Thr Ala Ala Leu Gly Cys Leu Val Lys
                165                 170                 175

Asp Tyr Phe Pro Glu Pro Val Thr Val Ser Trp Asn Ser Gly Ala Leu
            180                 185                 190

Thr Ser Gly Val His Thr Phe Pro Ala Val Leu Gln Ser Ser Gly Leu
        195                 200                 205

Tyr Ser Leu Ser Ser Val Val Thr Val Pro Ser Ser Ser Leu Gly Thr
    210                 215                 220

Gln Thr Tyr Ile Cys Asn Val Asn His Lys Pro Ser Asn Thr Lys Val
225                 230                 235                 240

Asp Lys Lys Val Glu Pro Lys Ser Cys Asp Lys Thr His Thr Cys Pro
                245                 250                 255

Pro Cys Pro Ala Pro Glu Leu Leu Gly Gly Pro Ser Val Phe Leu Phe
            260                 265                 270

Pro Pro Lys Pro Lys Asp Thr Leu Met Ile Ser Arg Thr Pro Glu Val
        275                 280                 285

Thr Cys Val Val Val Asp Val Ser His Glu Asp Pro Glu Val Lys Phe
    290                 295                 300

Asn Trp Tyr Val Asp Gly Val Glu Val His Asn Ala Lys Thr Lys Pro
305                 310                 315                 320

Arg Glu Glu Gln Tyr Asn Ser Thr Tyr Arg Val Val Ser Val Leu Thr
                325                 330                 335

Val Leu His Gln Asp Trp Leu Asn Gly Lys Glu Tyr Lys Cys Lys Val
            340                 345                 350

Ser Asn Lys Ala Leu Pro Ala Pro Ile Glu Lys Thr Ile Ser Lys Ala
        355                 360                 365

Lys Gly Gln Pro Arg Glu Pro Gln Val Tyr Thr Leu Pro Pro Ser Arg
    370                 375                 380

Asp Glu Leu Thr Lys Asn Gln Val Ser Leu Thr Cys Leu Val Lys Gly
385                 390                 395                 400

Phe Tyr Pro Ser Asp Ile Ala Val Glu Trp Glu Ser Asn Gly Gln Pro
                405                 410                 415

Glu Asn Asn Tyr Lys Thr Thr Pro Pro Val Leu Asp Ser Asp Gly Ser
            420                 425                 430

Phe Phe Leu Tyr Ser Lys Leu Thr Val Asp Lys Ser Arg Trp Gln Gln
        435                 440                 445

Gly Asn Val Phe Ser Cys Ser Val Met His Glu Ala Leu His Asn His
    450                 455                 460

Tyr Thr Gln Lys Ser Leu Ser Leu Ser Pro Gly Lys
465                 470                 475

<210>  10
<211>  1356
<212>  DNA
<213>  artificial

<220>
<223>  T069 heavy chain

<400>  10
gaggtgcagt tgttggagtc ggggggaggc ttggtactgc cggggggatt cctgagactc      60

tcctgtgtag gttctggatt cacttttaga aattacacca tgaactgggt ccgccaggct     120

ccagggaagg ggctggagtg ggtctcaggt atcagtggtg gcggtgatag gacatactac     180

gcagactccg tgcagggccg cttcagcatc tccagagaca attccaggaa cactctgttt     240

ttgcacatgt acagcctgag agccgaggac acggccctct attactgtgc gaaaaacaca     300

acggccgctg gcgactgttg ttttttcgac tcgtggggcc agggagccct ggtcaccgtc     360

tcctcagcca gcactaaagg accatcagta tttccccttg caccttcatc taagagtact     420

tctggtggaa ctgcagcttt gggttgctta gtgaaggatt atttccctga gcctgttact     480

gtttcttgga attctggtgc ccttacttct ggtgttcata ctttccctgc agtcttacaa     540

agcagtggtt tgtattccct gagcagtgtt gtaactgttc catcttcaag tctaggtacc     600

cagacctaca tctgtaatgt caaccacaag cccagcaata ctaaggtcga taagaaggtt     660

gagcccaagt catgtgataa gacacatact tgtccacctt gtccagctcc agaattgttg     720

ggcggaccat ctgttttttt gttcccaccc aagcctaaag ataccctcat gatatccaga     780

acccctgaag ttacatgcgt agttgtagac gttagtcatg aggatcctga ggtcaagttt     840

aattggtatg tggatggagt ggaagtgcat aatgcaaaga caaagccaag agaggagcag     900

tacaactcaa catacagagt ggtatcagtg ctgactgttc ttcaccaaga ttggctgaat     960

gggaaagagt ataagtgcaa ggtgtctaat aaggctttac cagctcccat cgaaaagacc    1020

attagcaagg ctaaaggaca accaagagag cctcaagtat atacacttcc tccatcccgt    1080

gatgaattga ctaagaacca ggtttctctc acatgtctag tgaaggggtt ttacccatct    1140

gacattgctg ttgaatggga aagcaatggc cagcctgaaa ataactataa gaccacacca    1200

cctgttctag acagtgatgg atcctttttc ctgtactcca agctcacagt agacaaatct    1260

agatggcaac aagggaacgt gttttcatgc agtgttatgc atgaagccct tcacaaccac    1320

tatacacaga agtcactgag tctctcacct ggtaag                              1356

<210>  11
<211>  239
<212>  PRT
<213>  artificial

<220>

<223>  T096 light chain

<400>  11

Met Ala Asn Lys His Leu Ser Leu Ser Leu Phe Leu Val Leu Leu Gly
1               5                   10                  15

Leu Ser Ala Ser Leu Ala Ser Gly Glu Ile Val Met Thr Gln Ser Pro
            20                  25                  30

Ala Thr Leu Ser Val Ser Pro Gly Glu Arg Ala Thr Leu Ser Cys Arg
        35                  40                  45

Ala Ser Gln Ser Val Ser Ser Asn Leu Ala Trp Tyr Gln Gln Lys Pro
    50                  55                  60

Gly Gln Ala Pro Arg Leu Leu Ile Tyr Gly Ala Ser Thr Arg Ala Thr
65                  70                  75                  80

Gly Ile Pro Ala Arg Phe Ser Gly Ser Gly Ser Gly Thr Glu Phe Thr
                85                  90                  95

Leu Thr Ile Ser Ser Leu Gln Ser Glu Asp Phe Ala Val Tyr Tyr Cys
            100                 105                 110

Gln Gln Tyr Asn Asn Trp Pro Pro Leu Thr Phe Gly Gly Gly Thr Lys
        115                 120                 125

Val Glu Ile Lys Arg Thr Val Ala Ala Pro Ser Val Phe Ile Phe Pro
    130                 135                 140

Pro Ser Asp Glu Gln Leu Lys Ser Gly Thr Ala Ser Val Val Cys Leu
145                 150                 155                 160

Leu Asn Asn Phe Tyr Pro Arg Glu Ala Lys Val Gln Trp Lys Val Asp
                165                 170                 175

Asn Ala Leu Gln Ser Gly Asn Ser Gln Glu Ser Val Thr Glu Gln Asp
            180                 185                 190

Ser Lys Asp Ser Thr Tyr Ser Leu Ser Ser Thr Leu Thr Leu Ser Lys
        195                 200                 205

Ala Asp Tyr Glu Lys His Lys Val Tyr Ala Cys Glu Val Thr His Gln
    210                 215                 220

Gly Leu Ser Ser Pro Val Thr Lys Ser Phe Asn Arg Gly Glu Cys
225                 230                 235

<210>  12
<211>  720
<212>  DNA

<213>  artificial

<220>
<223>  T096 light chain

<400>  12
atggcgaaca aacacttgtc cctctccctc ttcctcgtcc tccttggcct gtcggccagc      60

ttggcctcag gtgaaatagt gatgacgcag tctccagcca ccctgtctgt gtctccaggg     120

gaaagagcca ccctctcctg cagggccagt cagagtgtta gcagcaactt agcctggtac     180

cagcagaaac ctggccaggc tcccaggctc ctcatctatg gtgcatccac cagggccact     240

ggtatcccag ccaggttcag tggcagtggg tctgggacag agttcactct caccatcagc     300

agcctgcagt ctgaagattt tgcagtttat tactgtcagc agtataataa ctggcctccg     360

ctcactttcg gcggagggac caaggtggag atcaaacgaa cagttgctgc tcctagtgtt     420

tttatttttc ccccatccga tgaacaattg aaatctggaa ctgcatccgt agtatgcttg     480

ttgaacaatt tctaccctag agaagctaag gttcaatgga aagtcgataa tgcactacag     540

tctggtaatt cacaagagtc tgttactgaa caagactcta aggactctac ttacagtctt     600

tcttcaactc ttaccctatc aaaggcagat tacgaaaagc ataaggtcta tgcttgtgaa     660

gttacacatc aaggattgag ttcaccagtt acaaagagtt ttaaccgtgg tgagtgttaa     720

<210>  13
<211>  476
<212>  PRT
<213>  artificial

<220>
<223>  T096 heavy chain

<400>  13

Met Ala Asn Lys His Leu Ser Leu Ser Leu Phe Leu Val Leu Leu Gly
1               5                   10                  15

Leu Ser Ala Ser Leu Ala Ser Gly Gln Val Gln Leu Val Glu Ser Gly
            20                  25                  30

Gly Gly Leu Val Lys Pro Gly Gly Ser Leu Arg Leu Ser Cys Ala Ala
        35                  40                  45

Ser Gly Phe Thr Phe Ser Asp Tyr Tyr Met Ser Trp Ile Arg Gln Ala
    50                  55                  60

Pro Gly Lys Gly Leu Glu Trp Val Ser Tyr Ile Ser Ser Ser Gly Ser
65                  70                  75                  80

Thr Ile Tyr Tyr Ala Asp Ser Val Lys Gly Arg Phe Thr Ile Ser Arg
                85                  90                  95

Asp Asn Ala Lys Asn Ser Leu Tyr Leu Gln Met Asn Ser Leu Arg Ala
            100                 105                 110

Glu Asp Thr Ala Val Tyr Tyr Cys Ala Arg Gly Ser Ser Val Ala Ala
        115                 120                 125

Leu Arg Arg Leu Phe Asp Tyr Trp Gly Gln Gly Thr Leu Val Thr Val
    130                 135                 140

Ser Ser Ala Ser Thr Lys Gly Pro Ser Val Phe Pro Leu Ala Pro Ser
145                 150                 155                 160

Ser Lys Ser Thr Ser Gly Gly Thr Ala Ala Leu Gly Cys Leu Val Lys
                165                 170                 175

Asp Tyr Phe Pro Glu Pro Val Thr Val Ser Trp Asn Ser Gly Ala Leu
            180                 185                 190

Thr Ser Gly Val His Thr Phe Pro Ala Val Leu Gln Ser Ser Gly Leu
        195                 200                 205

Tyr Ser Leu Ser Ser Val Val Thr Val Pro Ser Ser Ser Leu Gly Thr
    210                 215                 220

Gln Thr Tyr Ile Cys Asn Val Asn His Lys Pro Ser Asn Thr Lys Val
225                 230                 235                 240

Asp Lys Lys Val Glu Pro Lys Ser Cys Asp Lys Thr His Thr Cys Pro
                245                 250                 255

Pro Cys Pro Ala Pro Glu Leu Leu Gly Gly Pro Ser Val Phe Leu Phe
            260                 265                 270

Pro Pro Lys Pro Lys Asp Thr Leu Met Ile Ser Arg Thr Pro Glu Val
        275                 280                 285

Thr Cys Val Val Val Asp Val Ser His Glu Asp Pro Glu Val Lys Phe
    290                 295                 300

Asn Trp Tyr Val Asp Gly Val Glu Val His Asn Ala Lys Thr Lys Pro
305                 310                 315                 320

Arg Glu Glu Gln Tyr Asn Ser Thr Tyr Arg Val Val Ser Val Leu Thr
                325                 330                 335

Val Leu His Gln Asp Trp Leu Asn Gly Lys Glu Tyr Lys Cys Lys Val
            340                 345                 350

Ser Asn Lys Ala Leu Pro Ala Pro Ile Glu Lys Thr Ile Ser Lys Ala
        355                 360                 365

Lys Gly Gln Pro Arg Glu Pro Gln Val Tyr Thr Leu Pro Pro Ser Arg

370                 375                 380

Asp Glu Leu Thr Lys Asn Gln Val Ser Leu Thr Cys Leu Val Lys Gly
385                 390                 395                 400

Phe Tyr Pro Ser Asp Ile Ala Val Glu Trp Glu Ser Asn Gly Gln Pro
                405                 410                 415

Glu Asn Asn Tyr Lys Thr Thr Pro Pro Val Leu Asp Ser Asp Gly Ser
            420                 425                 430

Phe Phe Leu Tyr Ser Lys Leu Thr Val Asp Lys Ser Arg Trp Gln Gln
        435                 440                 445

Gly Asn Val Phe Ser Cys Ser Val Met His Glu Ala Leu His Asn His
    450                 455                 460

Tyr Thr Gln Lys Ser Leu Ser Leu Ser Pro Gly Lys
465                 470                 475

<210>  14
<211>  1428
<212>  DNA
<213>  artificial

<220>
<223>  T096 heavy chain

<400>  14
atggcgaaca aacacttgtc cctctccctc ttcctcgtcc tccttggcct gtcggccagc      60

ttggcctcag gtcaggtgca gctggtggag tctgggggag gcttggtcaa gcctggaggg     120

tccctgagac tctcctgtgc agcctctgga ttcaccttca gtgactacta catgagctgg     180

atccgccagg ctccagggaa ggggctggag tgggtttcat acattagtag tagtggtagt     240

accatatact acgcagactc tgtgaagggc cgattcacca tctccaggga caacgccaag     300

aactcactgt atctgcaaat gaacagcctg agagccgagg acacggccgt gtattactgt     360

gcgagagggt cttcggtagc tgctctccga cgcctatttg actactgggg ccagggaacc     420

ctggtcaccg tctcctcagc cagcactaaa ggaccatcag tatttcccct tgcaccttca     480

tctaagagta cttctggtgg aactgcagct ttgggttgct tagtgaagga ttatttccct     540

gagcctgtta ctgtttcttg gaattctggt gcccttactt ctggtgttca tactttccct     600

gcagtcttac aaagcagtgg tttgtattcc ctgagcagtg ttgtaactgt tccatcttca     660

agtctaggta cccagaccta catctgtaat gtcaaccaca agcccagcaa tactaaggtc     720

gataagaagg ttgagcccaa gtcatgtgat aagacacata cttgtccacc ttgtccagct     780

ccagaattgt tgggcggacc atctgttttt ttgttcccac ccaagcctaa agataccctc     840

atgatatcca gaacccctga agttacatgc gtagttgtag acgttagtca tgaggatcct     900

gaggtcaagt ttaattggta tgtggatgga gtggaagtgc ataatgcaaa gacaaagcca     960

agagaggagc agtacaactc aacatacaga gtggtatcag tgctgactgt tcttcaccaa 1020

gattggctga atgggaaaga gtataagtgc aaggtgtcta ataaggcttt accagctccc 1080

atcgaaaaga ccattagcaa ggctaaagga caaccaagag agcctcaagt atatacactt 1140

cctccatccc gtgatgaatt gactaagaac caggtttctc tcacatgtct agtgaagggg 1200

ttttacccat ctgacattgc tgttgaatgg gaaagcaatg gccagcctga aaataactat 1260

aagaccacac cacctgttct agacagtgat ggatcctttt tcctgtactc caagctcaca 1320

gtagacaaat ctagatggca acaagggaac gtgttttcat gcagtgttat gcatgaagcc 1380

cttcacaacc actatacaca gaagtcactg agtctctcac ctggtaag 1428

<210> 15
<211> 243
<212> PRT
<213> artificial

<220>
<223> T113 light chain

<400> 15

Met Ala Asn Lys His Leu Ser Leu Ser Leu Phe Leu Val Leu Leu Gly
1               5                   10                  15

Leu Ser Ala Ser Leu Ala Ser Gly Asp Val Val Met Thr Gln Ser Pro
            20                  25                  30

Leu Ser Leu Pro Val Thr Pro Gly Glu Pro Ala Ser Met Ser Cys Arg
        35                  40                  45

Ser Ser Gln Ser Leu Leu His Ser Asn Gly Tyr Lys Tyr Leu Asp Trp
    50                  55                  60

Tyr Leu Gln Lys Pro Gly Gln Ser Pro Gln Leu Leu Ile Phe Leu Gly
65                  70                  75                  80

Ser Ser Arg Ala Ser Gly Val Pro Asp Arg Phe Ser Gly Ser Gly Ser
                85                  90                  95

Gly Thr Asp Phe Thr Leu Lys Ile Asn Lys Val Lys Ala Glu Asp Val
            100                 105                 110

Gly Ile Tyr Tyr Cys Met Gln Gly Leu Gln Thr Pro Ile Thr Phe Gly
        115                 120                 125

Gln Gly Thr Arg Leu Glu Ile Lys Arg Thr Val Ala Ala Pro Ser Val
    130                 135                 140

Phe Ile Phe Pro Pro Ser Asp Glu Gln Leu Lys Ser Gly Thr Ala Ser
145                 150                 155                 160

Val Val Cys Leu Leu Asn Asn Phe Tyr Pro Arg Glu Ala Lys Val Gln
                165                 170                 175

Trp Lys Val Asp Asn Ala Leu Gln Ser Gly Asn Ser Gln Glu Ser Val
            180                 185                 190

Thr Glu Gln Asp Ser Lys Asp Ser Thr Tyr Ser Leu Ser Ser Thr Leu
        195                 200                 205

Thr Leu Ser Lys Ala Asp Tyr Glu Lys His Lys Val Tyr Ala Cys Glu
    210                 215                 220

Val Thr His Gln Gly Leu Ser Ser Pro Val Thr Lys Ser Phe Asn Arg
225                 230                 235                 240

Gly Glu Cys

<210>  16
<211>  657
<212>  DNA
<213>  artificial

<220>
<223>  T113 light chain

<400>  16
gatgtcgtga tgactcagtc tccactctcc ctgcccgtca cccctggaga gccggcctcc      60

atgtcctgta ggtctagtca gagcctcctg catagcaatg gatacaagta tttggattgg     120

tacttgcaga agccaggaca gtctccacaa ctcctgatct ttttgggttc tagtcgggcc      180

tccggggtcc ctgacaggtt cagtggcagt ggatcaggca cagattttac actaaaaatt     240

aacaaagtga aggctgagga tgttgggatt tattactgca tgcaaggtct acaaactcca     300

atcaccttcg gccaagggac acgactggag attaaacgaa cagttgctgc tcctagtgtt     360

tttatttttc ccccatccga tgaacaattg aaatctggaa ctgcatccgt agtatgcttg     420

ttgaacaatt tctaccctag agaagctaag gttcaatgga aagtcgataa tgcactacag     480

tctggtaatt cacaagagtc tgttactgaa caagactcta aggactctac ttacagtctt     540

tcttcaactc ttaccctatc aaaggcagat tacgaaaagc ataaggtcta tgcttgtgaa     600

gttacacatc aaggattgag ttcaccagtt acaaagagtt ttaaccgtgg tgagtgt        657

<210>  17
<211>  469
<212>  PRT
<213>  artificial

<220>
<223>  T113 heavy chain

<400>  17

Met Ala Asn Lys His Leu Ser Leu Ser Leu Phe Leu Val Leu Leu Gly
1               5                   10                  15

Leu Ser Ala Ser Leu Ala Ser Gly Glu Val Gln Leu Val Glu Ser Gly
            20                  25                  30

Gly Asp Leu Val Gln Pro Gly Gly Ser Leu Arg Leu Ser Cys Ala Thr
        35                  40                  45

Ser Gly Phe Asn Phe Leu Ser Ser Asn Met Thr Trp Val Arg Gln Val
    50                  55                  60

Pro Gly Lys Gly Leu Glu Trp Val Ala Lys Ile Lys Pro Asp Gly Ser
65                  70                  75                  80

Glu Lys Tyr Tyr Ala Asp Ser Val Lys Gly Arg Phe Thr Ile Ser Arg
                85                  90                  95

Asp Asn Ala Lys Lys Ser Leu Tyr Leu Gln Met Thr Asn Leu Arg Val
            100                 105                 110

Glu Asp Thr Ser Val Tyr Tyr Cys Ala Arg Gly Gln Pro Phe Asp Tyr
        115                 120                 125

Trp Gly Gln Gly Thr Leu Val Ala Val Ser Ser Ala Ser Thr Lys Gly
    130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Lys Val Glu Pro Lys
225                 230                 235                 240

Ser Cys Asp Lys Thr His Thr Cys Pro Pro Cys Pro Ala Pro Glu Leu
                245                 250                 255

Leu Gly Gly Pro Ser Val Phe Leu Phe Pro Pro Lys Pro Lys Asp Thr
            260                 265                 270

Leu Met Ile Ser Arg Thr Pro Glu Val Thr Cys Val Val Val Asp Val
        275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Asn Ser
305                 310                 315                 320

Thr Tyr Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
            340                 345                 350

Pro Ile Glu Lys Thr Ile Ser Lys Ala Lys Gly Gln Pro Arg Glu Pro
        355                 360                 365

Gln Val Tyr Thr Leu Pro Pro Ser Arg Asp Glu Leu Thr Lys Asn Gln
    370                 375                 380

Val Ser Leu Thr Cys Leu Val Lys Gly Phe Tyr Pro Ser Asp Ile Ala
385                 390                 395                 400

Val Glu Trp Glu Ser Asn Gly Gln Pro Glu Asn Asn Tyr Lys Thr Thr
                405                 410                 415

Pro Pro Val Leu Asp Ser Asp Gly Ser Phe Phe Leu Tyr Ser Lys Leu
            420                 425                 430

Thr Val Asp Lys Ser Arg Trp Gln Gln Gly Asn Val Phe Ser Cys Ser
        435                 440                 445

Val Met His Glu Ala Leu His Asn His Tyr Thr Gln Lys Ser Leu Ser
    450                 455                 460

Leu Ser Pro Gly Lys
465

<210> 18
<211> 1335
<212> DNA
<213> artificial

<220>
<223> T113 heavy chain

<400> 18
gccagcacta aaggaccatc agtatttccc cttgcacctt catctaagag tacttctggt 60

ggaactgcag ctttgggttg cttagtgaag gattatttcc ctgagcctgt tactgtttct 120

tggaattctg gtgcccttac ttctggtgtt catactttcc ctgcagtctt acaaagcagt 180

ggtttgtatt ccctgagcag tgttgtaact gttccatctt caagtctagg tacccagacc 240

tacatctgta atgtcaacca caagcccagc aatactaagg tcgataagaa ggttgagccc 300

aagtcatgtg ataagacaca tacttgtcca ccttgtccag ctccagaatt gttgggcgga 360

ccatctgttt ttttgttccc acccaagcct aaagataccc tcatgatatc cagaacccct 420

gaagttacat gcgtagttgt agacgttagt catgaggatc ctgaggtcaa gtttaattgg 480

tatgtggatg gagtggaagt gcataatgca aagacaaagc caagagagga gcagtacaac 540

tcaacataca gagtggtatc agtgctgact gttcttcacc aagattggct gaatggaaaa 600

gagtataagt gcaaggtgtc taataaggct ttaccagctc ccatcgaaaa gaccattagc 660

aaggctaaag gacaaccaag agagcctcaa gtatatacac ttcctccatc ccgtgatgaa 720

ttgactaaga accaggtttc tctcacatgt ctagtgaagg ggttttaccc atctgacatt 780

gctgttgaat gggaaagcaa tggccagcct gaaaataact ataagaccac accacctgtt 840

ctagacagtg atggatcctt tttcctgtac tccaagctca cagtagacaa atctagatgg 900

caacaaggga acgtgttttc atgcagtgtt atgcatgaag cccttcacaa ccactataca 960

cagaagtcac tgagtctctc acctggtaag gaagttcagc tggtggagtc tgggggagac 1020

ttggtccagc ctggggggtc cctgagactc tcatgtgcaa cttctggatt caactttttg 1080

agttccaata tgacctgggt ccgccaggtt ccagggaagg ggctggagtg ggtggccaag 1140

ataaaaccag atggaagtga gaaatactat gcggactctg tgaagggccg attcaccatc 1200

tccagagaca acgccaagaa gtcactgtat ttgcaaatga ccaacctgag agtcgaggac 1260

acgtctgtat attactgtgc gagagggcag ccctttgact actggggcca gggaaccctg 1320

gtcgccgtct cctca 1335

<210> 19
<211> 240
<212> PRT
<213> artificial

<220>
<223> T117 light chain

<400> 19

Met Gly Lys Gln Met Ala Ala Leu Cys Gly Phe Leu Leu Val Ala Leu
1               5                   10                  15

Leu Trp Leu Thr Pro Asp Val Ala Ser Gly Ser Tyr Glu Leu Thr Gln
            20                  25                  30

Pro Pro Ser Val Ser Val Ser Pro Gly Gln Thr Ala Arg Ile Thr Cys
        35                  40                  45

Ser Gly Asp Ala Leu Pro Lys Lys Tyr Ala Tyr Trp Tyr Gln Gln Lys
    50                  55                  60

Ser Gly Gln Ala Pro Val Leu Val Ile Tyr Glu Asp Ser Lys Arg Pro
65                  70                  75                  80

Ser Gly Ile Pro Glu Arg Phe Ser Gly Ser Ser Ser Gly Thr Met Ala
                85                  90                  95

Thr Leu Thr Ile Ser Gly Ala Gln Val Glu Asp Glu Ala Asp Tyr Tyr
            100                 105                 110

Cys Tyr Ser Thr Asp Ser Ser Gly Asn His Tyr Val Phe Gly Thr Gly
        115                 120                 125

Thr Lys Val Thr Val Leu Gly Gln Pro Lys Ala Asn Pro Thr Val Thr
    130                 135                 140

Leu Phe Pro Pro Ser Ser Glu Glu Leu Gln Ala Asn Lys Ala Thr Leu
145                 150                 155                 160

Val Cys Leu Ile Ser Asp Phe Tyr Pro Gly Ala Val Thr Val Ala Trp
                165                 170                 175

Lys Ala Asp Gly Ser Pro Val Lys Ala Gly Val Glu Thr Thr Lys Pro
            180                 185                 190

Ser Lys Gln Ser Asn Asn Lys Tyr Ala Ala Ser Ser Tyr Leu Ser Leu
        195                 200                 205

Thr Pro Glu Gln Trp Lys Ser His Arg Ser Tyr Ser Cys Gln Val Thr
    210                 215                 220

His Glu Gly Ser Thr Val Glu Lys Thr Val Ala Pro Thr Glu Cys Ser
225                 230                 235                 240

<210>  20
<211>  720
<212>  DNA
<213>  artificial

<220>
<223>  T117 light chain

<400>  20
atggggaagc aaatggccgc cctgtgtggc tttctcctcg tggcgttgct ctggctcacg      60

cccgacgtcg cgtcaggttc ctatgagctg acacagccac cctcggtgtc agtgtcccca     120

ggacaaacgg ccaggatcac ctgctctgga gatgcattgc caaaaaaata tgcttattgg     180

tatcagcaga agtcaggcca ggcccctgtg ctggtcatct atgaggacag caaacgaccc     240

tccgggatcc ctgagagatt ctctggctcc agctcaggga caatggccac cttgactatc     300

agtggggccc aggtggagga tgaagctgac tactactgtt actcaacaga cagcagtggt     360

aatcattatg tcttcggaac tgggaccaag gtcaccgtcc tcggccagcc taaggccaat     420

cctacagtta ctttgtttcc accctcttct gaggaactac aagctaacaa agcaacactc 480

gtgtgcctta tttctgactt ctatcctggt gcagttactg ttgcttggaa agcagatgga 540

tcaccagtaa aggctggtgt tgaaactaca aagccctcca agcaaagcaa caataagtat 600

gctgcctctt catacctgtc cttaacacct gagcaatgga agagtcatag aagctacagt 660

tgtcaggtaa ctcacgaagg aagtactgtt gaaaagactg tcgctccaac agagtgttca 720

<210> 21
<211> 470
<212> PRT
<213> artificial

<220>
<223> T117 heavy chain

<400> 21

Met Gly Lys Gln Met Ala Ala Leu Cys Gly Phe Leu Leu Val Ala Leu
1               5                   10                  15

Leu Trp Leu Thr Pro Asp Val Ala Ser Gly His Val Gln Leu Gln Glu
            20                  25                  30

Ser Gly Pro Gly Leu Val Lys Pro Ser Gln Thr Leu Ser Leu Thr Cys
        35                  40                  45

Thr Val Ser Gly Ala Ser Ile Ser Ser Ser Asn Tyr Asn Trp Thr Trp
    50                  55                  60

Ile Arg Gln Pro Pro Gly Lys Gly Leu Glu Trp Ile Gly Asn Ile Ser
65                  70                  75                  80

Tyr Ser Gly Arg Thr Asn Tyr Asn Pro Ser Leu Lys Asn Arg Val Thr
                85                  90                  95

Leu Ser Ile Asp Thr Ser Lys Asn Gln Phe Ser Leu Asn Leu Asn Ser
            100                 105                 110

Val Thr Ala Ala Asp Thr Ala Val Tyr Tyr Cys Val Gly Gly Phe Asp
        115                 120                 125

Pro Trp Gly Gln Gly Thr Leu Val Thr Val Ser Ser Ala Ser Thr Lys
    130                 135                 140

Gly Pro Ser Val Phe Pro Leu Ala Pro Ser Ser Lys Ser Thr Ser Gly
145                 150                 155                 160

Gly Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr

            180                 185                 190

Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220

Val Asn His Lys Pro Ser Asn Thr Lys Val Asp Lys Lys Val Glu Pro
225                 230                 235                 240

Lys Ser Cys Asp Lys Thr His Thr Cys Pro Pro Cys Pro Ala Pro Glu
                245                 250                 255

Leu Leu Gly Gly Pro Ser Val Phe Leu Phe Pro Pro Lys Pro Lys Asp
            260                 265                 270

Thr Leu Met Ile Ser Arg Thr Pro Glu Val Thr Cys Val Val Val Asp
        275                 280                 285

Val Ser His Glu Asp Pro Glu Val Lys Phe Asn Trp Tyr Val Asp Gly
    290                 295                 300

Val Glu Val His Asn Ala Lys Thr Lys Pro Arg Glu Glu Gln Tyr Asn
305                 310                 315                 320

Ser Thr Tyr Arg Val Val Ser Val Leu Thr Val Leu His Gln Asp Trp
                325                 330                 335

Leu Asn Gly Lys Glu Tyr Lys Cys Lys Val Ser Asn Lys Ala Leu Pro
            340                 345                 350

Ala Pro Ile Glu Lys Thr Ile Ser Lys Ala Lys Gly Gln Pro Arg Glu
        355                 360                 365

Pro Gln Val Tyr Thr Leu Pro Pro Ser Arg Asp Glu Leu Thr Lys Asn
    370                 375                 380

Gln Val Ser Leu Thr Cys Leu Val Lys Gly Phe Tyr Pro Ser Asp Ile
385                 390                 395                 400

Ala Val Glu Trp Glu Ser Asn Gly Gln Pro Glu Asn Asn Tyr Lys Thr
                405                 410                 415

Thr Pro Pro Val Leu Asp Ser Asp Gly Ser Phe Phe Leu Tyr Ser Lys
            420                 425                 430

Leu Thr Val Asp Lys Ser Arg Trp Gln Gln Gly Asn Val Phe Ser Cys
        435                 440                 445

Ser Val Met His Glu Ala Leu His Asn His Tyr Thr Gln Lys Ser Leu
    450                 455                 460

Ser Leu Ser Pro Gly Lys
465                 470

<210>  22
<211>  1410
<212>  DNA
<213>  artificial

<220>
<223>  T117 heavy chain

<400>  22
atggggaagc aaatggccgc cctgtgtggc tttctcctcg tggcgttgct ctggctcacg      60

cccgacgtcg cgtcaggtca cgtgcagctg caagagtcgg gcccaggact ggtgaagcct     120

tcacagactt tgtccctcac ctgcactgtc tctggtgcct ccatcagcag ttctaattat     180

aactggactt ggatccgcca gcccccaggg aagggcctgg agtggattgg caacatctct     240

tacagtgggc gcaccaacta caacccgtcc ctcaagaatc gcgtgaccct ctcaatagac     300

acgtcgaaga accagttctc cctcaacttg aactctgtga ctgccgcaga cacagccgtc     360

tattattgtg tcgggggatt cgacccctgg ggccagggaa ccctggtcac cgtctcctca     420

gccagcacta aaggaccatc agtatttccc cttgcacctt catctaagag tacttctggt     480

ggaactgcag ctttgggttg cttagtgaag gattatttcc ctgagcctgt tactgtttct     540

tggaattctg gtgcccttac ttctggtgtt catactttcc ctgcagtctt acaaagcagt     600

ggtttgtatt ccctgagcag tgttgtaact gttccatctt caagtctagg tacccagacc     660

tacatctgta atgtcaacca caagcccagc aatactaagg tcgataagaa ggttgagccc     720

aagtcatgtg ataagacaca tacttgtcca ccttgtccag ctccagaatt gttgggcgga     780

ccatctgttt ttttgttccc acccaagcct aaagataccc tcatgatatc cagaacccct     840

gaagttacat gcgtagttgt agacgttagt catgaggatc ctgaggtcaa gtttaattgg     900

tatgtggatg gagtggaagt gcataatgca aagacaaagc caagagagga gcagtacaac     960

tcaacataca gagtggtatc agtgctgact gttcttcacc aagattggct gaatgggaaa    1020

gagtataagt gcaaggtgtc taataaggct ttaccagctc ccatcgaaaa gaccattagc    1080

aaggctaaag gacaaccaag agagcctcaa gtatatacac ttcctccatc ccgtgatgaa    1140

ttgactaaga accaggtttc tctcacatgt ctagtgaagg ggttttaccc atctgacatt    1200

gctgttgaat gggaaagcaa tggccagcct gaaaataact ataagaccac accacctgtt    1260

ctagacagtg atggatcctt tttcctgtac tccaagctca cagtagacaa atctagatgg    1320

caacaaggga acgtgttttc atgcagtgtt atgcatgaag cccttcacaa ccactataca    1380

cagaagtcac tgagtctctc acctggtaag                                     1410

<210>  23

<211>  242
<212>  PRT
<213>  artificial

<220>
<223>  T118 light chain

<400>  23

Met Gly Lys Gln Met Ala Ala Leu Cys Gly Phe Leu Leu Val Ala Leu
1               5                   10                  15

Leu Trp Leu Thr Pro Asp Val Ala Ser Gly Gln Ser Val Val Thr Gln
            20                  25                  30

Pro Pro Ser Ala Ser Gly Thr Pro Gly Gln Arg Val Ser Ile Ser Cys
        35                  40                  45

Ser Gly Gly Thr Ser Asn Ile Gly Val Asn Thr Val Asn Trp Tyr Gln
    50                  55                  60

Gln Phe Pro Gly Thr Ala Pro Lys Leu Leu Ile Tyr Asn Asn Asp Gln
65                  70                  75                  80

Arg Pro Ser Gly Val Pro Asp Arg Phe Ser Gly Ser Lys Ser Gly Thr
                85                  90                  95

Ser Ala Ser Leu Ala Ile Ser Gly Leu Gln Ser Ala Asp Glu Ala Asp
            100                 105                 110

Tyr Tyr Cys Ala Thr Trp Asp Asp Ser Leu Asn Gly Trp Val Phe Gly
        115                 120                 125

Gly Gly Ser Lys Leu Thr Val Leu Gly Gln Pro Lys Ala Asn Pro Thr
    130                 135                 140

Val Thr Leu Phe Pro Pro Ser Ser Glu Glu Leu Gln Ala Asn Lys Ala
145                 150                 155                 160

Thr Leu Val Cys Leu Ile Ser Asp Phe Tyr Pro Gly Ala Val Thr Val
                165                 170                 175

Ala Trp Lys Ala Asp Gly Ser Pro Val Lys Ala Gly Val Glu Thr Thr
            180                 185                 190

Lys Pro Ser Lys Gln Ser Asn Asn Lys Tyr Ala Ala Ser Ser Tyr Leu
        195                 200                 205

Ser Leu Thr Pro Glu Gln Trp Lys Ser His Arg Ser Tyr Ser Cys Gln
    210                 215                 220

Val Thr His Glu Gly Ser Thr Val Glu Lys Thr Val Ala Pro Thr Glu
225                 230                 235                 240

Cys Ser

<210> 24
<211> 726
<212> DNA
<213> artificial

<220>
<223> T118 light chain

<400> 24
atggggaagc aaatggccgc cctgtgtggc tttctcctcg tggcgttgct ctggctcacg 60

cccgacgtcg cgtcaggtca gtctgtggtg actcagccac cctcagcgtc tgggaccccc 120

gggcagaggg tctccatctc ttgttctgga ggcacctcca atatcggagt taatactgtt 180

aactggtacc agcagttccc agggacggcc cccaaactcc tcatctataa taatgatcag 240

cggccctcag gggtccctga ccgattctct ggctccaagt ctggcacctc agcctccctg 300

gccatcagtg ggctccagtc tgcggatgag gctgattatt actgtgccac atgggatgac 360

agcctgaatg gttgggtgtt cggcggaggg tccaagctga ccgtcctcgg ccagcctaag 420

gccaatccta cagttacttt gtttccaccc tcttctgagg aactacaagc taacaaagca 480

acactcgtgt gccttatttc tgacttctat cctggtgcag ttactgttgc ttggaaagca 540

gatggatcac cagtaaaggc tggtgttgaa actacaaagc cctccaagca aagcaacaat 600

aagtatgctg cctcttcata cctgtcctta acacctgagc aatggaagag tcatagaagc 660

tacagttgtc aggtaactca cgaaggaagt actgttgaaa agactgtcgc tccaacagag 720

tgttca 726

<210> 25
<211> 485
<212> PRT
<213> artificial

<220>
<223> T118 heavy chain

<400> 25

Met Gly Lys Gln Met Ala Ala Leu Cys Gly Phe Leu Leu Val Ala Leu
1               5                   10                  15

Leu Trp Leu Thr Pro Asp Val Ala Ser Gly Gln Val Lys Leu Val Glu
            20                  25                  30

Ser Gly Gly Ala Val Val Gln Pro Gly Arg Ser Leu Arg Leu Ser Cys
        35                  40                  45

Leu Ala Ser Gly Phe Thr Phe Gly Asn Tyr Gly Met Tyr Trp Val Arg
    50                  55                  60

Gln Ala Pro Gly Arg Gly Leu Glu Trp Val Ala Leu Ile Ser Tyr Asp
65                  70                  75                  80

Gly Ser Asn Glu Asn Tyr Ala Asp Cys Val Lys Gly Arg Phe Thr Ile
                85                  90                  95

Ser Arg Asp Asn Ser Lys Asn Thr Val Phe Leu Gln Met Asp Arg Leu
            100                 105                 110

Arg Val Glu Asp Thr Ala Ile Tyr Phe Cys Ala Lys Asn Val Ser Val
        115                 120                 125

Ser Glu His Arg Thr Ser Thr Ala Lys Gly Gly Asn Gly Met Asp Val
    130                 135                 140

Trp Gly Gln Gly Thr Thr Val Ile Ala Ser Ser Ala Ser Thr Lys Gly
145                 150                 155                 160

Pro Ser Val Phe Pro Leu Ala Pro Ser Ser Lys Ser Thr Ser Gly Gly
                165                 170                 175

Thr Ala Ala Leu Gly Cys Leu Val Lys Asp Tyr Phe Pro Glu Pro Val
            180                 185                 190

Thr Val Ser Trp Asn Ser Gly Ala Leu Thr Ser Gly Val His Thr Phe
        195                 200                 205

Pro Ala Val Leu Gln Ser Ser Gly Leu Tyr Ser Leu Ser Ser Val Val
    210                 215                 220

Thr Val Pro Ser Ser Ser Leu Gly Thr Gln Thr Tyr Ile Cys Asn Val
225                 230                 235                 240

Asn His Lys Pro Ser Asn Thr Lys Val Asp Lys Lys Val Glu Pro Lys
                245                 250                 255

Ser Cys Asp Lys Thr His Thr Cys Pro Pro Cys Pro Ala Pro Glu Leu
            260                 265                 270

Leu Gly Gly Pro Ser Val Phe Leu Phe Pro Pro Lys Pro Lys Asp Thr
        275                 280                 285

Leu Met Ile Ser Arg Thr Pro Glu Val Thr Cys Val Val Val Asp Val
    290                 295                 300

Ser His Glu Asp Pro Glu Val Lys Phe Asn Trp Tyr Val Asp Gly Val
305                 310                 315                 320

Glu Val His Asn Ala Lys Thr Lys Pro Arg Glu Glu Gln Tyr Asn Ser
                325                 330                 335

Thr Tyr Arg Val Val Ser Val Leu Thr Val Leu His Gln Asp Trp Leu
            340                 345                 350

Asn Gly Lys Glu Tyr Lys Cys Lys Val Ser Asn Lys Ala Leu Pro Ala
        355                 360                 365

Pro Ile Glu Lys Thr Ile Ser Lys Ala Lys Gly Gln Pro Arg Glu Pro
    370                 375                 380

Gln Val Tyr Thr Leu Pro Pro Ser Arg Asp Glu Leu Thr Lys Asn Gln
385                 390                 395                 400

Val Ser Leu Thr Cys Leu Val Lys Gly Phe Tyr Pro Ser Asp Ile Ala
                405                 410                 415

Val Glu Trp Glu Ser Asn Gly Gln Pro Glu Asn Asn Tyr Lys Thr Thr
            420                 425                 430

Pro Pro Val Leu Asp Ser Asp Gly Ser Phe Phe Leu Tyr Ser Lys Leu
        435                 440                 445

Thr Val Asp Lys Ser Arg Trp Gln Gln Gly Asn Val Phe Ser Cys Ser
    450                 455                 460

Val Met His Glu Ala Leu His Asn His Tyr Thr Gln Lys Ser Leu Ser
465                 470                 475                 480

Leu Ser Pro Gly Lys
                485

<210>  26
<211>  1455
<212>  DNA
<213>  artificial

<220>
<223>  T118 heavy chain

<400>  26
atggggaagc aaatggccgc cctgtgtggc tttctcctcg tggcgttgct ctggctcacg      60

cccgacgtcg cgtcaggtca ggtgaaactg gtggagtctg ggggagccgt ggtccagcct     120

gggaggtccc tgagactctc ctgtctagcc tctggattca ctttcggaaa ttatggcatg     180

tactgggtcc gccaggctcc aggcaggggg ctggagtggg tggcacttat ttcatatgat     240

ggaagtaatg agaactatgc agactgcgtg aagggccgat tcaccatctc cagagacaat     300

tccaagaata cggtgtttct gcaaatggac cgcctgagag ttgaggacac ggccatatat     360

ttctgtgcga aaaatgtgtc agtcagtgaa catagaacat caaccgcgaa aggggggaac     420

ggaatggacg tctggggcca agggaccacg gtgatcgcct cctcagccag cactaaagga     480

ccatcagtat ttccccttgc accttcatct aagagtactt ctggtggaac tgcagctttg 540

ggttgcttag tgaaggatta tttccctgag cctgttactg tttcttggaa ttctggtgcc 600

cttacttctg gtgttcatac tttccctgca gtcttacaaa gcagtggttt gtattccctg 660

agcagtgttg taactgttcc atcttcaagt ctaggtaccc agacctacat ctgtaatgtc 720

aaccacaagc ccagcaatac taaggtcgat aagaaggttg agcccaagtc atgtgataag 780

acacatactt gtccaccttg tccagctcca gaattgttgg gcggaccatc tgtttttttg 840

ttcccaccca agcctaaaga taccctcatg atatccagaa cccctgaagt tacatgcgta 900

gttgtagacg ttagtcatga ggatcctgag gtcaagttta attggtatgt ggatggagtg 960

gaagtgcata atgcaaagac aaagccaaga gaggagcagt acaactcaac atacagagtg 1020

gtatcagtgc tgactgttct tcaccaagat tggctgaatg ggaaagagta taagtgcaag 1080

gtgtctaata aggctttacc agctcccatc gaaaagacca ttagcaaggc taaaggacaa 1140

ccaagagagc ctcaagtata tacacttcct ccatcccgtg atgaattgac taagaaccag 1200

gtttctctca catgtctagt gaaggggttt tacccatctg acattgctgt tgaatgggaa 1260

agcaatggcc agcctgaaaa taactataag accacaccac ctgttctaga cagtgatgga 1320

tcctttttcc tgtactccaa gctcacagta gacaaatcta gatggcaaca agggaacgtg 1380

ttttcatgca gtgttatgca tgaagccctt cacaaccact atacacagaa gtcactgagt 1440

ctctcacctg gtaag 1455

## Description

### FIELD OF THE INVENTION

The present invention relates to a pharmaceutical preparation and an aqueous formulation comprising an immunoglobulin or a protein conjugate comprising an immunoglobulin conjugated to a carrier protein. The pharmaceutical formulation may be for parenteral administration such as subcutaneous administration for the treatment of B-cell non-Hodgkin lymphoma. The invention further relates to a set of at least two different protein conjugate preparations.

### BACKGROUND OF THE INVENTION

Anti-idiotype antibodies and vaccines are a promising tool for the treatment of various cancers such as B-cell lymphoma, see López-Díaz de Cerio et al., Oncogene 26 (2007) 3594-3602; Levy, J. Clinical Oncology 17(11s) (1999) 7-13; J Natl Cancer Inst 98 (2006) 1292-1301. A blood cancer for which successful treatment has been demonstrated in clinical experiments is non-Hodgkin lymphoma (herein: NHL). Tumor therapy by anti-idiotype antibodies or vaccines requires patient-specific antibodies or vaccines, respectively, which represents a major challenge for large-scale application to many patients, since sufficient amounts of antibody or vaccine have to be produced for each patient separately within a reasonable time and at reasonable costs. Several technologies for the production of human immunoglobulins are known such as the concomitant production of heavy and light chains of immunoglobulin G in plants (WO2006/079546; Giritch et al., Proc. Natl. Acad. Sci. USA 2006 103 (40) 14701-14706; McCormick et al., Proc. Natl. Acad. Sci. USA 96 (1999) 703-708; Osterroth et al., Journal of Immunological Methods 229 (1999) 141-153). The technical challenges for cloning of relevant complementarity determining regions (CDRs) from autopsy-derived RNA and expression of heavy and light chains of immunoglobulins is also feasible. Due to the enormous costs for providing patient-specific immunoglobulins, automation of as many production steps as possible is imperative.

A problem inherent to the production of patient-specific medicaments is that the physical properties of individual immunoglobulins vary due to the differences in their variable regions. These differences affect the solubility and stability of the immunoglobulin preparations. It has been observed that immunoglobulins differing in their variable region have a high variability in terms of solubility of the antibodies in typical buffers used for extracting the immunoglobulins from cells as well as buffers used for downstream processing. In order to be able to provide predetermined dosages of the immunoglobulin to the medical personnel for administration to patients, immunoglobulin preparations must fulfil certain minimum stability requirements. However, it is not possible in a standardized manufacturing procedure to search for optimal conditions for each individual immunoglobulin. Thus, conditions must be found that allow achieving sufficiently stable immunoglobulin preparations over a wide range of immunoglobulins differing in their variable region.

It is therefore an object of the present invention to provide a pharmaceutical formulation of immunoglobulins, notably of IgG, and of their conjugates with suitable carrier proteins that provides high stability and solubility of the immunoglobulins and their conjugates. It is also an object to provide a method of treatment of B-cell lymphoma by anti-idiotype vaccines using the formulation of the present invention.

### Summary of the invention

The present invention provides a pharmaceutical preparation comprising a protein conjugate comprising an immunoglobulin (such as immunoglobulin G) conjugated to a carrier protein, and histidine as a buffering agent.

The present invention further provides an aqueous pharmaceutical formulation comprising a protein conjugate comprising an immunoglobulin (such as immunoglobulin G) conjugated to a carrier protein, and histidine as a buffering agent.

Further, the present invention provides a formulation for use in a therapeutic method such as for the treatment of non-Hodgkin lymphoma. The present invention provides a formulation for use in a method for the treatment of B-cell lymphoma, notably follicular B-cell non-Hodgkin lymphoma.

The present invention further provides a set of at least two different protein conjugate preparations, each protein conjugate preparation comprising histidine as a buffering agent and a protein conjugate comprising one or more immunoglobulin moieties conjugated to a carrier protein; wherein the immunoglobulin moieties of each protein conjugate preparation have identical complementarity determining regions (CDRs); and wherein different protein conjugate preparations differ in that the immunoglobulin moieties of the protein conjugates have different CDRs. In one embodiment, the immunoglobulin moieties of each protein conjugate preparation have identical variable regions, and different protein conjugate preparations differ in that the immunoglobulin moieties of the protein conjugates have different variable regions.

Also provided is a method of treating a patient suffering from B-cell non-Hodgkin lymphoma (notably follicular B-cell lymphoma), comprising administering to the patient an aqueous pharmaceutical formulation, said formulation comprising a protein conjugate comprising an immunoglobulin conjugated to a carrier protein, and histidine as a buffering agent; wherein the complementarity determining regions of the immunoglobulin conjugated to the carrier protein elicits an immune response in the patient against the non-Hodgkin lymphoma B-cells.

In an important embodiment, the immunoglobulins used in the invention are plant-expressed immunoglobulins, such as plant-expressed IgGs. A preferred plant for expressing are *Nicotiana* plants such as *Nicotiana benthamiana* or *Nicotiana tabacum.*

Immunoglobulins and protein conjugates comprising immunoglobulins can vary considerably in their solubility and stability in solution due to differences in their variable region, notably in the complementary determining regions (CDR) of the immunoglobulins. It has now surprisingly been found by the inventors that aqueous formulations containing histidine as a buffering agent can stabilise a wide range of immunoglobulins and protein conjugates comprising immunoglobulins and a carrier protein. Using histidine as a buffering agent thus allows high throughput generation of different immunoglobulins and protein conjugates of sufficient stability without the need to search for suitable additives or buffers for immunoglobulins or protein conjugates of problematic stability. Further, use of histidine buffer, optionally in combination with suitable surfactants as described below, provides sufficient stability against shaking or shear stress as occurs during sterile filtration, storage, shipment or filtering of formulations before administration to patients. Thus, the invention provides an important contribution for making individualised medicine using immunoglobulins or protein conjugates thereof commercially viable.

### Brief description of the figures

Fig. 1 is a scheme showing amplification of variable region fragments of an immunoglobulin and cloning in viral vectors for heavy chain expression. BAP stands for an adaptor sequence as in Fig. 2 of WO 2010/040531. Nucleotide quadruplett AGGT is the cleavage site of the Bsal sites and, accordingly, the sequence of a single-stranded overhang produced by Bsal restriction. Similarly, NNNN stands for any predetermined sequence forming a single-stranded overhang produced by Bsal restriction. Rice ats is the rice amylase transit signal peptide.
Fig. 2 is a workflow showing steps for amplification, cloning, and expression in Nicotiana benthamiana of heavy and light IgG chains. See section "Preparation of idiotypic antibodies for vaccination against NHL" for more information on the working steps depicted.
Fig. 3A and B show schematically binary vectors encoding RNA viral vectors used for expressing the light and heavy chains of Ig T038 used in the examples.

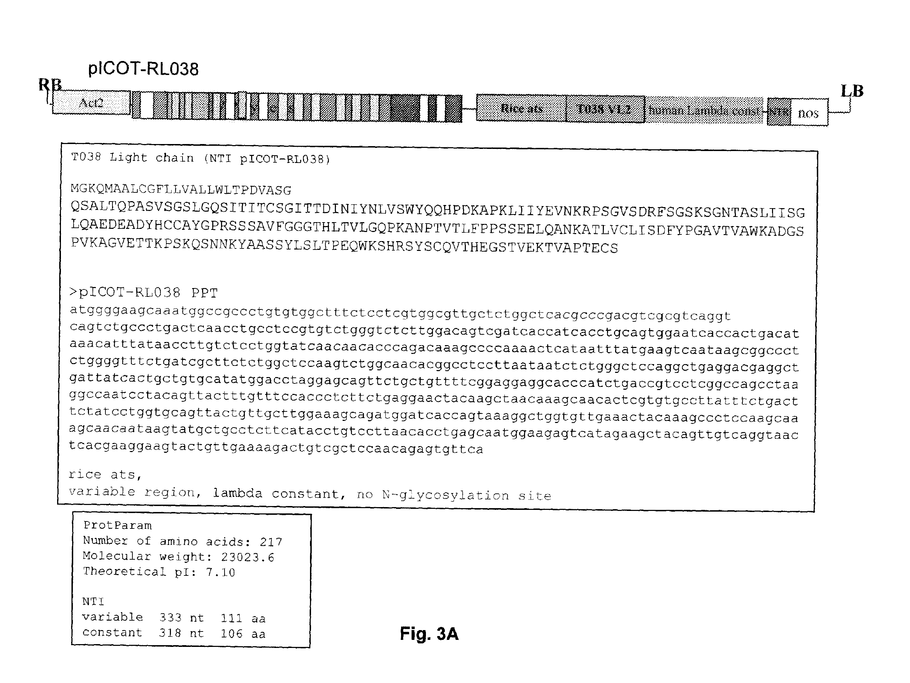

Fig. 3A depicts binary TMV-based vector pICOT-RL038 encoding the T038 light chain. RB and LB are the right and left T-DNA borders. Act2 indicates the rice actin2 promoter. The long extended box downstream from Act2 is a replicase ORF derived from tobacco mosaic virus (TMV); small boxes therein indicate artificially introduced introns. T038 VL2 is the light chain variable region of Ig T038 that is followed by the codon-optimised constant region of a human lambda chain. NTR stands for non-translated region. NOS stands for the nopaline synthase terminator. The amino acid sequence (SEQ ID NO: 3) and nucleotide sequence (SEQ ID NO: 4) of the fragment consisting of or encoding, respectively, the rice ats (shown in the first line of the sequences), the variable region, and the lambda constant region are shown.

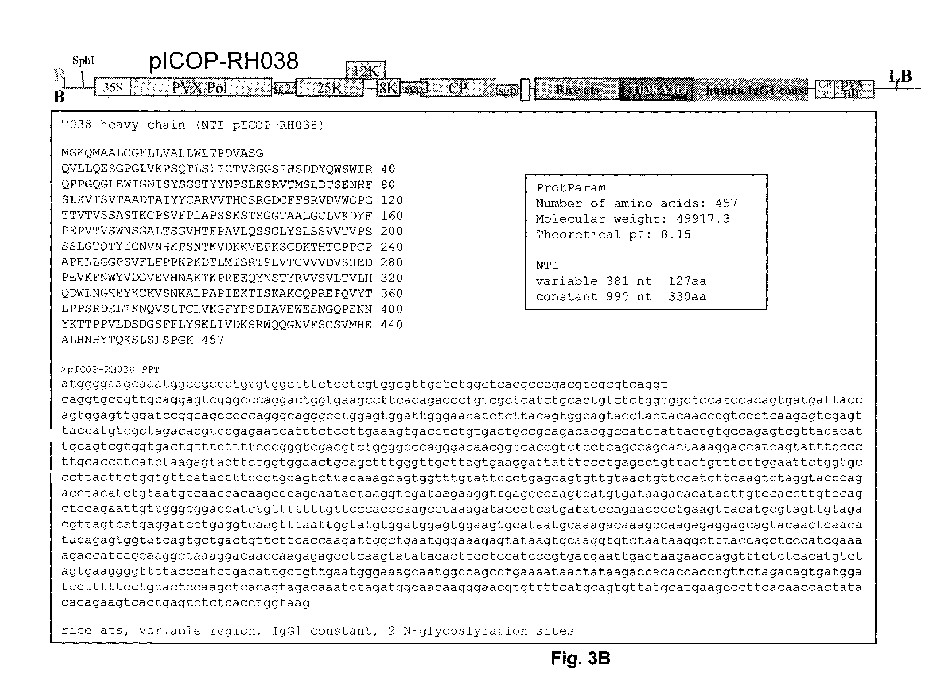

Fig. 3B depicts binary vector PICOP-RH038 containing the T038 heavy chain. The potato virus X (PVX)-based vector is under the control of the 35S promoter. PVX Pol is the PVX polymerase. Dowstream thereof, the subgenomic promoter sg25 is depicted followed by the PVX-based triple gene block encoding the 25K, 12K and 8K proteins. sgp is the PVX coat protein (CP) subgenomic promoter. T038 VH4 is the sequence encoding the variable heavy chain fragment of T038 linked to the human IgG1 constant region. CP3' stands for the coat protein ORF located 3' of the vector. The amino acid sequence (SEQ ID NO: 5) and nucleotide sequence (SEQ ID NO: 6) of the fragment consisting of and encoding, respectively, the rice ats (first line of the sequences shown), the variable region, and the IgG1 constant region are shown.

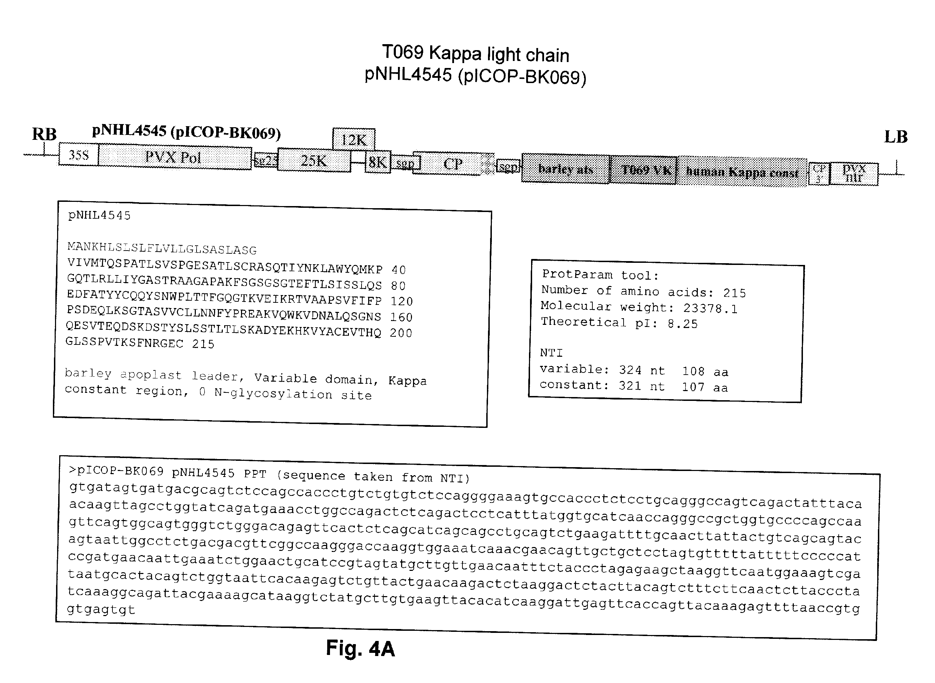

Fig. 4A shows schematically the binary PVX-based vector pICOP-BK069 encoding an RNA viral vector used for expressing the light chains of Ig T069 used in the examples. The amino acid sequence (SEQ ID NO: 7) of the fragment consisting the barley amylase ats (first line of the sequence), the variable region, and the kappa constant region is shown. The nucleotide sequence (SEQ ID NO: 8) of the fragment encoding the variable region and the kappa constant region is shown in the box at the bottom.

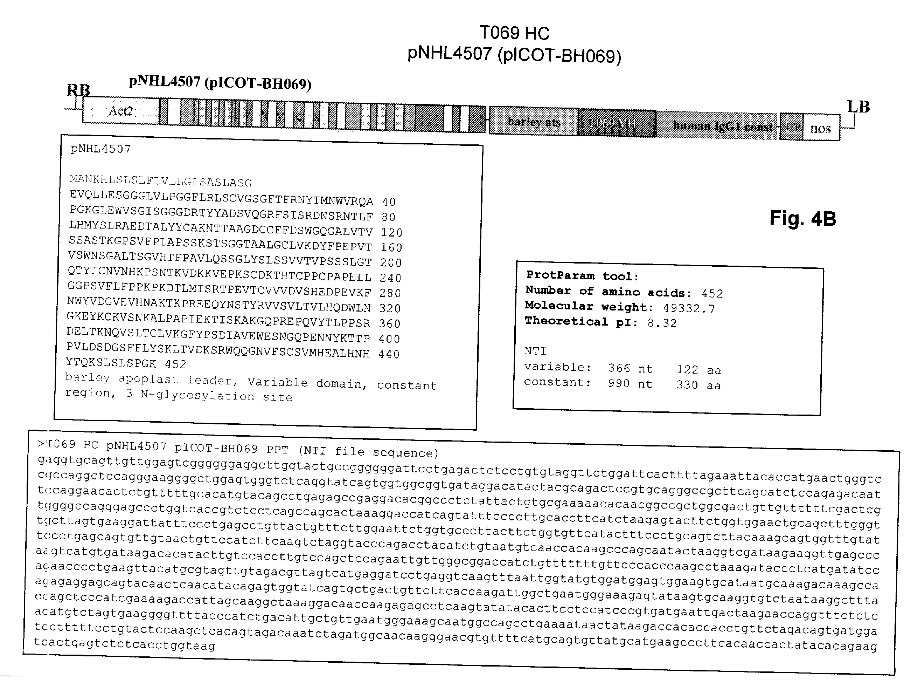

Fig. 4B shows at the top the TMV-based vector pICOT-BH069 used for expressing the heavy chain of Ig T069. The top box shows the amino acid sequence (SEQ ID NO: 9) of the fragment consisting of the barley amylase ats (first line), the variable domain, and the human IgG1 constant region of the T069 heavy chain. The box at the bottom shows the nucleotide sequence (SEQ ID NO: 10) of the fragment encoding the variable domain and the human IgG1 constant region of the T069 heavy chain.

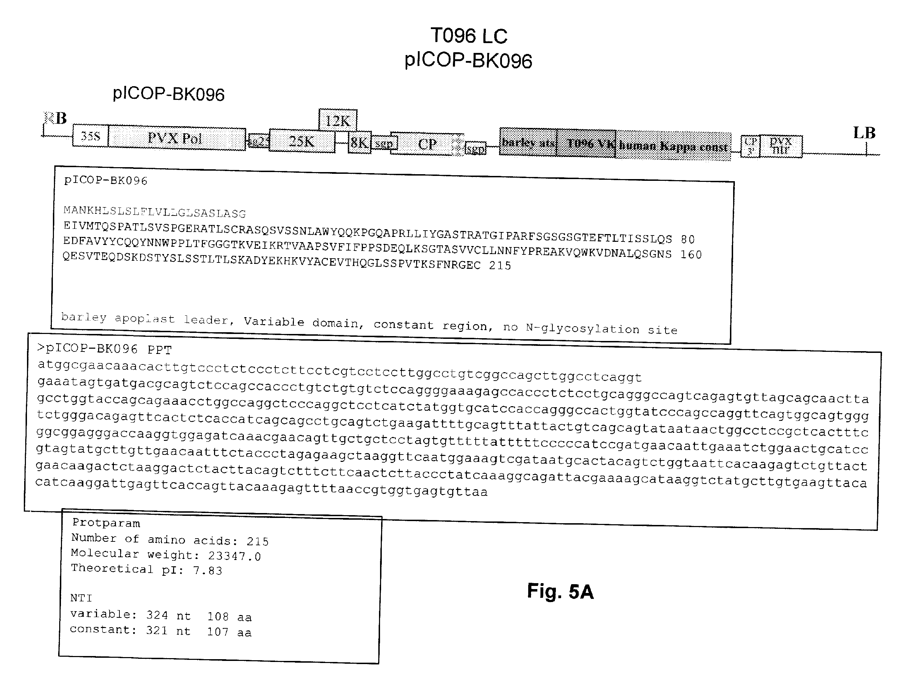

Fig. 5A shows the PVX-based binary vector pICOP-BK096 usable for expressing the light chain of T096 and the amino acid sequence (SEQ ID NO: 11) and nucleotide sequence (SEQ ID NO: 12) of the fragment consisting of or encoding, respectively, the barley amylase ats (first lines of the sequences), the variable domain, and the kappa constant region of the T096 light chain.

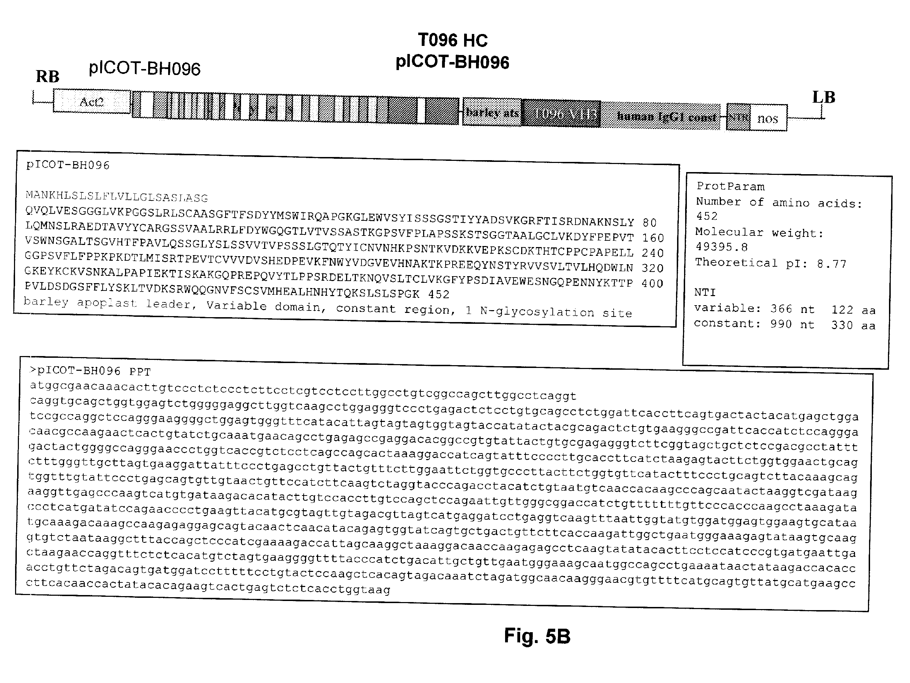

Fig. 5B shows the TMV-based binary vector pICOT-BH096 usable for expressing the heavy chain of T096 and the amino acid sequence (SEQ ID NO: 13) and in the box at the bottom the nucleotide sequence (SEQ ID NO: 14) of the fragment consisting of or encoding, respectively, the barley ats, the variable domain, and the constant region of the T096 heavy chain.

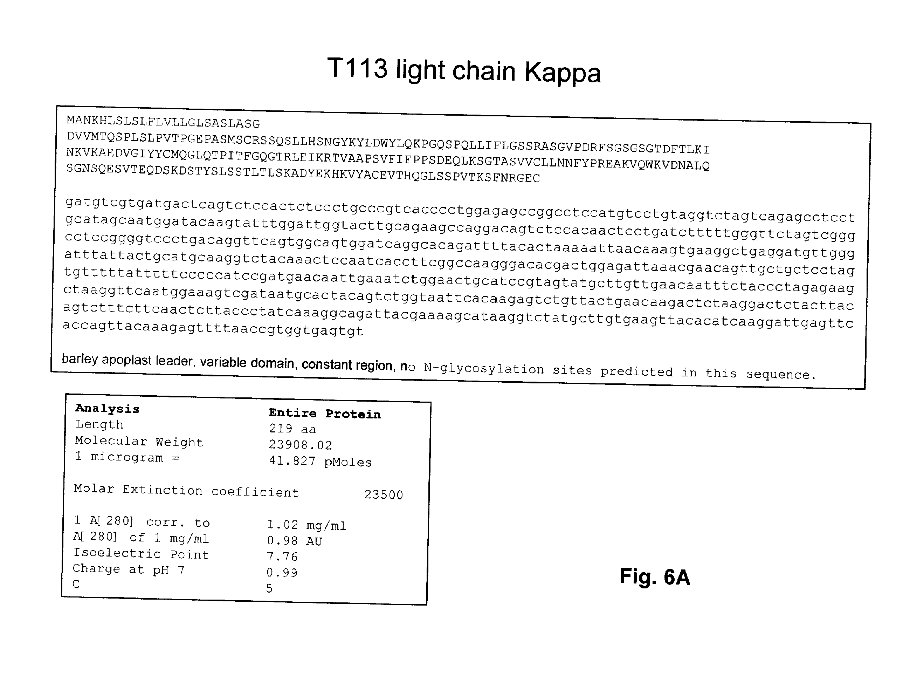

Fig. 6A shows the amino acid sequence (SEQ ID NO: 15) of the fragment consisting of the barley apoplast leader (first line of the amino acid sequence), the variable domain, and the constant region of the T113 kappa light chain. Also shown is the nucleotide sequence (SEQ ID NO: 16) of the fragment encoding the variable domain and the constant region of the T113 kappa light chain.

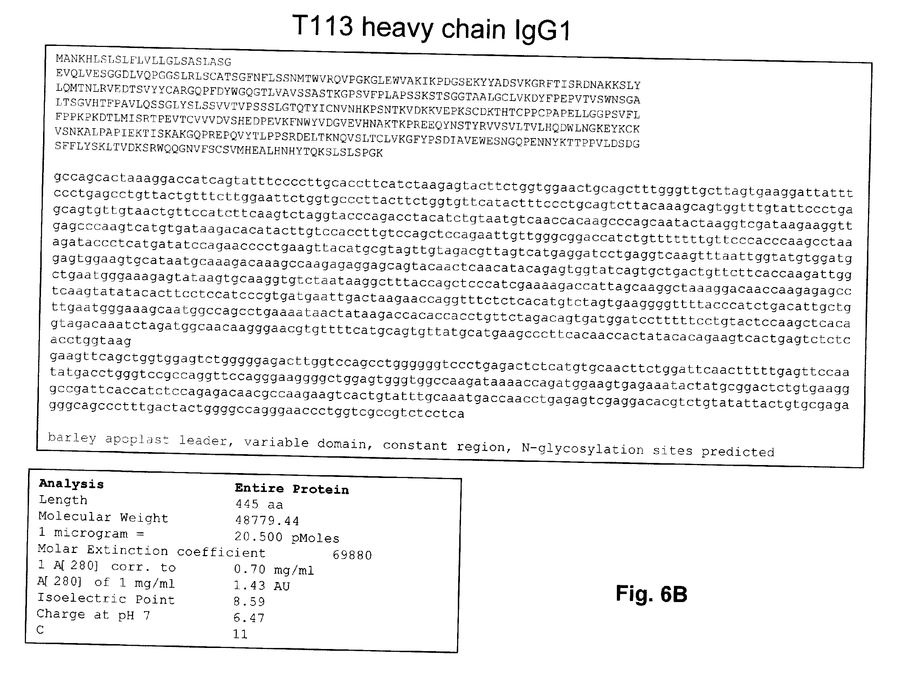

Fig. 6B shows the amino acid sequence (SEQ ID NO: 17) of the fragment consisting of the barley ats (first line of the amino acid sequence), the variable domain, and the constant region of the T113 heavy chain of IgG1 T113. Also shown is the nucleotide sequence (SEQ ID NO: 18) of the fragment encoding the variable domain and the constant region of the T113 heavy chain of IgG1 T113.

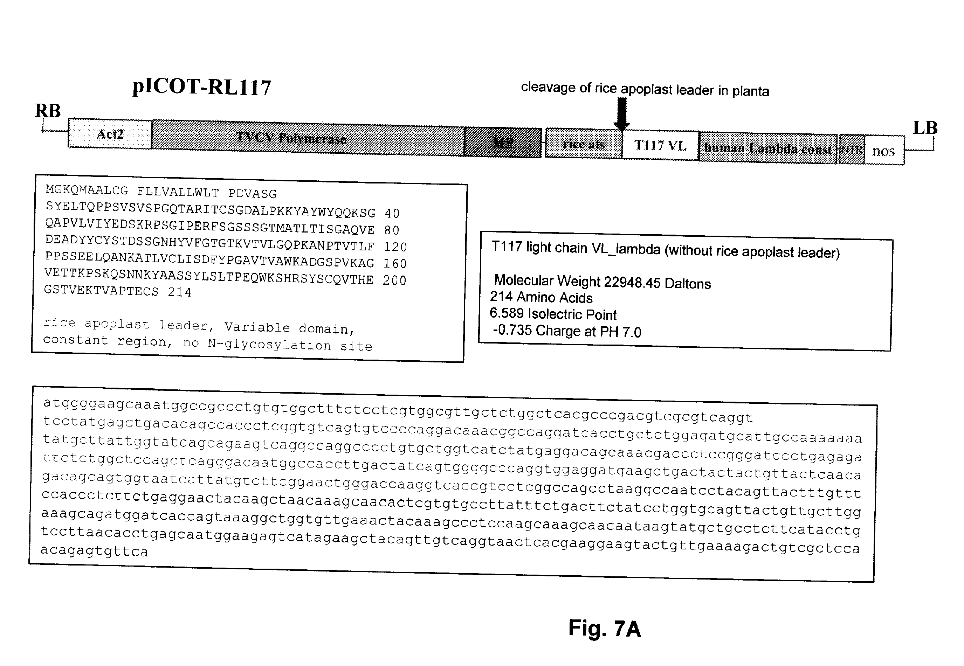

Fig. 7A depicts the TMV-based binary vector PICOT-RL117 encoding the T117 light chain. The amino acid sequence (SEQ ID NO: 19) and the nucleotide sequence (SEQ ID NO: 20) of the fragment consisting of or encoding, respectively, the rice apoplast leader, the variable domain, and a human lambda constant region are shown.

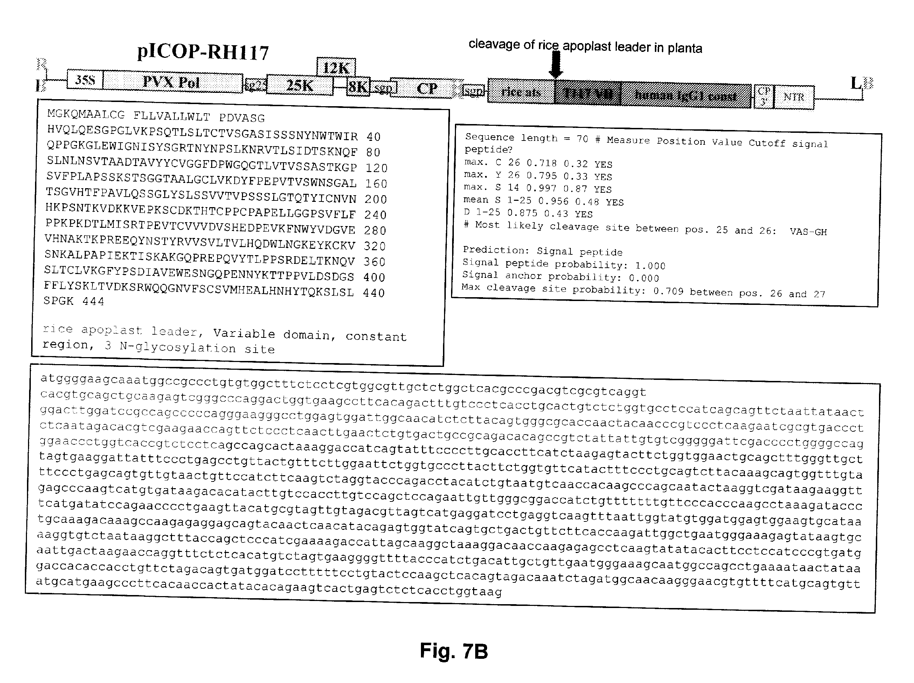

Fig. 7B depicts binary vector PICOP-RH117 containing the T117 heavy chain with rice apoplast leader. The amino acid sequence (SEQ ID NO: 21) and the nucleotide sequence (SEQ ID NO: 22) of the fragment consisting of or encoding, respectively, the rice apoplast leader, the variable domain, and the IgG1 constant region are shown.

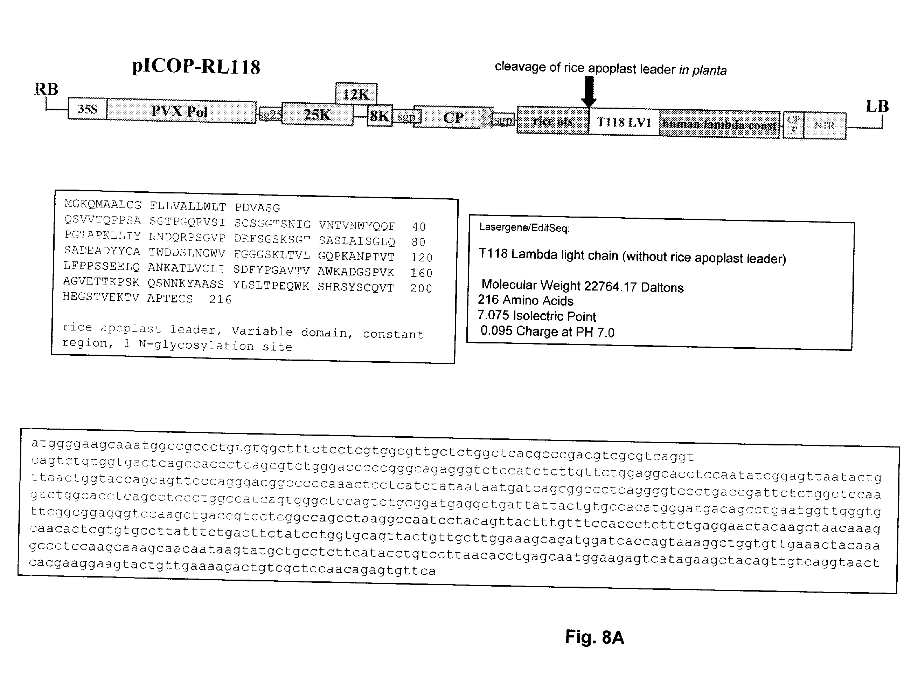

Fig. 8A depicts the PVX-based binary vector PICOP-RL118 encoding the T118 light chain. The amino acid sequence (SEQ ID NO: 23) and the nucleotide sequence (SEQ ID NO: 24) of the fragment consisting of or encoding, respectively, the rice apoplast leader (first line of the sequences), the variable domain, and a human lambda constant region are shown.

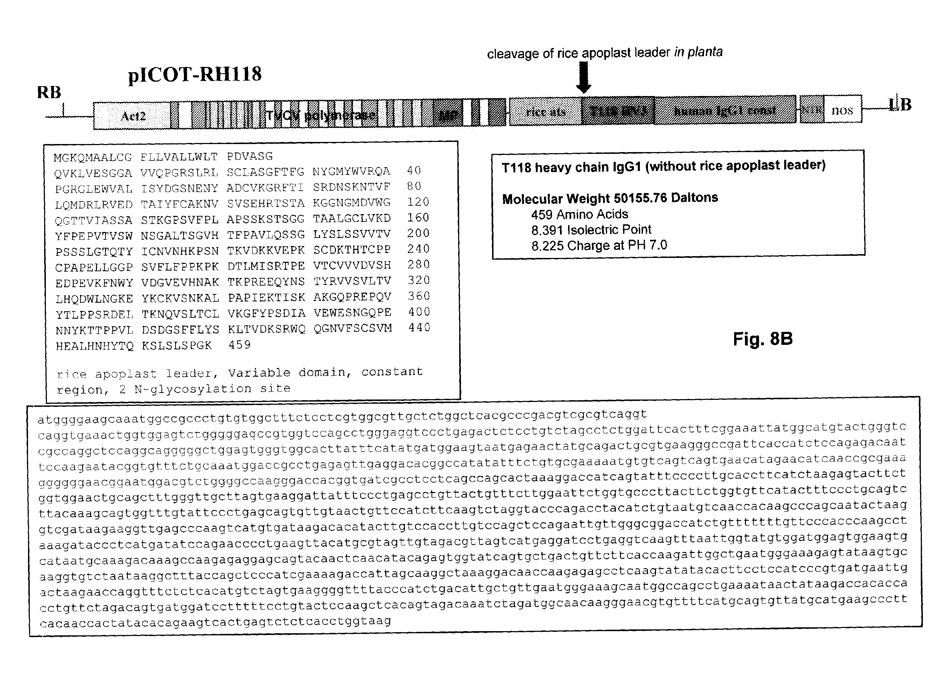

Fig. 8B depicts binary TMV-based vector PICOT-RH118 containing the T118 heavy chain with rice apoplast leader. The amino acid sequence (SEQ ID NO: 25) and the nucleotide sequence (SEQ ID NO: 26) of the fragment consisting of or encoding, respectively, the rice apoplast leader, the variable domain, and the IgG1 constant region are shown.

### Detailed Description of the Invention

The immunoglobulin used in the invention may be an immunoglobulin A, M or G. Immunoglobulins A and G are preferred and immunoglobulin G is most preferred. Immunoglobulin G refers to immunoglobulin G molecules of the subclasses such as IgG1, IgG2, IgG3 or IgG4. Preferably, the immunoglobulin G of the present invention is of the subclass IgG1 or IgG3, more preferably IgG1.

Examples of immunoglobulins (Igs) to be used in the present invention are tumor-derived idiotypic immunoglobulins from non-Hodgkin lymphoma patients. For example, immunoglobulins G (IgGs) can be used for this purpose. Tumor-specific antibodies from non-Hodgkin lymphoma patients contain a collection of epitopes present in the variable region of the clonal immunoglobulin which can be used as a target for active immunotherapy. The tumor-specific immunoglobulins from individual B cell lymphomas can be identified and isolated using "rescue hybridization" or molecular cloning techniques (Levy and Dilley, PNAS, 75 (1978) 2411-2415; Hawkins et al., Blood, 83 (1994), 3279-3288), yielding patient-specific (idiotypic) immunoglobulin which can be used as a tumor antigen for vaccination. Immunoglobulins can also be recombinantly expressed in host cells. A suitable process of expressing immunoglobulins, including patient-specific idiotypic immunoglobulins, is expression in plants or plant cells as described in WO 2006/079546 and Giritch et al., Proc. Natl. Acad. Sci. USA 2006 103 (40) 14701-14706.

When used for vaccination such as in the treatment of non-Hodgin lymphoma (NHL), the immunoglobulins are preferably administered together with suitable adjuvants for increasing immune response. Established adjuvants can be used for this purpose such as hGM-CSF (human granulocyte-macrophage colony stimulating factor). Another possibility preferred herein is conjugation of the immunoglobulins to a highly immunogenic carrier protein for enhancing the immunogenicity. It is also possible to combine various adjuvants. For example, a protein conjugate having the immunoglobulin conjugated to a carrier protein can be administered together with an adjuvant such as hGM-CSF.

Carrier proteins to which immunoglobulins can be conjugated are known to the skilled person. Examples are diphtheria toxin, tetanus toxin, human serum albumin (HSA), bovine serum albumin (BSA), or hemocyanin such as keyhole limpet hemocyanin (KLH) or subunits thereof. In one embodiment, BSA, hemocyanin (such as KLH) or subunits of hemocyanin are used as the carrier protein. In a preferred embodiment, hemocyanin (such as KLH) or a subunit thereof is used as carrier proteins. KLH is commcercially available e.g. from biosyn GmbH (see below).

Hemocyanins are generally isolated from mollusks such as the giant keyhole limpet. Hemocyanins are oligomeric proteins i.e. have multiple subunits. The hemocyanin subunits associate to form the multi-subunit oligomers. Native KLH is a cylindrical copper containing blue protein with a molecular mass ranging from 8 to 32 MDa. The most prevalent oligomeric forms are didecamers with a molecular mass of 8 MDa and multidecamers with a molecular mass of 12-32 MDa. The didecamer has 20 subunits and occurs in two different forms named KLH1 and KLH2. The amino acid sequences of KLH1 and KLH2 are given in the annex as SEQ ID NO: 1 and SEQ ID NO:2, respectively. Instead of native KLH, subunits of KLH can be used as carrier protein in the present invention. Subunits of KLH are also referred to as immunocyanin. A commercially available immunocyanin is VACMUNE^{®} from biosyn.

Herein, "protein conjugate" means a protein comprising one or more immunoglobulin molecules that are covalently bound to a carrier protein. Thus, a protein conjugate molecule comprises a carrier protein and one or more immunoglobulin moieties. The "carrier protein" may be an oligomer, as described above for hemocyanin. It is possible that the immunoglobulin moieties are bound to the carrier protein via linkers. If more than one immunoglobulin molecule is bound to a carrier protein, the immunoglobulin moieties generally have the same CDRs or the same variable regions. In one embodiment, the immunoglobulin moieties are identical.

Methods for conjugating proteins are known to the skilled person and are widely used for conjugating haptens to carrier proteins in processes of antibody generation, and suitable kits are commercially available. As an example for the conjugation reaction, the reaction of an immunoglobulin (such as an IgG) and the carrier protein using the bifunctional linker glutaraldehyde may be used.

In the conjugation reaction, the Ig (such as IgG) molecule and the carrier protein can be employed in different ratios depending on whether multiple immunoglobulin molecules are to be bound to a carrier protein. Accordingly, the molar ratio of Ig to carrier protein subunit may be from about 4:1 to about 1:1, preferably the molar ratio is from about 2:1 to 1:1. In terms of mass ratio, the mass ratio of Ig to carrier protein may be from about 1.5 to 0.4, preferably from about 0.75 to 0.4.

A commercial kit for conjugating proteins to KLH as a carrier protein and that has been used in the examples of this invention for conjugating idiotypic IgG molecules to a carrier protein is VACMUNE^{®}(biosyn GmbH, Fellbach, Germany). In this system, proteins are conjugated to 400 kDa subunits of hemocyanin in water, using 0.1 % glutaraldehyde as the conjugating reagent with the molar ratio of IgG to the 400 kDa KLH subunits of about 2.5:1 in the conjugation reaction. The resulting protein conjugate was further purified and can be formulated into the formulation of the present invention by introducing the reaction product in the desired buffer.

The preparations of the invention contain histidine as the buffering agent and the immunoglobulin or the protein conjugate comprising the immunoglobulin. The preparations of the invention may be aqueous liquid preparations or solid preparations. The solid preparations may be prepared from the aqueous liquid preparation by freeze-drying (lyophilisation). The lyophilised solid preparation may be reconstituted to form a liquid aqueous preparation by adding water or an aqueous solvent. An example of the aqueous liquid preparation is the aqueous pharmaceutical formulation of the invention that may, in addition to the immunoglobulin or the protein conjugate, histidine and water, further contain pharmaceutical excipients such as an isotonising agent, a preservative or others (see further below).

In the aqueous formulation of the invention, the protein conjugate may be present in a concentration of from about 0.1 to about 10mg/ml, preferably in an amount of from about 0.5 to about 5mg/ml and more preferably of about 0.7 to 2.5 mg/ml.

The histidine concentration in the aqueous formulation or the aqueous liquid preparations may be 5mM to 100mM histidine, preferably 10mM to 50mM, more preferably 20 mM to 30 mM histidine. Regarding the pH of the aqueous formulation or the aqueous liquid preparations, a pH which is close to the physiological pH of humans should be chosen when used for administration to patients. The pH may be between 7.0 to 7.8. In one embodiment it is between 7.2 and 7.6 and more preferably 7.3 to 7.5. The pH may be set as commonly known by adding an acid to an aqueous histidine solution. Examples of the acid are inorganic acids such as hydrochloric acid and sulfuric acid, or organic acids such as citric acid, lactic acid, tartaric acid and other physiologically acceptable acids.

The aqueous formulation or the aqueous liquid preparation of the present invention may optionally further contain a nonionic surfactant. Examples of surfactants are known to the skilled person e.g. from McCutcheon's Emulsifiers and Detergents, 1986 North American Edition, McCutcheon Division Publishing Co., Glen Rock, NJ. For medical applications such as for the formulation of the invention, pharmaceutically acceptable nonionic surfactants can be used as known by the skilled person. Combinations of nonionic surfactants can also be used in the present invention.

Among nonionic surfactants, polysorbate surfactants are particularly preferable. Polysorbate surfactants are understood herein to comprise polyoxyethylene sorbitan fatty acid esters, such as polysorbate 80 (poly(oxyethylene)sorbitan monooleate, also known as Tween-80), polysorbate 60 (poly(oxyethylene)sorbitan monostearate, also known as Tween-60), polysorbate 40 (poly(oxyethylene)sorbitan monopalmitate, also known as Tween-40), polysorbate 20 (poly(oxyethylene)sorbitan monolaurate, also known as Tween-20), polysorbate 85 (poly(oxyethylene)sorbitan trioleate, also known as Tween-85) and polysorbate 65 (poly(oxyethylene)sorbitan tristearate, also known as Tween-65). Of these, polysorbate 20 and polysorbate 80 are preferred, with polysorbate 80 being particularly preferred.

Other nonionic surfactants usable in the invention are poly(propylenoxide) and block copolymers of poly(propylenoxide) and poly(ethylenoxide) such as Synperonic^{®} F-68, Pluronic^{®} F68, Lutrol^{®} F68 or Poloxamer 188.

Further additives to the formulations of the invention that may be used instead of the nonionic surfactants are cyclodextrins, such as HP-β-CD, that may be used for protein stabilisation and can be used for parenteral administration of drugs.

The non-ionic surfactant may be present in the aqueous formulation or the aqueous liquid preparation in a concentration of from about 0.001 % to about 2% (w/v), preferably from about 0.01% to about 0.5% (w/v). In a preferred embodiment, the surfactant is present in a concentration of about 0.1 % (w/v), in an alternative preferred embodiment in a concentration of about 0.01 %(w/v).

The formulation or the liquid preparation of the present invention may comprise an isotonisation agent. For the parenteral administration of a pharmaceutical formulation, it is desirable to adjust the tonicity of the formulation to the fluids of the human body. The isotonisation agents to be used in the formulation of the present invention will be apparent to a person skilled in the art. However, sugar alcohols, polyvinyl pyrrolidone and disaccharides are explicitly mentioned. Of these, mannitol and disaccharides are preferred with disaccharides being more preferred. Particularly preferred disaccharides comprise trehalose and sucrose. In a preferred embodiment of the formulation of the present invention, trehalose is used as isotonisation agent.

The pharmaceutical formulation of the invention may further contain one or more additional pharmaceutically acceptable excipients such as buffers, anti-oxidants, bacteriostatic agents, diluents, carriers, surfactants, salts, preserving, stabilizing, wetting or solubilizing agents, and/or excipients for regulating the osmolarity.

In one embodiment of the formulation of the present invention, the formulation comprises histidine as a buffering agent in an amount of from 10mM to 50mM and polysorbate 20 or polysorbate 80 in an amount of from about 0.01% to about 0.1 % (w/v), and an isotonization agent. In another embodiment, the formulation comprises histidine as a buffering agent in an amount of from 10mM to 50mM and polysorbate 20 or polysorbate 80 in an amount of from about 0.01% to about 0.1% (w/v), an isotonization agent, and a protein conjugate comprising an immunoglobulin G conjugated to a carrier protein (e.g. KLH or a subunit thereof) in a concentration of from 0.1 to 10mg/ml, the formulation having a pH of from 7.3 to 7.5.

Generally, a pharmaceutical formulation herein means a formulation which is suitable for administration to patients, notably of human patients. The pharmaceutical formulation may be administered to the patient by way of parenteral administration, preferably by subcutaneous or intramuscular administration, more preferably by way of subcutaneous administration.

The invention has its full potential when a set of at least two different protein conjugate preparations are to be prepared, such as for the production of multiple B-cell lymphoma vaccines for two or more patients. Use of a histidine buffer as described herein then allows to obtain two or more conjugate preparations, each having a high likelihood of having sufficient stability and solubility. Each of the multiple protein conjugate preparations comprises histidine as a buffering agent and a protein conjugate comprising one or more immunoglobulin moieties conjugated to a carrier protein. The immunoglobulin moieties of each element of said set of protein conjugate preparation have identical complementarity determining regions (CDRs) or identical variable regions. Different protein conjugate preparations of said set differ in that the immunoglobulin moieties of the protein conjugates have different CDRs and thus different variable regions.

A method of preparing a plurality of at least two different protein conjugate preparations as defined above may comprise the following steps:
(i) separately providing at least two different immunoglobulins;
(ii) separately conjugating the at least two different immunoglobulins to a carrier protein to obtain said plurality of at least two different protein conjugates;
(iii) separately introducing each protein conjugates of step (ii) into a histidine buffer to obtain the plurality of at least two different protein conjugate preparations.

The invention also provides a pharmaceutical preparation comprising an immunoglobulin and histidine as a buffering agent, and an aqueous pharmaceutical formulation comprising an immunoglobulin (such as immunoglobulin G) and histidine as a buffering agent.

Also provided is a set of at least two different immunoglobulin preparations, each immunoglobulin preparation comprising histidine as a buffering agent and an immunoglobulin; wherein the immunoglobulin molecules of each immunoglobulin preparation have identical CDRs; and wherein different immunoglobulin preparations differ in that the immunoglobulins of different preparations have different CDRs.

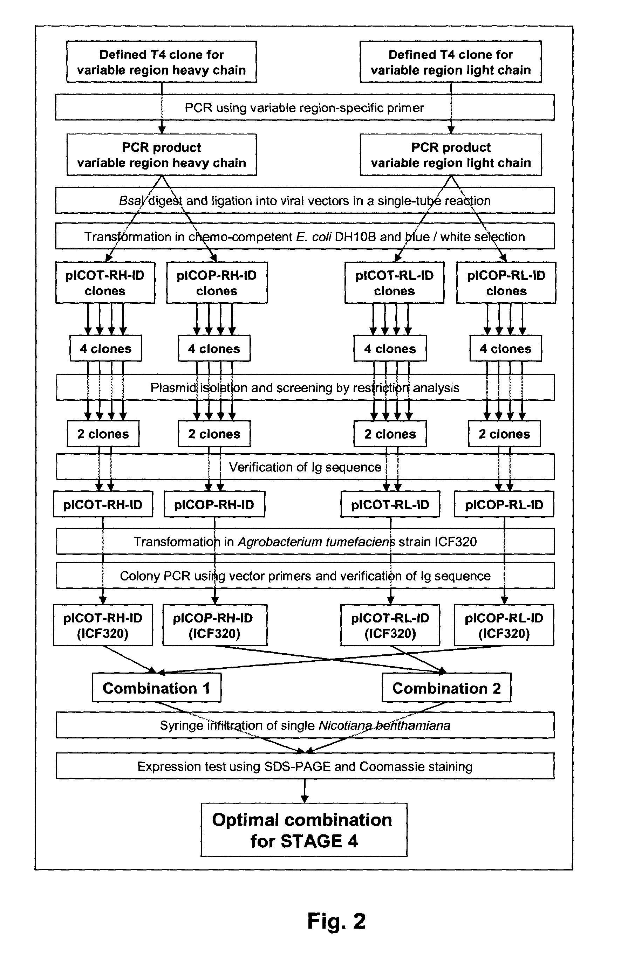

In the following, preparation of idiotypic antibodies for vaccination against NHL and expression in plants will be described. Cloning methods that can be used for cloning and expression of variable regions of Igs are described in WO 2010/040531 that is incorporated herein by reference. Notably, cloning of variable Ig regions is described in detail in Example 2 and with reference to Fig. 2 in WO 2010/040531. Simultaneous expression of heavy and light chains in plants can be done as described in WO2006/079546 or Giritch et al., Proc. Natl. Acad. Sci. USA 2006 103 (40) 14701-14706.

### Expression Vector Preparation

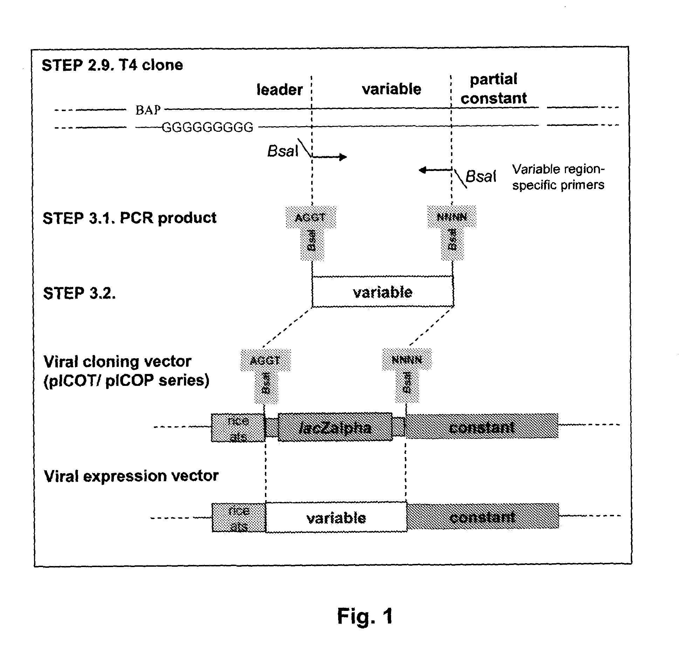

When the tumor sequence of the idiotypic antibody has been determined unequivocally (e.g. after cloning as described in Example 2 of WO 2010/040531), specific primers are designed to amplify the variable regions of the heavy and light chains for subsequent cloning in viral expression vectors of the pICOT (T for TMV) and pICOP (P for PVX) series. The variable region-specific primers (cf. Fig. 1) are designed to contain 6-10 codons of the variable region and the recognition site for the restriction enzyme Bsal, which allows direct cloning of the variable region in the viral expression vectors.

Two T4 clones (Fig. 1, top) are selected as PCR templates whose inserts represent the identified tumor idiotype, one for the light chain and one for the heavy chain. These clones originate from the tumor sequence identification steps of the process (cf. Example 2 of WO 2010/040531).

All amplified variable regions can be cloned in TMV and PVX expression vectors with a rice alpha-amylase signal peptide referred to as "ats" in the figures (an alternative thereto is barley amylase signal peptide). The cloning vectors for the heavy and light chains are TMV (qualified vector designation: pICOT series) and PVX (qualified vector designation: pICOP series) based vectors. Both contain two Bsal restriction sites located between a plant signal peptide and a plant codon-optimized immunoglobulin constant domain. The variable region of heavy and light chain can both be cloned in TMV and PVX vector, as both combinations (heavy chain in TMV and light chain in PVX, and heavy chain in PVX and light chain in TMV) will be tested for expression. The optimal combination of vectors is chosen for production.

### Cloning in viral expression vectors

PCR products which should correspond to 300-400 bp in size are analyzed by agarose gel electrophoresis and column-purified prior to cloning in viral vectors. The PCR fragment is flanked by recognition sites for the class Ils restriction enzyme Bsal. This restriction enzyme is used because the sequence of the 4-nucleotide 5' overhang obtained after digestion can be chosen to be any sequence of choice (the enzyme cuts outside of its recognition site). Therefore, the same enzyme can be used to digest both ends of the PCR product while producing overhangs that are different and therefore incompatible, but compatible with the target vector. Moreover, the recognition sites are eliminated after cloning, resulting in seamless junctions. See Engler et al. PLoS ONE, 2008; 3(11): e3647 for details on cloning methods using type Ils restriction enzymes.

The cloning vectors for the heavy and light chains are TMV (pICOT series) and PVX (pICOP series) based vectors. TMV stands for tobacco mosaic virus. PVX stands for potato virus X. Both types of cloning vectors contain two Bsal restriction sites located between a rice alpha-amylase signal peptide and a plant codon-optimized immunoglobulin constant domain, which can be, for example, IgG1, IgK, or IgL, resulting in a total of six different vectors. The cloning vectors also contain a *lacZ*-alpha gene for blue-white selection to facilitate screening of recombinant clones and a kanamycin-resistance cassette. The variable region of the heavy chain will be cloned in pICOT-RH and pICOP-RH, and the variable region of the light chain in either pICOT-RK and pICOP-RK if it belongs to a kappa chain, or in pICOT-RL and pICOP-RL if it belongs to a lambda chain. For an overview see Fig. 1 and 2.

For cloning, vector and PCR product are digested with Bsal and ligated in a single reaction in a DNA thermocycler. The cloning reaction is transformed in chemo-competent *E. coli* DH10B and plated on LB agar medium containing X-gal and kanamycin and incubated overnight at 37°C. At least one clone per construct with correct sequence (alpha-amylase apoplast targeting signal, variable and constant region) is prepared for long-term storage as glycerol stock and the corresponding plasmid DNA can be transformed in *Agrobacterium tumefaciens.*

### Transformation of viral expression vectors into Agrobacterium tumefaciens

Plasmid DNA of all four viral expression vectors verified by sequencing is electroporated into *Agrobacterium* strain ICF 320, and the transformation plated on selective medium containing kanamycin (viral vector resistance marker), rifampicin (ICF 320 resistance marker) and X-gal (blue/white selection based on lacZ on the Ti plasmid of ICF 320). For an overview see Fig. 2. Plates are incubated for three to four days at 28°C. Transformants are analyzed by colony PCR using vector primers (pICOT-F and pICOT-R; pICOP-F and pICOP-R) designed to amplify the complete immunoglobulin chain. The PCR products are analyzed by agarose gel electrophoresis for correct size (light chain: 1.1 kb; heavy chain: 1.7 kb), column-purified and sequenced (alpha-amylase apoplast targeting signal, variable and constant region). Verified clones are then grown overnight at 28°C in liquid LBS medium containing rifampicin and kanamycin for preparation of glycerol stock cultures and for expression testing.

### Antibody expression test by agro-infiltration of single N. benthamiana plants and SDS-PAGE analysis

To determine which vector combination (heavy chain in TMV or PVX and light chain in the complementary vector) provides the highest expression level, both combinations are infiltrated manually using a syringe without a needle on individual *Nicotiana benthamiana* plants (see Fig. 2). Plants are derived from the same seed bank as the ones used for production.

Overnight cultures of all constructs are diluted in infiltration buffer to a final OD₆₀₀ = 0.002 and the appropriate strains are mixed in one tube. The mixture is infiltrated in the lower side of upper leaves of different *N. benthamiana* plants. Plants are incubated in the greenhouse for about seven days, and infiltrated leaf areas harvested from different plants for each combination (approximately 100 mg fresh weight each). Proteins are extracted and the crude extract is analyzed using SDS-PAGE and Coomassie staining. The combination of vectors which results in the highest expression level is chosen, and the selected *Agrobacterium* strains used for upstream processing.

Igs expressed in plants can be isolated and purified according to generally known methods. For example, standard Protein A-based purification methods using commercial antibody purification kits can be used. Another method for Ig purification is described in WO2007/031339 using plant viral particles having bound affinity proteins such as protein A or derivatives displayed on the surface of the plant viral particles.

### EXAMPLES

### Example 1

### Preparation of conjugates of keyhole limpet hemocyanine and IgG antibodies

0.5 mg/ml keyhole limpet hemocyanine (VACMUNE^{®} liquid IEX from biosyn) and 0.5 mg/ml IgG antibody in PBS buffer pH 7.4 are mixed and cross-linked by adding 0.1 % (v/v) glutardialdehyde. The mixture is incubated at 25°C for 120 minutes. Then, the cross-linking reaction is quenched by adding 1% (v/v) 1 M aqueous glycine solution and incubating for 30 minutes. The protein conjugate solution is then frozen in liquid nitrogen until further use.

### Example 2

### Stability tests using various KLH-IgG conjugates

Conjugates T038 and T096 were rebuffered into the buffers shown below. The final conjugate concentration was 1 mg/ml. The obtained solutions were analysed by the Thermofluor method and by Micro DSC (differential scanning calorimetry) as well as by shaking and by applying shear forces. The following buffers were tested:
i. 20mM citrate pH 5.0
ii. 20mM citrate pH 6.0
iii. 20mM citrate pH 6.6
iv. 20 mM histidine 7.0
v. 20 mM histidine pH 7.4
vi. 20 mM histidine pH 8.0

The conjugates were provided in PBS buffer after conjugation of the IgG antibodies to KLH (see Example 1. Size-exclusion chromatography (SEC) was performed on an ÄKTA^{™} Explorer (GE) system to yield the conjugate in the buffers listed above and indicated in the tables below.

The Thermofluor method was performed using a 750 Fast Real Time PCR System (Applied Biosystems). The formulations were added to a fluorescent dye in a 96 well-plate and were analyzed in the PCR System. The temperature was increased from 20°C to 90°C. The melting points of proteins were determined by fluorescence detection.

Micro DSC was measured using a VP-DSC (GE Healthcare). The temperature was moved from 20°C to 105°C and the melting point of the protein was determined with the calorimeter.

The shear stress was generated by shaking on shaker (IKA, HS 260 control) into a climate chamber (MMM, FrioCell 200). The Critical Quality Parameter (aggregation) was decided after 1 h at 300 rpm and 20°C.

The stability of the solutions after the stress test was checked by noting the visual appearance. Further, dynamic light scattering (DLS) was performed using a Horiba LB550 (Retsch^{®} Technology). The hydrodynamic diameter d_{H} (median) as determined by DLS are given in the tables.

The concentration C_{NHL} was measured by using a Nanodrop 2000 (Thermo Scientific). The NHL vaccines were calibrated at a wavelength of 280 nm.

The results are shown in the following Table 1. "Flocculation" means that individual flocculation particles could be seen by visual inspection.

**Table 1**

| | **after ÄKTA** | | | **after stress test (1 h at 20°C, 300 rpm)** | | |
|---|---|---|---|---|---|---|
| **NHL vaccine T096** | visual appearance | C_{NHL}* [mg/ml] | d_{H} [nm] | visual appearance | CNHL [mg/ml] | d_{H} [nm] |
| citrate pH 5.0 | slightly turbid | 0.11 | 3332 | - | - | - |
| citrate pH 6.0 | slightly turbid | 0.12 | 2427 | - | - | - |
| citrate pH 6.6 | slightly turbid | 0.15 | 199 | flocculation | 0.08 | 193 |
| histidine pH 7.0 | slightly turbid | 0.15 | 227 | flocculation | 0.17 | 190 |
| PBS pH 7.4 | slightly turbid | 0.18 | 199 | flocculation | 0.09 | 198 |
| histidine pH 8.0 | slightly turbid | 0.19 | 181 | no flocculation | 0.17 | 197 |

| **NHL vaccine T038** | | | | | | |
|---|---|---|---|---|---|---|
| citrate pH 5.0 | clear | 0.22 | 2865 | - | - | - |
| citrate pH 6.0 | clear | 0.18 | 14 | - | - | - |
| citrate pH 6.6 | clear | 0.18 | 11 | flocculation | 0,13 | 12 |
| histidine pH 7.0 | clear | 0.19 | 16 | flocculation | 0,16 | 16 |
| PBS pH 7.4 | clear | 0.18 | 26 | flocculation | 0,14 | 9 |
| histidine pH 8.0 | clear | 0.20 | 15 | no flocculation | 0,19 | 11 |

The data in Table 1 show that both conjugates tested are not stable after application of shear stress in any buffer tested except histidine. In histidine, a pH above pH 7.0 was required for achieving stability even upon application of shear stress.

### Example 3

### Stability tests using various KLH-IgG conjugates

The buffer of conjugates T069 and T096 was changed to 20 mM histidine pH 7.4, 0.01% Tween 80 during the workup of the conjugation reaction for storage. For the tests of this example, the buffers of the conjugates were changed to the following ones.
20 mM phosphate (Soerensen) pH 7.4
20 mM HEPES pH 7.4
20 mM TRIS pH 7.4
20 mM MOPS pH 7.4
20 mM histidine pH 7.4

These formulations were prepared with or without Tween 80 (0.1 %), Pluronic® F68 (0.1 %) or Tween 20 (0.1 %). Filtration was performed using a membrane filter (Millipoore, Millex GV, filter unit 0.22 µm. Shear stress was applied by as described in Example 2. DSC assays were performed as described in Example 2 using a VP-Capillary DSC System (Microcal). Thermal stress was applied in differential scanning fluorimetry (DSF) experiments using ThermoFluor^{™} assays, where the impact of external factors on the stability of a protein in solution can be determined by measuring its unfolding temperature. This technology is based on the interaction of a hydrophobic fluorescent dye (Sypro Orange) with hydrophobic domains upon heating. C_{surf}. indicates the surfactant concentration.

**Table 2**

| **NHL vaccine T069** | | **before filtration** | | **after filtration** | | | |
|---|---|---|---|---|---|---|---|
| buffer | C_{surf} [%] | visual appear. | C_{NHL}* [mg/ml] | visual appear. | C_{NHL}* [mg/ml] | d_{H} [nm] | Span |
| histidine pH 7.4 | 0.00 | clear | 0.97 | clear | 1.10 | 29 | 0.58 |
| HEPES pH 7.4 | 0.00 | clear | 0.94 | clear | 0.94 | 20 | 0.74 |
| TRIS pH 7.4 | 0.00 | clear | 0.90 | clear | 0.90 | 30 | 0.61 |
| phosphate pH 7.4 | 0.00 | clear | 1.00 | clear | 1.04 | 23 | 0.65 |
| MOPS pH 7.4 | 0.00 | clear | 0.83 | clear | 0.84 | 34 | 0.49 |
| | **Tween 80** | | | | | | |
| histidine pH 7.4 | 0.01 | clear | - | clear | 1.08 | 31 | 0.5 |
| | **Tween 80** | | | | | | |
| histidine pH 7.4 | 0.1 | clear | - | clear | 0.98 | - | - |
| HEPES pH 7.4 | 0.1 | clear | - | clear | 0.89 | 14 | 0.64 |
| TRIS pH 7.4 | 0.1 | clear | - | clear | 0.85 | 25 | 0.65 |
| phosphate pH 7.4 | 0.1 | clear | - | clear | 0.98 | 25 | 0.59 |
| MOPS pH 7.4 | 0.1 | clear | - | clear | 0.75 | - | - |
| | **Tween 20** | | | | | | |
| histidine pH 7.4 | 0.1 | clear | - | clear | 0.97 | 19 | 0.74 |
| HEPES pH 7.4 | 0.1 | clear | - | clear | 0.86 | 24 | 0.62 |
| TRIS pH 7.4 | 0.1 | clear | - | clear | 0.82 | 29 | 0.51 |
| phosphate pH 7.4 | 0.1 | clear | - | clear | 0.95 | 23 | 0.60 |
| MOPS pH 7.4 | 0.1 | clear | - | clear | 0.75 | 29 | 0.49 |
| | **F68** | | | | | | |
| histidine pH 7.4 | 0.1 | clear | - | clear | 0.98 | 29 | 0.64 |
| HEPES pH 7.4 | 0.1 | clear | - | clear | 0.86 | 27 | 0.64 |
| TRIS pH 7.4 | 0.1 | clear | - | clear | 0.82 | 23 | 0.63 |
| phosphate pH 7.4 | 0.1 | clear | - | clear | 0.93 | 25 | 0.59 |
| MOPS pH 7.4 | 0.1 | clear | - | clear | 0.75 | 25 | 0.68 |

**Table 3**

| **NHL vaccine T069** | | **thermal stress** | | | **shear stress (32h; 30°C, 300 rpm)** | | | |
|---|---|---|---|---|---|---|---|---|
| buffer | C_{surf}. [%] | DSF Tₘ₁ [°C] | DSC T_{m1/}Tₘ₂ [°C] | | visual appear. | C_{NHL} [%] | d_{H} [nm] | Span |
| histidine pH 7.4 | 0.00 | 69.6 | 69.8 | 79.5 | clear | 144.2 | 36 | 0.57 |
| HEPES pH 7.4 | 0.00 | 68.8 | 69.0 | | clear | 104.0 | 30 | 0.58 |
| TRIS pH 7.4 | 0.00 | 69.6 | - | | clear | 103.3 | 37 | 0.54 |
| phosphate pH 7.4 | 0.00 | 68.1 | 69.3 | | clear | 101.2 | 32 | 0.61 |
| MOPS pH 7.4 | 0.00 | 68.8 | 67.3 | | clear | 103.4 | 36 | 0.54 |
| | **Tween 80** | | | | | | | |
| histidine pH 7.4 | 0.01 | -* | 79 | | clear | 108.9 | 33 | 0.44 |
| | **Tween 80** | | | | | | | |
| histidine pH 7.4 | 0.1 | -* | - | | clear | 108.3 | 34 | 0.46 |
| HEPES pH 7.4 | 0.1 | -* | 79.1 | | clear | 115.0 | 26 | 0.66 |
| TRIS pH 7.4 | 0.1 | -* | - | | clear | 104.1 | 28 | 0.64 |
| phosphate pH 7.4 | 0.1 | -* | 80.7 | | clear | 100.4 | 31 | 0.56 |
| MOPS pH 7.4 | 0.1 | -* | - | | clear | 108.7 | 25 | 0.70 |
| | **Tween 20** | | | | | | | |
| histidine pH 7.4 | 0.1 | -* | 79 | | clear | 111,6 | 23 | 0.68 |
| HEPES pH 7.4 | 0.1 | -* | 67.6 | | clear | 102.2 | 34 | 0.54 |
| TRIS pH 7.4 | 0.1 | -* | - | | clear | 103.1 | 36 | 0.61 |
| phosphate pH 7.4 | 0.1 | -* | 66.5 | | clear | 100.4 | 38 | 0.51 |
| MOPS pH 7.4 | 0.1 | -* | 68 | | clear | 104.3 | 31 | 0.63 |
| | **F68** | | | | | | | |
| histidine pH 7.4 | 0.1 | 67.2 | 80.1 | | clear | 112.6 | 23 | 0.71 |
| HEPES pH 7.4 | 0.1 | 66.1 | 73.7 | | clear | 106.4 | 31 | 0.59 |
| TRIS pH 7.4 | 0.1 | 66.9 | 68.2 | | clear | 103.2 | 36 | 0.60 |
| phosphate pH 7.4 | 0.1 | 62.9 | 67.6 | | clear | 101.7 | 39 | 0.48 |
| MOPS pH 7.4 | 0.1 | 65.6 | 68.3 | | clear | 103.8 | 36 | 0.57 |

| | | | | | | | | |
|---|---|---|---|---|---|---|---|---|
| * not measured since surfactants disturb the DSF measurement. | | | | | | | | |

**Table 4**

| **NHL vaccine T096** | | **before filtration** | | **after filtration** | | | |
|---|---|---|---|---|---|---|---|
| buffer | C_{surf}. [%] | visual appear. | C_{NHL}* [mg/ml] | visual appear. | C_{NHL}* [mg/ml] | d_{H} [nm] | Span |
| histidine pH 7.4 | 0.00 | opalescent | 0.97 | opalescent | 0.90 | 75 | 0.50 |
| HEPES pH 7.4 | 0.00 | opalescent | 0.98 | opalescent | 0.83 | 88 | 0.66 |
| TRIS pH 7.4 | 0.00 | turbid | 1.03 | opalescent | 0.64 | 95 | 0.61 |
| phosphate pH 7.4 | 0.00 | opalescent | 1.02 | opalescent | 0.92 | 72 | 0.59 |
| MOPS pH 7.4 | 0.00 | opalescent | 1.07 | opalescent | 0.98 | 96 | 0.53 |
| | **Tween 80** | | | | | | |
| histidine pH 7.4 | 0.01 | clear solution | - | clear solution | 1.10 | 75 | 0.51 |
| | **Tween 80** | | | | | | |
| histidine pH 7.4 | 0.1 | opalescent | - | opalescent | 0.95 | 75 | 0.52 |
| HEPES pH 7.4 | 0.1 | opalescent | - | opalescent | 0.88 | 87 | 0.79 |
| TRIS pH 7.4 | 0.1 | turbid | - | opalescent | 0.66 | 81 | 0.69 |
| phosphate pH 7.4 | 0.1 | opalescent | - | opalescent | 0.98 | 75 | 0.52 |
| MOPS pH 7.4 | 0.1 | opalescent | - | opalescent | 0.94 | 82 | 0.70 |
| | **Tween 20** | | | | | | |
| histidine pH 7.4 | 0.1 | opalescent | - | opalescent | 0.92 | 73 | 0.54 |
| HEPES pH 7.4 | 0.1 | opalescent | - | opalescent | 0.84 | 96 | 0.58 |
| TRIS pH 7.4 | 0.1 | turbid | - | opalescent | 0.64 | 104 | 0.56 |
| phosphate pH 7.4 | 0.1 | opalescent | - | opalescent | 0.95 | - | - |
| MOPS pH 7.4 | 0.1 | opalescent | - | opalescent | 0.97 | 93 | 0.64 |
| | **F68** | | | | | | |
| histidine pH 7.4 | 0.1 | opalescent | - | opalescent | 0.90 | 70 | 0.59 |
| HEPES pH 7.4 | 0.1 | opalescent | - | opalescent | 0.83 | 99 | 0.57 |
| TRIS pH 7.4 | 0.1 | turbid | - | opalescent | 0.64 | 92 | 0.64 |
| phosphate pH 7.4 | 0.1 | opalescent | - | opalescent | 0.92 | 74 | 0.59 |
| MOPS pH 7.4 | 0.1 | opalescent | - | opalescent | 1.07 | 97 | 0.57 |

| | | | | | | | |
|---|---|---|---|---|---|---|---|
| * The measured data are referenced to calibration with the NHL vaccines into the transport buffer (10mM NaH₂PO₄, 0.8% NaCl pH 7.3). | | | | | | | |

**Table 5**

| **NHL vaccine T096** | | **thermal stress** | | **shear stress (32h; 30°C, 300 rpm)** | | | |
|---|---|---|---|---|---|---|---|
| buffer | Csurf [%] | DSF Tₘ₁ [°C] | DSC Tₘ₁ [°C] | visual appear. | CNHL [%] | d_{H} [nm] | Span |
| histidine pH 7.4 | 0.00 | 75.2 | 77.9 | opalescent | 118.5 | 65 | 0.56 |
| HEPES pH 7.4 | 0.00 | 75.2 | 75.2 | opalescent | 102.7 | 136 | 0.77 |
| TRIS pH 7.4 | 0.00 | 75.4 | 74.9 | opalescent | 110.7 | 136 | 0.58 |
| phosphate pH 7.4 | 0.00 | 74.5 | 76.4 | opalescent | 101.1 | 80 | 0.51 |
| MOPS pH 7.4 | 0.00 | 75.4 | 74.7 | opalescent | 101.0 | 94 | 0.67 |
| | **Tween 80** | | | | | | |
| histidine pH 7.4 | 0.01 | -* | - | opalescent | 107.9 | 71 | 0.55 |
| | **Tween 80** | | | opalescent | | | |
| histidine pH 7.4 | 0.1 | -* | - | clear solution | 109.1 | 67 | 0.55 |
| HEPES pH 7.4 | 0.1 | -* | 79.9 | opalescent | 101.6 | 100 | 0.67 |
| TRIS pH 7.4 | 0.1 | -* | 70.0 | opalescent | 119.1 | 129 | 0.80 |
| phosphate pH 7.4 | 0.1 | -* | 83.4 | opalescent | 101.3 | 77 | 0.53 |
| MOPS pH 7.4 | 0.1 | -* | 84.9 | opalescent | 101.1 | 95 | 0.58 |
| | **Tween 20** | | | | | | |
| histidine pH 7.4 | 0.1 | -* | 77.1 | opalescent | 106.2 | 66 | 0.57 |
| HEPES pH 7.4 | 0.1 | -* | 75.1 | opalescent | 100.8 | 99 | 0.67 |
| TRIS pH 7.4 | 0.1 | -* | 68.0 | opalescent | 111.0 | 145 | 0.57 |
| phosphate pH 7.4 | 0.1 | -* | 76.8 | opalescent | 101.3 | 82 | 0.47 |
| MOPS pH 7.4 | 0.1 | -* | 74.2 | opalescent | 102.2 | 95 | 0.61 |
| | **F68** | | | | | | |
| Histidine pH 7.4 | 0.1 | 68.8 | 77.0 | opalescent | 109.1 | 73 | 0.52 |
| HEPES pH 7.4 | 0.1 | 59.1/ 70.4 | 75.2 | opalescent | 105.1 | 98 | 0.60 |
| TRIS pH 7.4 | 0.1 | 58.1/ 71.2 | 74.9 | opalescent | 119.6 | 186 | 0.77 |
| phosphate pH 7.4 | 0.1 | 66.9 | 75 | opalescent | 101.5 | 86 | 0.49 |
| MOPS pH 7.4 | 0.1 | 75.1 | 75.4 | opalescent | 104.3 | 99 | 0.55 |

| | | | | | | | |
|---|---|---|---|---|---|---|---|
| * surfactants disturb the DSF measurement | | | | | | | |

### Example 4

### Tests using further conjugates

In the above examples, histidine buffer showed the best stabilisation at pH 7.4. Histidine buffer was further tested in the presence of 0.01 % Tween 80 with six further conjugates. The conjugates used were T038, T069, T096, T113, T117, T118 at the concentration of 1 mg/ml. Solutions were isotonised with trehalose. The test solutions were made by exchanging a phosphate buffer used for purification of the conjugates into 20 mM histidine pH 7.4 mit 0.01% Tween 80, trehalose. Tests were performed as described above.

**Table 6**

| | **after ÄKTA** | | | **stress test (≥48h 20°C, 300 rpm)** | | |
|---|---|---|---|---|---|---|
| NHL vaccine | visual appear. | C_{NHL}* [mg/ml] | d_{H} [nm] | visual appear. | C_{NHL} [mg/ml] | d_{H} [nm] |
| T038 | clear | 1.45 | 40 | unchanged | 1,46 | 46 |
| T069 | clear | 0.94 | 95 | unchanged | 1,03 | 93 |
| T096 | slightly turbid | 1.32 | 110 | unchanged | 1,31 | 93 |
| T113 | clear | 0.97 | 114 | unchanged | 0,94 | 114 |
| T117 | clear | 0.66 | 76 | unchanged | 0,72 | 70 |
| T118 | clear | 1.03 | 32 | unchanged | 1,02 | 31 |

| | | | | | | |
|---|---|---|---|---|---|---|
| * The measured data are referenced to calibration with the NHL vaccines into the transport buffer (10mM NaH₂PO₄, 0.8% NaCl pH 7.3). | | | | | | |

The data of Table 6 show that a buffer containing 20 mM histidine, 0.01 % Tween 80 stabilises all conjugates tested against temperature and shear stress.

### Example 5

### Stability tests using surfactants

Conjugate T038 was tested in 20 mM histidine pH 7.4 containing various surfactants. The following surfactants were separately tested in this buffer: Tween80 (Polysorbat 80), Tween20 (Polysorbat 20), Synperonic F68. Additionally, HP-ß-CD was used as alternative stabiliser. Results are shown in the following tables.

**Table 7**

| **NHL Va. T038** 20mM His pH7.4 | | **after ÄKTA** | | | |
|---|---|---|---|---|---|
| surfactant | C_{surf}. [%] | visual appear. | C_{NHL}* [mg/ml] | d_{H} [nm] | Span |
| Tween80 | 0.01 | clear sol. | 1.45 | 40 | 0.63 |
| Tween20 | 0.01 | clear sol. | 0.97 | 56 | 0.51 |
| F68 | 0.01 | clear sol. | 1.11 | 55 | 0.50 |
| HP-ß-CD | 0.40 | clear sol. | 1.02 | - | - |

| **NHL Va. T038** 20mM His pH7.4 | | **shear stress (48h, 20°C; 300 rpm)** | | | |
|---|---|---|---|---|---|
| surfactant | C_{surf}. [%] | visual appear. | C_{NHL}* [%] | d_{H} [nm] | Span |
| Tween80 | 0.01 | clear sol. | 98.1 | 51 | 0.45 |
| Tween20 | 0.01 | clear sol. | 106.3 | 55 | 0.52 |
| F68 | 0.01 | clear sol. | 90.1 | 45 | 0.55 |
| HP-ß-CD | 0.40 | clear sol. | 92.7 | 46 | 0.53 |

| **Annex** |
|---|
| Keyhole limpet hemocyanin 1 (KLH1) |
| GenBank entry **Q53IP9** |
| 3408 aa, calculated molecular weight: 391.5 kDa |

SEQ ID NO: 1:

10 20 30 40 50 60
MLSVRLLIVV LALANAENLV RKSVEHLTQE ETLDLQAALR ELQMDSSSIG FQKIAAAHGA

70 80 90 100 110 120
PASCVHKDTS IACCIHGMPT FPHWHRAYVV HMERALQTKR RTSGLPYWDW TEPITQLPSL

130 140 150 160 170 180
AADPVYIDSQ GGKAHTNYWY RGNIGFLDKK TNRAADDRLF EKVKPGQHTH LMESVLDPLE

190 200 210 220 230 240
QDEFCKFEIQ FELPHNAIHY LVGGKHDYSM ANLEYTAYDP IFFLHHSNVD RIFAIWQRLQ

250 260 270 280 290 300

ELRNKDPKAM DCAQELLHQK MEPFSWEDND IPLTNDYDTL NLNGMTPEEL KTYLDERSSR

310 320 330 340 350 360
ARAFASFRLK GFGGSANVFV YVCIPDDNDR NDDHCEKAGD FFVLGGPSEM KWQFYRPYLF

370 380 390 400 410 420
DLSDTVHKMG MKLDGHYTVK AELFSVNGTA LPDDLLPHPV VVHHPEKGFT DPPVKHHQSA

430 440 450 460 470 480
NLLVRKNIND LTREEVLNLR EAFHKFQEDR SVDGYQATAE YHGLPARCPR PDAKDRYACC

490 500 510 520 530 540
VHGMPIFPHW HRLFVTQVED ALVGRGATIG IPYWGLPYWD WTEPMTHIPG LAGNKTYVDS

550 560 570 580 590 600
HGASHTNPFH SSVIAFEENA PHTKRQIDQR LFKPATFGHH TDLFNQILYA FEQEDYCDFE

610 620 630 640 650 660
VQFEITHNTI HAWTGGSEHF SMSSLHYTAF DPLFYFHHSN VDRLWAVWQA LQMRRHKPYR

670 680 690 700 710 720
AHCAISLEHM HLKPFAFSSP LNNNEKTHAN AMPNKIYDYE NVLHYTYEDL TFGGISLENI

730 740 750 760 770 780
EKMIHENQQE DRIYAGFLLA GIRTSANVDI FIKTTDSVQH KAGTFAVLGG SKEMKWGFDR

790 800 810 820 830 840
VFKFDITHVL KDLDLTADGD FEVTVDITEV DGTKLASSLI PHASVIREHA RGKLNRVKFD

850 860 870 880 890 900
KVPRSRLIRK NVDRLSPEEM NELRKALALL KEDKSAGGFQ QLGAFHGEPK WCPSPEASKK

910 920 930 940 950 960
FACCVHGMSV FPHWHRLLTV QSENALRRHG YDGALPYWDW TSPLNHLPEL ADHEKYVDPE

970 980 990 1000 1010 1020
DGVEKHNPWF DGHIDTVDKT TTRSVQNKLF EQPEFGHYTS IAKQVLLALE QDNFCDFEIQ

1030 1040 1050 1060 1070 1080
YEIAHNYIHA LVGGAQPYGM ASLRYTAFDP LFYLHHSNTD RIWAIWQALQ KYRGKPYNVA

1090 1100 1110 1120 1130 1140
NCAVTSMREP LQPFGLSANI NTDHVTKEHS VPFNVFDYKT NFNYEYDTLE FNGLSISQLN

1150 1160 1170 1180 1190 1200
KKLEAIKSQD RFFAGFLLSG FKKSSLVKFN ICTDSSNCHP AGEFYLLGDE NEMPWAYDRV

1210 1220 1230 1240 1250 1260
FKYDITEKLH DLKLHAEDHF YIDYEVFDLK PASLGKDLFK QPSVIHEPRI GHHEGEVYQA

1270 1280 1290 1300 1310 1320
EVTSANRIRK NIENLSLGEL ESLRAAFLEI ENDGTYESIA KFHGSPGLCQ LNGNPISCCV

1330 1340 1350 1360 1370 1380
HGMPTFPHWH RLYVVVVENA LLKKGSSVAV PYWDWTKRIE HLPHLISDAT YYNSRQHHYE

1390 1400 1410 1420 1430 1440
TNPFHHGKIT HENEITTRDP KDSLFHSDYF YEQVLYALEQ DNFCDFEIQL EILHNALHSL

1450 1460 1470 1480 1490 1500
LGGKGKYSMS NLDYAAFDPV FFLHHATTDR IWAIWQDLQR FRKRPYREAN CAIQLMHTPL

1510 1520 1530 1540 1550 1560
QPFDKSDNND EATKTHATPH DGFEYQNSFG YAYDNLELNH YSIPQLDHML QERKRHDRVF

1570 1580 1590 1600 1610 1620
AGFLLHNIGT SADGHVFVCL PTGEHTKDCS HEAGMFSILG GQTEMSFVFD RLYKLDITKA

1630 1640 1650 1660 1670 1680
LKKNGVHLQG DFDLEIEITA VNGSHLDSHV IHSPTILFEA GTDSAHTDDG HTEPVMIRKD

1690 1700 1710 1720 1730 1740
ITQLDKRQQL SLVKALESMK ADHSSDGFQA IASFHALPPL CPSPAASKRF ACCVHGMATF

1750 1760 1770 1780 1790 1800
PQWHRLYTVQ FQDSLRKHGA VVGLPYWDWT LPRSELPELL TVSTIHDPET GRDIPNPFIG

1810 1820 1830 1840 1850 1860
SKIEFEGENV HTKRDINRDR LFQGSTKTHH NWFIEQALLA LEQTNYCDFE VQFEIMHNGV

1870 1880 1890 1900 1910 1920
HTWVGGKEPY GIGHLHYASY DPLFYIHHSQ TDRIWAIWQS LQRFRGLSGS EANCAVNLMK

1930 1940 1950 1960 1970 1980
TPLKPFSFGA PYNLNDHTHD FSKPEDTFDY QKFGYIYDTL EFAGWSIRGI DHIVRNRQEH

1990 2000 2010 2020 2030 2040
SRVFAGFLLE GFGTSATVDF QVCRTAGDCE DAGYFTVLGG EKEMPWAFDR LYKYDITETL

2050 2060 2070 2080 2090 2100
DKMNLRHDEI FQIEVTITSY DGTVLDSGLI PTPSIIYDPA HHDISSHHLS LNKVRHDLST

2110 2120 2130 2140 2150 2160
LSERDIGSLK YALSSLQADT SADGFAAIAS FHGLPAKCND SHNNEVACCI HGMPTFPHWH

2170 2180 2190 2200 2210 2220
RLYTLQFEQA LRRHGSSVAV PYWDWTKPIH NIPHLFTDKE YYDVWRNKVM PNPFARGYVP

2230 2240 2250 2260 2270 2280
SHDTYTVRDV QEGLFHLTST GEHSALLNQA LLALEQHDYC DFAVQFEVMH NTIHYLVGGP

2290 2300 2310 2320 2330 2340
QVYSLSSLHY ASYDPIFFIH HSFVDKVWAV WQALQEKRGL PSDRADCAVS LMTQNMRPFH

2350 2360 2370 2380 2390 2400
YEINHNQFTK KHAVPNDVFK YELLGYRYDN LEIGGMNLHE IEKEIKDKQH HVRVFAGFLL

2410 2420 2430 2440 2450 2460
HGIRTSADVQ FQICKTSEDC HHGGQIFVLG GTKEMAWAYN RLFKYDITHA LHDAHITPED

2470 2480 2490 2500 2510 2520
VFHPSEPFFI KVSVTAVNGT VLPASILHAP TIIYEPGLDH HEDHHSSSMA GHGVRKEINT

2530 2540 2550 2560 2570 2580
LTTAEVDNLK DAMRAVMADH GPNGYQAIAA FHGNPPMCPM PDGKNYSCCT HGMATFPHWH

2590 2600 2610 2620 2630 2640
RLYTKQMEDA LTAHGARVGL PYWDGTTAFT ALPTFVTDEE DNPFHHGHID YLGVDTTRSP

2650 2660 2670 2680 2690 2700
RDKLFNDPER GSESFFYRQV LLALEQTDFC QFEVQFEITH NAIHSWTGGL TPYGMSTLEY

2710 2720 2730 2740 2750 2760
TTYDPLFWLH HANTDRIWAI WQALQEYRGL PYDHANCEIQ AMKRPLRPFS DPINHNAFTH

2770 2780 2790 2800 2810 2820
SNAKPTDVFE YSRFNFQYDN LRFHGMTIKK LEHELEKQKE EDRTFAAFLL HGIKKSADVS

2830 2840 2850 2860 2870 2880
FDVCNHDGEC HFAGTFAILG GEHEMPWSFD RLFRYDITQV LKQMHLEYDS DFTFHMRIID

2890 2900 2910 2920 2930 2940
TSGKQLPSDL IKMPTVEHSP GGKHHEKHHE DHHEDILVRK NIHSLSHHEA EELRDALYKL

2950 2960 2970 2980 2990 3000
QNDESHGGYE HIAGFHGYPN LCPEKGDEKY PCCVHGMSIF PHWHRLHTIQ FERALKKHGS

3010 3020 3030 3040 3050 3060
HLGIPYWDWT QTISSLPTFF ADSGNNNPFF KYHIRSINQD TVRDVNEAIF QQTKFGEFSS

3070 3080 3090 3100 3110 3120
IFYLALQALE EDNYCDFEVQ YEILHNEVHA LIGGAEKYSM STLEYSAFDP YFMIHHASLD

3130 3140 3150 3160 3170 3180
KIWIIWQELQ KRRVKPAHAG SCAGDIMHVP LHPFNYESVN NDDFTRENSL PNAVVDSHRF

3190 3200 3210 3220 3230 3240
NYKYDNLNLH GHNIEELEEV LRSLRLKSRV FAGFVLSGIR TTAVVKVYIK SGTDSDDEYA

3250 3260 3270 3280 3290 3300
GSFVILGGAK EMPWAYERLY RFDITETVHN LNLTDDHVKF RFDLKKYDHT ELDASVLPAP

3310 3320 3330 3340 3350 3360
IIVRRPNNAV FDIIEIPIGK DVNLPPKVVV KRGTKIMFMS VDEAVTTPML NLGSYTAMFK

3370 3380 3390 3400
CKVPPFSFHA FELGKMYSVE SGDYFMTAST TELCNDNNLR IHVHVDDE

Keyhole limpet hemocyanin 2 (KLH2)
GenBank entry **CAG28308**
3421 aa, calculated molecular weight: 391.5 kDa 
SEQ ID NO: 2:

10 20 30 40 50 60
MWTILALLTA TLLFEGAFSV DTVVRKNVDS LSSDEVLALE KALDDLQQDD SNQGYQAIAG

70 80 90 100 110 120
YHGVPTMCVD KHEKNVACCL HGMPSFPLWH RLYVVQLERA LIRKKATISI PYWDWTSELT

130 140 150 160 170 180
HLPELVSHPL FVGTEGGKAH DNSWYRADIT FLNKKTSRAV DDRLFEKVQP GHHTRLMEGI

190 200 210 220 230 240
LDALEQDEFC KFEIQFELAH NAIHYLVGGR HTYSMSHLEY TSYDPLFFLH HSNTDRIFAI

250 260 270 280 290 300
WQRLQQLRGK DPNSADCAHN LIHTPMEPFD RDTNPLDLTR EHAKPADSFD YGRLGYQYDD

310 320 330 340 350 360
LSLNGMSPEE LNVYLGERAA KERTFASFIL SGFGGSANVV VYVCRPAHDE ISDDQCIKAG

370 380 390 400 410 420
DFFLLGGPTE MKWGFYRAYH FDVTDSVASI DDDGHGHYYV KSELFSVNGS ALSNDILRQP

430 440 450 460 470 480
TLVHRPAKGH FDKPPVPVAQ ANLAVRKNIN DLTAEETYSL RKAMERFQND KSVDGYQATV
 
490 500 510 520 530 540
EFHALPARCP RPDAKDRFAC CVHGMATFPH WHRLFVTQVE DALLRRGSTI GLPNWDWTMP

550 560 570 580 590 600
MDHLPELATS ETYLDPVTGE TKNNPFHHAQ VAFENGVTSR NPDAKLFMKP TYGDHTYLFD

610 620 630 640 650 660
SMIYAFEQED FCDFEVQYEL THNAIHAWVG GSEKYSMSSL HYTAFDPIFY LHHSNVDRLW

670 680 690 700 710 720
AIWQALQIRR GKSYKAHCAS SQEREPLKPF AFSSPLNNNE KTYHNSVPTN VYDYVGVLHY

730 740 750 760 770 780
RYDDLQFGGM TMSELEEYIH KQTQHDRTFA GFFLSYIGTS ASVDIFINRE GHDKYKVGSF

790 800 810 820 830 840
VVLGGSKEMK WGFDRMYKYE ITEALKTLNV AVDDGFSITV EITDVDGSPP SADLIPPPAI

850 860 870 880 890 900
IFERADAKDF GHSRKIRKAV DSLTVEEQTS LRRAMADLQD DKTSGGFQQI AAFHGEPKWC

910 920 930 940 950 960
PSPEAEKKFA CCVHGMAVFP HWHRLLTVQG ENALRKHGFT GGLPYWDWTR SMSALPHFVA

970 980 990 1000 1010 1020
DPTYNDAISS QEEDNPWHHG HIDSVGHDTT RDVRDDLYQS PGFGHYTDIA KQVLLAFEQD

1030 1040 1050 1060 1070 1080
DFCDFEVQFE IAHNFIHALV GGNEPYSMSS LRYTTYDPIF FLHRSNTDRL WAIWQALQKY

1090 1100 1110 1120 1130 1140
RGKPYNTANC AIASMRKPLQ PFGLDSVINP DDETREHSVP FRVFDYKNNF DYEYESLAFN

1150 1160 1170 1180 1190 1200
GLSIAQLDRE LQRRKSHDRV FAGFLLHEIG QSALVKFYVC KHNVSDCDHY AGEFYILGDE

1210 1220 1230 1240 1250 1260
AEMPWRYDRV YKYEITQQLH DLDLHVGDNF FLKYEAFDLN GGSLGGSIFS QPSVIFEPAA

1270 1280 1290 1300 1310 1320
GSHQADEYRE AVTSASHIRK NIRDLSEGEI ESIRSAFLQI QKEGIYENIA KFHGKPGLCE

1330 1340 1350 1360 1370 1380
HDGHPVACCV HGMPTFPHWH RLYVLQVENA LLERGSAVAV PYWDWTEKAD SLPSLINDAT

1390 1400 1410 1420 1430 1440
YFNSRSQTFD PNPFFRGHIA FENAVTSRDP QPELWDNKDF YENVMLALEQ DNFCDFEIQL

1450 1460 1470 1480 1490 1500
ELIHNALHSR LGGRAKYSLS SLDYTAFDPV FFLHHANVDR IWAIWQDLQR YRKKPYNEAD

1510 1520 1530 1540 1550 1560
CAVNEMRKPL QPFNNPELNS DSMTLKHNLP QDSFDYQNRF RYQYDNLQFN HFSIQKLDQT

1570 1580 1590 1600 1610 1620
IQARKQHDRV FAGFILHNIG TSAVVDIYIC VEQGGEQNCK TKAGSFTILG GETEMPFHFD

1630 1640 1650 1660 1670 1680
RLYKFDITSA LHKLGVPLDG HGFDIKVDVR AVNGSHLDQH ILNEPSLLFV PGERKNIYYD

1690 1700 1710 1720 1730 1740
GLSQHNLVRK EVSSLTTLEK HFLRKALKNM QADDSPDGYQ AIASFHALPP LCPSPSAAHR

1750 1760 1770 1780 1790 1800
HACCLHGMAT FPQWHRLYTV QFEDSLKRHG SIVGLPYWDW LKPQSALPDL VTQETYEHLF
 
1810 1820 1830 1840 1850 1860
SHKTFPNPFL KANIEFEGEG VTTERDVDAE HLFAKGNLVY NNWFCNQALY ALEQENYCDF

1870 1880 1890 1900 1910 1920
EIQFEILHNG IHSWVGGSKT HSIGHLHYAS YDPLFYIHHS QTDRIWAIWQ ALQEHRGLSG

1930 1940 1950 1960 1970 1980
KEAHCALEQM KDPLKPFSFG SPYNLNKRTQ EFSKPEDTFD YHRFGYEYDS LEFVGMSVSS

1990 2000 2010 2020 2030 2040
LHNYIKQQQE ADRVFAGFLL KGFGQSASVS FDICRPDQSC QEAGYFSVLG GSSEMPWQFD

2050 2060 2070 2080 2090 2100
RLYKYDITKT LKDMKLRYDD TFTIKVHIKD IAGAELDSDL IPTPSVLLEE GKHGINVRHV

2110 2120 2130 2140 2150 2160
GRNRIRMELS ELTERDLASL KSAMRSLQAD DGVNGYQAIA SFHGLPASCH DDEGHEIACC

2170 2180 2190 2200 2210 2220
IHGMPVFPHW HRLYTLQMDM ALLSHGSAVA IPYWDWTKPI SKLPDLFTSP EYYDPWRDAV

2230 2240 2250 2260 2270 2280
VNNPFAKGYI KSEDAYTVRD PQDILYHLQD ETGTSVLLDQ TLLALEQTDF CDFEVQFEVV

2290 2300 2310 2320 2330 2340
HNAIHYLVGG RQVYALSSQH YASYDPAFFI HHSFVDKIWA VWQALQKKRK RPYHKADCAL

2350 2360 2370 2380 2390 2400
NMMTKPMRPF AHDFNHNGFT KMHAVPNTLF DFQDLFYTYD NLEIAGMNVN QLEAEINRRK

2410 2420 2430 2440 2450 2460
SQTRVFAGFL LHGIGRSADV RFWICKTADD CHASGMIFIL GGSKEMHWAY DRNFKYDITQ

2470 2480 2490 2500 2510 2520
ALKAQSIHPE DVFDTDAPFF IKVEVHGVNK TALPSSAIPA PTIIYSAGEG HTDDHGSDHI

2530 2540 2550 2560 2570 2580
AGSGVRKDVT SLTASEIENL RHALQSVMDD DGPNGFQAIA AYHGSPPMCH MHDGRDVACC

2590 2600 2610 2620 2630 2640
THGMASFPHW HRLFVKQMED ALAAHGAHIG IPYWDWTSAF SHLPALVTDH EHNPFHHGHI

2650 2660 2670 2680 2690 2700
AHRNVDTSRS PRDMLFNDPE HGSESFFYRQ VLLALEQTDF CQFEVQFEIT HNAIHSWTGG

2710 2720 2730 2740 2750 2760
HTPYGMSSLE YTAYDPLFYL HHSNTDRIWA IWQALQKYRG FQYNAAHCDI QVLKQPLKPF

2770 2780 2790 2800 2810 2820
SESRNPNPVT RANSRAVDSF DYERLNYQYD TLTFHGHSIS ELDAMLQERK KEERTFAAFL

2830 2840 2850 2860 2870 2880
LHGFGASADV SFDVCTPDGH CAFAGTFAVL GGELEMPWSF ERLFRYDITK VLKQMNLHYD

2890 2900 2910 2920 2930 2940
SEFHFELKIV GTDGTELPSD RIKSPTIEHH GGDHHGGDTS GHDHSERHDG FFRKEVGSLS

2950 2960 2970 2980 2990 3000
LDEANDLKNA LYKLQNDQGP NGYESIAGYH GYPFLCPEHG EDQYACCVHG MPVFPHWHRL

3010 3020 3030 3040 3050 3060
HTIQFERALK EHGSHLGIPY WDWTKSMIAL PAFFADSSNS NPFYKYHIMK AGHDTARSPS

3070 3080 3090 3100 3110 3120
 
DLLFNQPQLH GYDYLYYLAL STLEEDNYCD FEVHYEILHN AVHLWLGGTE TYSMSSLAFS

3130 3140 3150 3160 3170 3180
AYDPVFMILH SGLDRLWIIW QELQKLRKKP YNAAKCAGHM MDEPLHPFNY ESANHDSFTR

3190 3200 3210 3220 3230 3240
ANAKPSTVFD SHKFNYHYDN PDVRGNSIQE ISAIIHDLRN QPRVFAGFVL SGIYTSANVK

3250 3260 3270 3280 3290 3300
IYLVREGHDD ENVGSFVVLG GPKEMPWAYE RIFKYDITEV ANRLNMHHDD TFNFRLEVQS

3310 3320 3330 3340 3350 3360
YTGEMVTHHL PEPLIIYRPA KQEYDVLVIP LGSGHKLPPK VIVKRGTRIM FHPVDDTVNR

3370 3380 3390 3400 3410 3420
PVVDLGSHTA LYNCVVPPFT YNGYELDHAY SLRDGHYYIA GPTKDLCTSG NVRIHIHIED

E
 "Keyhole limpet hemocyanin: Structural and functional characterization of two different subunits and multimers" ; Swerdlow RD et al.; Comparative Biochemistry and Physiology Part B: Biochemistry and Molecular Biology, Volume 113, Issue 3, March 1996, Pages 537-548

## Claims

1. A pharmaceutical preparation comprising a protein conjugate comprising an immunoglobulin conjugated to a carrier protein, and histidine as a buffering agent.

2. The preparation according to claim 1, which is an aqueous formulation.

3. The preparation according to claim 2, wherein the aqueous formulation comprises histidine in an amount of from 5 mM to 100 mM, preferably in an amount of from 10 mM to 50mM.

4. The preparation according to any one of claims 2 or 3, wherein the formulation has a pH of from 7.0 to 7.8, preferably a pH of from 7.2 to 7.6.

5. The preparation according to any one of the claims 1 to 4, further comprising a nonionic surfactant.

6. The preparation according to claim 5, wherein the nonionic surfactant is a polysorbate surfactant such as polysorbate 20 or polysorbate 80.

7. The preparation according to claim 5 or 6, wherein the preparation is an aqueous formulation comprising the nonionic surfactant in a concentration of from about 0.001 % to about 2% (w/v), preferably from about 0.01 % to about 0.5% (w/v).

8. The preparation according to any one of claims 2 to 4 or 7, further comprising an isotonization agent.

9. The preparation according to any one of claims 1 to 8, wherein the carrier protein is keyhole limpet hemocyanin or a subunit thereof.

10. The preparation according to any one of claims 1 to 9, wherein the immunoglobulin is an immunoglobulin containing a patient-specific (idiotypic) antigen for B-cell non-Hodgkin lymphoma, preferably said immunoglobulin is immunoglobulin G.

11. The preparation according to any one of claims 2 to 4, 7 or 8, wherein the protein conjugate is present in a concentration of from 0.1 to 10 mg/ml.

12. A preparation according to any of claims 1 to 11 for use in a method for treatment of the human or animal body by therapy such as for therapy of B-cell non-Hodgkin lymphoma.

13. A set of at least two different protein conjugate preparations, each protein conjugate preparation comprising histidine as a buffering agent and a protein conjugate comprising one or more immunoglobulin moieties conjugated to a carrier protein;
wherein the immunoglobulin moieties of each element of said set of protein conjugate preparation have identical complementarity determining regions (CDRs); and wherein different protein conjugate preparations differ in that the immunoglobulin moieties of the protein conjugates have different CDRs.

14. A method of treating a patient suffering from B-cell non-Hodgkin lymphoma, comprising administering to the patient an aqueous pharmaceutical formulation, said formulation comprising a protein conjugate comprising an immunoglobulin conjugated to a carrier protein, and histidine as a buffering agent; wherein the complementarity determining regions of the immunoglobulin conjugated to the carrier protein elicits an immune response in the patient against the non-Hodgkin lymphoma B-cells.

15. A set of at least two different immunoglobulin preparations, each immunoglobulin preparation comprising histidine as a buffering agent and an immunoglobulin; wherein the immunoglobulin molecules of each immunoglobulin preparation have identical complementarity determining regions (CDRs); and wherein different immunoglobulin preparations differ in that the immunoglobulins of different preparations have different CDRs.
